(19) Europäisches Patentamt
European Patent Office
Office européen des brevets

(11) Publication number: 0 444 533 A2

# (12) EUROPEAN PATENT APPLICATION

(21) Application number: 91102545.0

(22) Date of filing: 21.02.91

(51) Int. Cl.5: C07F 9/58, A61K 31/675, C07F 9/6561, C07F 9/6558, C07D 213/89, C07D 213/55, C07D 221/16, C07F 9/60

The application is published incomplete as filed (Article 93 (2) EPC). The point in the description or the claim(s) at which the omission obviously occurs has been left blank.

(30) Priority: 26.02.90 US 485398

(43) Date of publication of application: 04.09.91 Bulletin 91/36

(84) Designated Contracting States: AT BE CH DE DK ES FR GB GR IT LI LU NL SE

(71) Applicant: E.R. SQUIBB & SONS, INC.
P.O.Box 4000
Princeton New Jersey 08543-4000(US)

(72) Inventor: Robl, Jeffrey Adam
103 Plumly Way
Holland, PA 18966(US)

(74) Representative: Josif, Albert et al
c/o MODIANO, JOSIF, PISANTY & STAUB,
Baaderstrasse 3
W-8000 München 5(DE)

## (54) Quinoline and pyridine anchors for HMG-CoA reductase inhibitors.

(57) Compounds which are useful as inhibitors of cholesterol biosynthesis and thus as hypocholesterolemic agents are provided which have a quinoline or a pyridine anchor attached by means of a linker to a binding domain sidechain, which compounds inhibit the enzyme 3-hydroxy-3-methylglutaryl-coenzyme A reductase.

EP 0 444 533 A2

The present invention is related to compounds and pharmaceutical compositions useful as hypocholesterolemic and hypolipidemic agents. More particularly, this invention concerns (1) certain inhibitors of the enzyme 3-hydroxy-3-methylglutaryl-coenzyme A reductase (HMG-CoA reductase) that contain a quinoline or pyridine nucleus attached by means of a linker to an HMG-binding domain sidechain, (2) pharmaceutical compositions containing such compounds and (3) a method of lowering blood serum cholesterol levels employing such pharmaceutical compositions.

In accordance with the present invention, there are provided certain quinoline- and pyridine-containing compounds that are potent inhibitors of cholesterol biosynthesis by virtue of their ability to inhibit the enzyme-3-hydroxy-3-methyl-glutaryl-coenzyme A reductase (HMG-CoA reductase).

In particular, in its broadest chemical compound aspect, the present invention provides compounds of the formula

I

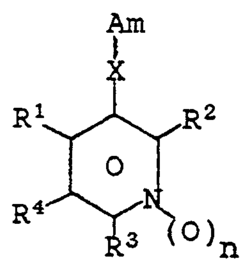

wherein:

Am is a binding domain sidechain;

X is a linker;

$R^1$ and $R^2$ are the same or different and are each independently selected from
- (i) hydrogen,
- (ii) alkyl,
- (iii) aryl,
- (iv) cycloalkyl,
- (v) aralkyl,
- (vi) aralkoxy,
- (vii) alkenyl,
- (viii)cycloalkenyl, and
- (ix) heterocyclo (e.g., thienyl, benzodioxolyl);

$R^3$ is selected from
- (i) hydrogen,
- (ii) lower alkyl,
- (iii) aryl,
- (iv) cycloalkyl,
- (v) alkoxy,
- (vi) aralkyl,
- (vii) aralkoxy,
- (viii)alkenyl,
- (ix) cycloalkenyl,
- (x) halo-substituted alkyl,
- (xi) adamantyl, and
- (xii) heterocyclo (e.g., thienyl, benzodioxolyl);

$R^4$ is selected from
- (i) hydrogen,
- (ii) lower alkyl,
- (iii) aryl,
- (iv) cycloalkyl,
- (v) alkoxy,
- (vi) aralkyl,
- (vii) aralkoxy,

(viii)alkenyl,
(ix) cycloalkenyl,
(x) adamantyl,
(xi) halogen,
(xii)halo-substituted alkyl (e.g., trifluoromethyl), and
(xiii)heterocyclo (e.g., thienyl, benzodioxolyl);

or

$R^3$ and $R^4$ taken together can be $-(CH_2)p-$,

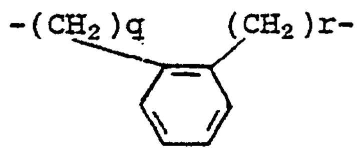

or $(CH=CH)_2$; but when $A_m$ is

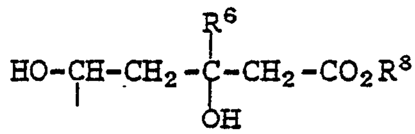

or a δ lactone thereof, $R^3$ and $R^4$ cannot be $(CH=CH)_2$;

$R^6$ is hydrogen or lower alkyl;
$R^8$ is hydrogen, lower alkyl, alkali metal, or alkaline earth metal;
n is 0 or 1;
p is 3, 4 or 5;
q is 0, 1, 2, or 3; and
r is 0, 1, 2, or 3;

provided that at least one of $R^1$, $R^2$, $R^3$ or $R^4$ is alkenyl, cycloalkyl, cycloalkenyl, or heterocyclo, or one of $R^3$ and $R^4$ is adamantyl or together are $-(CH_2)p-$,

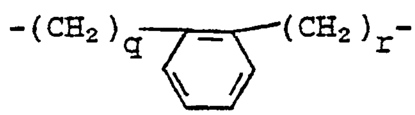

or $(CH=CH)_2$ or $R^4$ is halogen.

In another aspect, the present invention provides pharmaceutical compositions, useful as hypolipidemic or hypocholesterolemic agents comprising a hypolipidemic or hypocholesterolemic amount of a compound in accordance with this invention as set forth above, in combination with a pharmaceutically acceptable carrier.

In another aspect, the present invention provides a method of inhibiting cholesterol biosynthesis in a patient in need of such treatment by administering a pharmaceutical composition in accordance with the present invention as defined above.

In accordance with the present invention, there is provided compounds useful in inhibiting the enzyme 3-hydroxy-3-methylglutaryl-coenzyme A (HMG-CoA reductase), which inhibitors are useful as hypocholesterolemic and hypolipidemic agents, and which compounds comprise a quinoline nucleus or pyridine nucleus

Ia

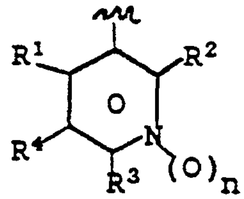

linked by a suitable linker (X) to an HMG-binding domain sidechain (Am) ($R^1$-$R^4$ and n are as defined above).

The quinoline and pyridine nuclei appear to act as hydrophobic anchors of the inhibitor, by binding to a hydrophobic pocket of the reductase enzyme, which results in enhanced inhibitory activity relative to compounds without such an anchor.

## Preferred Moieties

In preferred embodiments of the invention, (Am) is an HMG-binding domain sidechain having a dihydroxy or a phosphinic acid function.

A suitable phosphinic (or phosphonic when X is $CH_2$-O-) acid HMG-binding domain sidechain ($A_1$) is

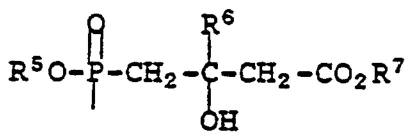

wherein $R^5$ and $R^7$ are independently selected from hydrogen, lower alkyl, alkali metal ion and alkaline earth metal ion; and $R^6$ is hydrogen or lower alkyl. Sidechain $A_1$ is novel and forms an integral part of this invention.

A suitable dihydroxy acid binding domain sidechain ($A_2$) is

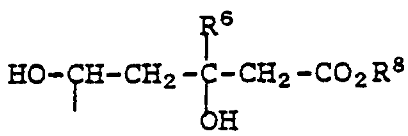

wherein $R^6$ is hydrogen or lower alkyl, $R^8$ is hydrogen or lower alkyl in free acid form or in the form of a physiologically acceptable and hydrolyzable ester or $\delta$ lactone thereof (i.e., when $A_2$ is

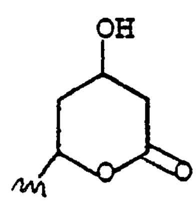

).

In addition, $R^8$ can be alkali metal ion or alkaline earth metal ion.

A suitable linker (X) is $-(CH_2)_a-$, $-CH=CH-$, $-C\equiv C-$, $-CH_2O-$, wherein O is linked to the phosphorous atom or the aromatic anchor when Am is $A_1$, and wherein O is linked to the aromatic anchor when Am is $A_2$, and wherein "a" is 1, 2, or 3.

## Definitions of Terms

The terms "salt" and "salts" refer to basic salts formed with inorganic and organic bases. Such salts include ammonium salts; alkali metal salts, such as lithium, sodium and potassium salts (which are preferred); alkaline earth metal salts, such as calcium and magnesium salts; salts with organic bases, such as amine like salts (e.g., dicyclohexylamine salt, benzathine, N-methyl-D-glucamine, and hydrabamine salts); and salts with amino acids like arginine, lysine and the like; and zwitterions, the so-called "inner salts". Nontoxic, pharmaceutically acceptable salts are preferred, although other salts are also useful, e.g., in isolating or purifying the product.

The term "lower alkyl" or "alkyl" as employed herein alone or as part of another group includes both straight and branched chain hydrocarbons, containing 1 to 12 carbons in the normal, chain or the various branched isomers thereof, preferably 1 to 7 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, and the like, as well as such groups including one or more substituents selected from halogen, (such as F, Br, Cl, and I), $CF_3$, aryl, cycloalkyl, alkoxy, hydroxy, alkylamino, alkanoylamino, carbonylamino, nitro, cyano, mercapto, and alkylthio.

The terms "cycloalkyl" and "cycloalkenyl" as employed herein alone or as part of another group includes cyclic hydrocarbon groups containing 3 to 12 carbons, preferably 3 to 8 carbons, which include cyclopropyl, cyclobutyl, cyclobutenyl, cyclopentyl, cyclopentenyl, cyclohexyl, and cyclohexenyl, as well as groups substituted with 1 or 2 halogens, 1 or 2 lower alkyl groups, 1 or 2 lower alkoxy groups, 1 or 2 hydroxy groups, 1 or 2 alkylamino groups, 1 or 2 alkanoylamino groups, 1 or 2 arylcarbonylamino groups, 1 or 2 amino groups, 1 or 2 nitro groups, 1 or 2 cyano groups, 1 or 2 mercapto groups, and/or 1 or 2 alkylthio groups.

The terms "aryl" or "ar" as employed herein refer to monocyclic or bicyclic aromatic groups containing 6 to 10 carbons in the ring portion, such as phenyl, naphthyl, substituted phenyl or substituted naphthyl, wherein the substituents on either the phenyl or naphthyl may be alkyl, halogen (Cl, Br or F), $CF_3$, 1, 2 or 3 lower alkoxy groups, 1, 2 or 3 aralkyl groups, 1, 2 or 3 hydroxy groups, 1, 2 or 3 phenyl groups, 1, 2 or 3 alkanoyloxy groups, 1, 2 or 3 benzoyloxy groups, 1, 2 or 3 halophenyl groups, 1, 2 or 3 alkyl groups, 1, 2 or 3 alkylamino groups, 1, 2 or 3 alkanoylamino groups, 1, 2 or 3 arylcarbonylamino groups, 1, 2 or 3 amino groups, 1, 2 or 3 nitro groups, 1, 2 or 3 cyano groups, and 1, 2 or 3 thiol groups, with the aryl group preferably containing 3 substituents.

The terms "aralkyl", "aryl-alkyl" or "aryl-lower alkyl" as used herein alone or as part of another group refer to groups having at least one alkyl and at least one aryl group as defined above, such as benzyl, as well as such groups having one or more substituents selected from cycloalkyl, alkylcycloalkyl, amino, oxy, alkoxy, and adamantyl.

The term "alkenyl" by itself or as part of another groups refers to both straight and branched chain groups. Those groups having 2 to 10 carbon atoms are preferred. The term "alkenyl" further includes groups having one or two halo substituents, an alkoxy substituent, an aryl substituent, an alkyl-aryl substituent, a haloaryl substituent, a cycloalkyl substituent, or an alkylcycloalkyl substituent.

The term "alkanoyl" as used herein as part of another group refers to lower alkyl linked to a carbonyl group.

The terms "halogen" or "halo" as used herein refer to chlorine, bromine, fluorine, and iodine, with chlorine and fluorine being preferred.

The term "halo substituted alkyl" refers to alkyl groups in which one or more hydrogens have been replaced by chloro, bromo, or fluoro- groups.

The term "heterocyclo" refers to fully saturated or unsaturated, monocyclic or bicyclic, aromatic or nonaromatic hydrocarbon groups having 5 or 6 atoms in each ring and at least one heteroatom. The heterocyclo group has 1 or 2 oxygen atoms, 1 or 2 sulfur atoms, or 1 to 4 nitrogen atoms in the ring. The bicyclic heterocyclo group may comprise a benzene ring, provided that the bicyclic group is attached by way of an available carbon atom in the benzene ring. The heterocyclo group may be substituted with halogen (Cl, Br or F), $CF_3$, 1, 2 or 3 lower alkoxy groups, 1, 2, or 3 aralkyl groups, 1, 2 or 3 hydroxy groups, 1, 2 or 3 phenyl groups, 1, 2 or 3 alkanoyloxy groups, 1, 2 or 3 benzoyloxy groups, 1, 2 or 3 halophenyl groups, 1, 2 or 3 alkyl groups, 1, 2 or 3 alkylamino groups, 1, 2 or 3 alkanoylamino groups, 1, 2 or 3 arylcarbonylamino groups, 1, 2 or 3 amino groups, 1, 2 or 3 nitro groups, 1, 2 or 3 cyano groups, and 1, 2 or 3 thiol groups, with the aryl group preferably containing 3 groups, preferably having 3 substituents. Exemplary heterocyclo groups are 2- and 3-thienyl, 2- and 3-furyl, 2- and 3-pyrrolyl, 2-, 3- and 4-pyridyl, 2-, 4- and 5-imidazolyl, 2- and 3-pyrrolidinyl, 2-, 3- and 4-piperidinyl, 2-, 3- and 4-azepinyl, 4, 5, 6 or 7-indolyl, 4, 5, 6 or 7-isoindolyl, 5, 6, 7 or 8-quinolinyl, 5, 6, 7 or 8-isoquinolinyl, 4, 5, 6 or 7-benzothiazolyl, 4, 5, 6 or 7-benzoxazolyl, 4, 5, 6 or 7-benzimidazolyl, 4, 5, 6 or 7-benzoxadiazolyl, and 4, 5, 6 or 7-benzofurazanyl.

Processes of Preparation

The compounds of the present invention are made generally in the following manner.

The syntheses of aldehyde 5, which is the starting material for compounds of the invention when $R^3$ and $R^4$ taken together can be $(CH=CH)_2$, are shown in Scheme 1. Reaction of anthranilonitrile 1 with an excess of Grignard reagent ($R^1MgBr$) in an aprotic solvent, such as ether ($Et_2O$) or tetrahydrofuran (THF), followed by acidic and basic work-up affords keto-aniline 2. Friedlander condensation of 2 with an appropriately substituted $\beta$-keto-ester under acidic conditions (i.e. $H_2SO_4$/HOAc/$\Delta$, $H_2SO_4$/ethanol (EtOH)/$\Delta$) provides quinoline 3. Reduction of the ester group in 3 with reducing agents such as $LiAlH_4$ or DIBAL-H in inert solvents such as THF, $Et_2O$, or toluene gives alcohol 4. Oxidants such as Dess-Martin periodinane or oxalyl chloride/dimethylsulfoxide (DMSO)/ triethylamine convert compound 4 to aldehyde 5.

SCHEME 1

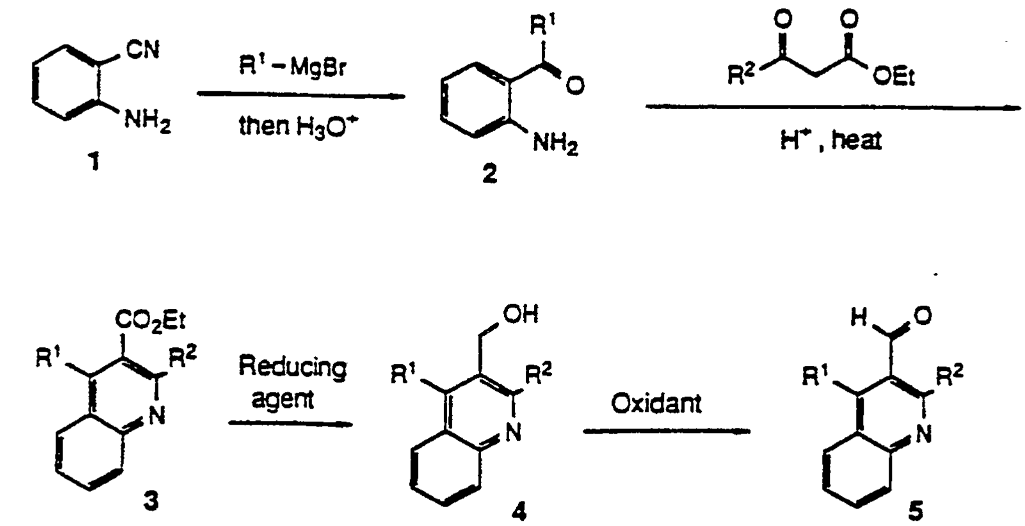


The syntheses of aldehyde 10, which is the starting material for compounds of the invention I wherein $R_3$ and $R_4$ are not $(CH=CH)_2$, are shown in Scheme 2. Condensation of ketone $R^3COCH_2R^4$ with aldehyde $R^1CHO$ under either acidic or basic conditions (i.e. $H_2SO_4$/EtOH or EtONa/EtOH) provides $\alpha,\beta$-unsaturated ketone 6. Michael addition of a $\beta$-keto-ester to compound 6 under basic- conditions (i.e. EtONa/EtOH, KOBut/EtOH, NaH/THF) gives 1,5-diketone 7.

Alternatively, compound 7 may be prepared by an additional route. Knoevenagel condensation of an appropriately substituted $\beta$-keto-ester with aldehyde $R^1CHO$ (piperidine/HOAc/benzene/heat) provides compound 11. Addition of a ketone enolate, generated by treatment of $R^3COCH_2R^4$ with bases such as LDA, LiTMP, or $LiN(TMS)_2$ in an aprotic solvent such as THF, to compound 11 gives 1,5-diketone 7.

Compound 8 is formed by treatment of compound 7 with $NH_2OH.HCl$ or $NH_4OAc/Cu(OAc)_2$ in hot HOAc. Reduction of the ester group in 8 with a reducing agent such as $LiAlH_4$ or DIBAL-H in inert solvents such as THF, $Et_2O$, or toluene gives alcohol 9. Oxidants such as Dess-Martin periodinane or oxalyl chloride/DMSO/triethylamine convert compound 9 to aldehyde 10.

SCHEME 2

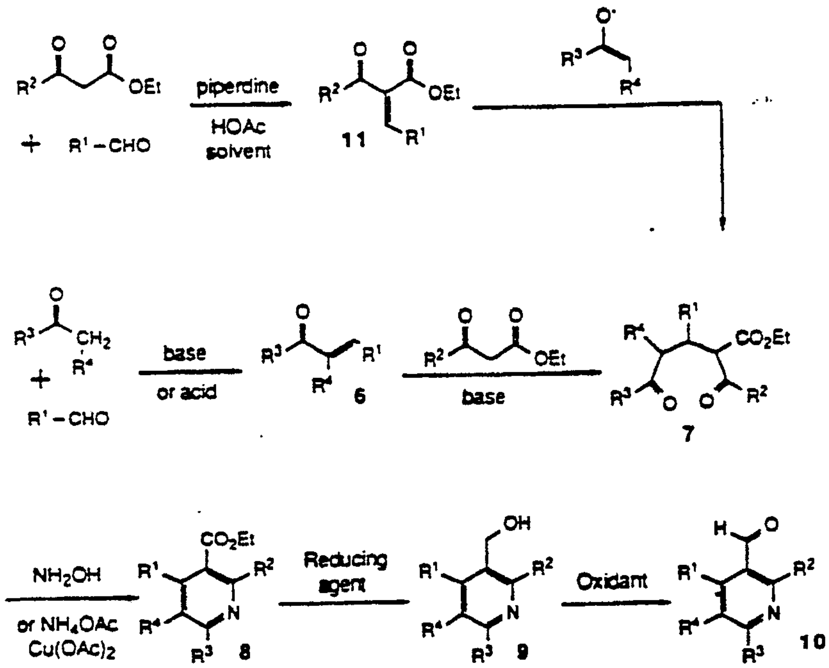


The synthesis of compounds I wherein Am is $A_2$ and X is CH=CH and $CH_2CH_2$ (Compounds 16 and 18) is described in Scheme 3. Reaction of aldehyde 12 with cis-1-ethoxy-2-lithioethylene at low temperatures (-78° C) in an appropriate solvent such as THF, followed by acidic work-up provides enal 13. Condensation of the dianion of methyl acetoacetate with enal 13 in an aprotic solvent (THF, $Et_2O$) affords compound 14. Compound 15 is obtained via stereoselective reduction of 14 with a trialkylborane and a reducing agent such as $NaBH_4$ in a solvent such as THF containing MeOH and a small amount of catalyst such as $O_2$ or pivalic acid. Compound 15 can be saponified to 16 using aqueous solutions of a metal hydroxide in an appropriate solvent (i.e. MeOH, dioxane). Additionally, 15 may be hydrogenated to 17 in the presence of a suitable catayst (Pd, Pt, Ru) and solvent (MeOH, EtOAc, EtOH) and then saponified as described above to provide compound 18.

SCHEME 3

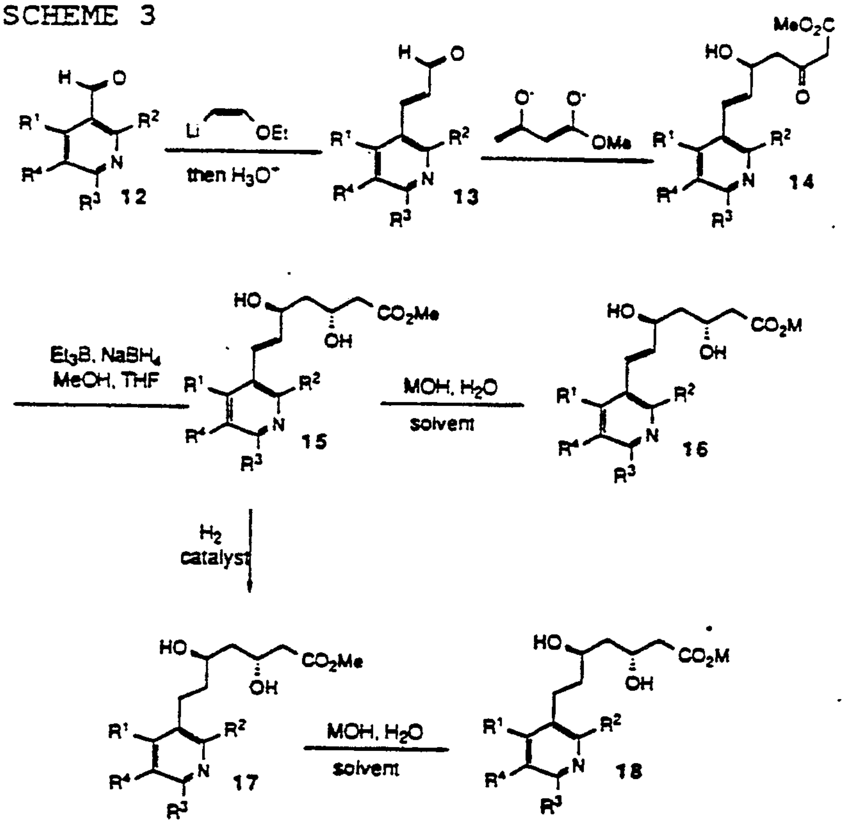


The synthesis of phosphinic acid derviatives I, wherein Am is $A_1$, and X is C≡C and $CH_2CH_2$ - (Compounds 24 and 26) is described in Scheme 4. Reaction of aldehyde 12 with carbon tetrabromide and triphenylphosphine in solvents such as $CH_2Cl_2$ or $CH_3CN$ provides dibromide 19. Conversion of 19 to 20 is effected by treatment of 19 with alkyl lithium reagents such as n-BuLi at low temperature (-78° C) in an aprotic solvent such as THF. Conversely, 20 may be obtained directly from 12 utilizing dimethyl diazomethylphosphonate and KOBut in a solvent such as THF (-78° C to room temperature). Metallation of 20 - (RLi, THF, -78° C) followed by treatment with the chiral phosphonochloridate 21 affords compound 22 which can subsequently be desilylated (TBAF/HOAc) in an appropriate solvent such as THF to provide 23. Compound 23 can be saponified to 24 using aqueous solutions of a metal hydroxide in an appropriate solvent (i.e., MeOH, dioxane). Additionally, 23 may be hydrogenated to 25 in the presence of a suitable catayst (Pd, Pt, Ru) and solvent (MeOH, EtOAc, EtOH) and then saponified as above to provide compound 26.

SCHEME 4

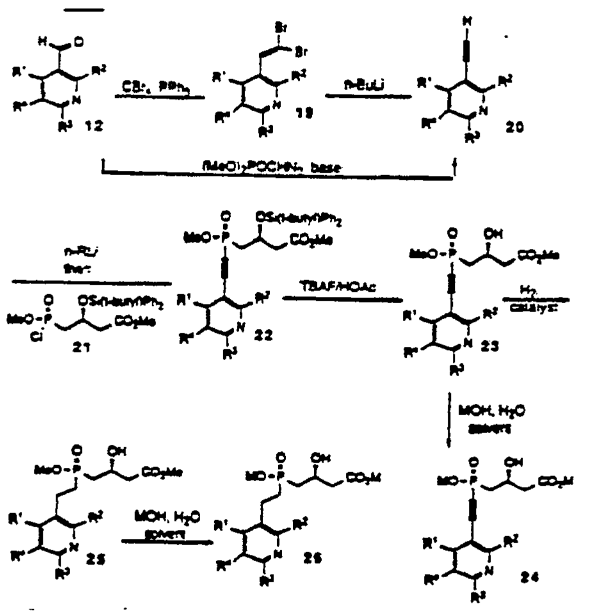


Scheme 5 describes the synthetic route to compounds I where Am is $A_1$ and X is CH=CH (e.g. compound 31. Treatment of acetylene 20 with neat $Bu_3SnH$ in the presence of AIBN at an elevated temperature (i.e. 140° C) affords trans- vinyl stannane 27. Compound 27 was converted to 28 by iodine treatment of 27 in an appropriate solvent such as $Et_2O$ or $CHCl_3$. Metallation of 28 (t-butylLi) at -78° C in an aprotic solvent such as THF gives the corresponding vinyl anion, which may be coupled with phosphonochloridate 21 at -100° C to provide 29. Subsequently 29 may be desilylated (TBAF/HOAc) in an appropriate solvent such as THF to provide 30. Compound 30 can be saponified to 31 using aqueous solutions of a metal hydroxide in an appropriate solvent (e.g., MeOH, dioxane).

SCHEME 5

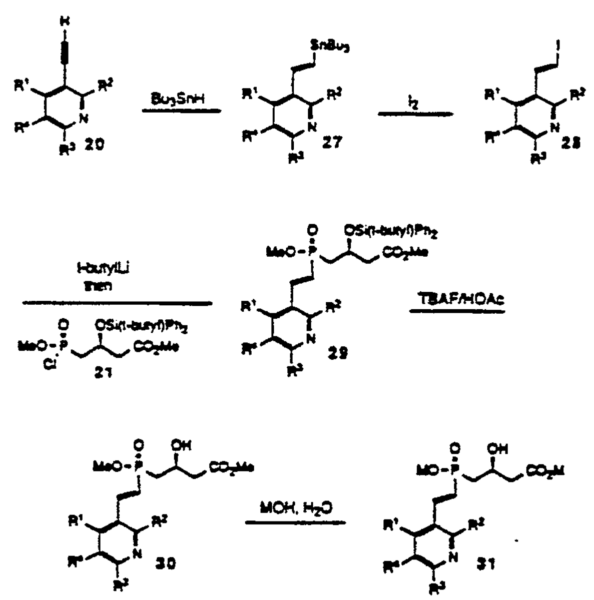


The synthesis of compounds I wherein Am is $A_2$, X is CH=CH and n is 1 (e.g., compound 35) is described in Scheme 6. Bis-silylation of compound 15 with a bulky silylchloride (e.g., ClSi(t-butyl)$Ph_2$,ClSi(t-butyl)$Me_2$, $ClSiPh_3$) in the presence of a suitable base (e.g., TEA, imidazole, pyridine) and solvent (e.g., $CH_2Cl_2$, THF) provides compound 32. Treatment of 32 with oxidants such as m-CPBA or $CF_3CO_3H$ in an appropriate solvent such as $CH_2Cl_2$ or HOAc affords N-oxide 33. Desilylation of 33 (TBAF/HOAc/THF or HF/$CH_3CN$) gives 34, which may be saponified to 35 using aqueous solutions of a metal hydroxide in an appropriate solvent (e.g., MeOH, dioxane).

SCHEME 6

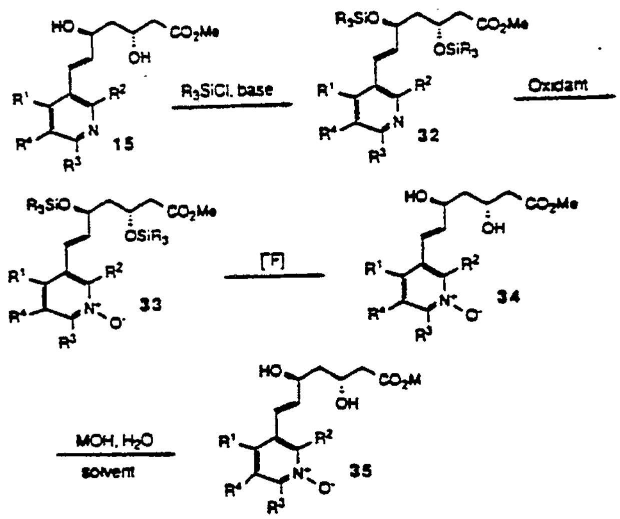


Additionally, compound 17 may be oxidized and saponified, as described above, to provide compounds I wherein Am is $A_2$, X is $CH_2CH_2$ and n is 1 (e.g., compound 37) as shown in Scheme 7.

SCHEME 7

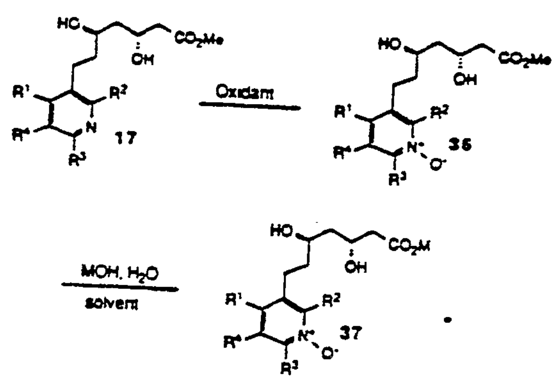


Scheme 8 depicts the chemistry used to synthesize compounds I wherein Am is $A_1$ and n is 1 (e.g., compound 40). Treatment of 38 with oxidants such as m-CPBA or $CF_3CO_3H$ in an appropriate solvent such as $CH_2Cl_2$ or HOAc affords N-oxide 39. Compound 39 may be saponified to 40 using aqueous solutions of a metal hydroxide in an appropriate solvent (i.e. MeOH, dioxane).

SCHEME 8

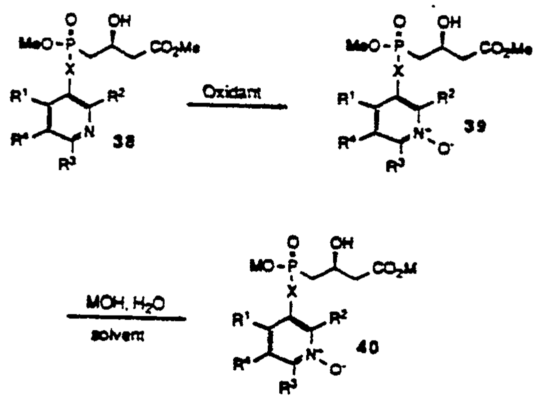


Reaction scheme 9 shows a preferred method for preparing the preferred quinolinealdehyde 5, which can be used as described in schemes 3 and 4 to prepare a corresponding compound of formula I. Reagent 42 (wherein M is lithium or magnesium halide) is reacted with anthranilonitrile 41 to form ketoaniline 43. Acylation of ketoaniline 43 with ethylmalonyl chloride affords amide 44 ("Et" refers to ethyl). Ethanolic sodium ethoxide is used to cause Knoevenagel condensation of amide 44 to form quinoline ester 45. A reducing agent (e.g., lithium aluminum hydride) converts quinoline ester 45 to quinolinolmethanol 46. An oxidizing agent (e.g., manganese dioxide), converts compound 46 to aldehyde 47. Triflation of aldehyde 47 (e.g., with trifluoromethanesulfonic anhydride) affords aldehyde 48. Stannane 49 is prepared by reaction of the corresponding lithium or Grignard reagent with tri-n-alkyltin chloride in diethy ether at -78° C. Aldehyde 48 and stannane 49 undergo palladium-catalyzed coupling to form 2,4-substituted 3-quinolinealdehyde 5. All reactions is scheme 9 take place under argon atmosphere.

SCHEME 9

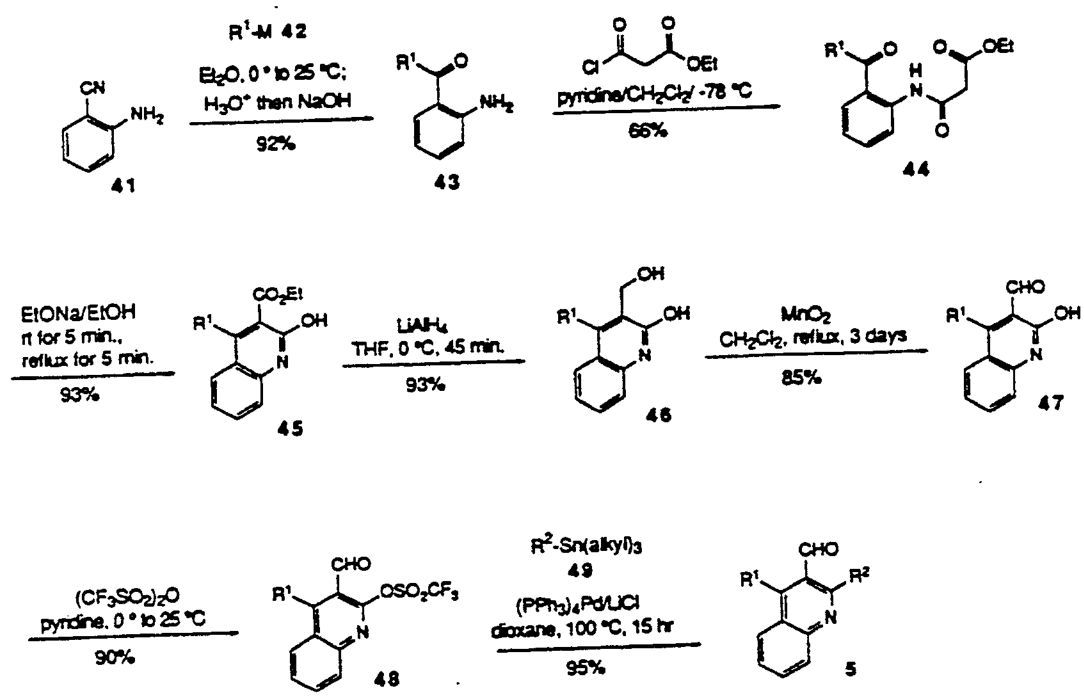


Scheme 10 describes the synthetic route to compounds I wherein Am is $A_1$ and X is $CH_2O$ (e.g. compound 52). Reaction of alcohol 9 with phosphonochloridate 21 in a solvent such as pyridine affords phosphonate 50. Compound 50 may be desilylated (HOAc/TBAF) in THF to give compound 51. Saponification of 51 using aqueous solutions of a metal hydroxide in an organic solvent (e.g., MeOH, dioxane)

provides compound 52. Further reaction conditions are described in Example 99.

SCHEME 10

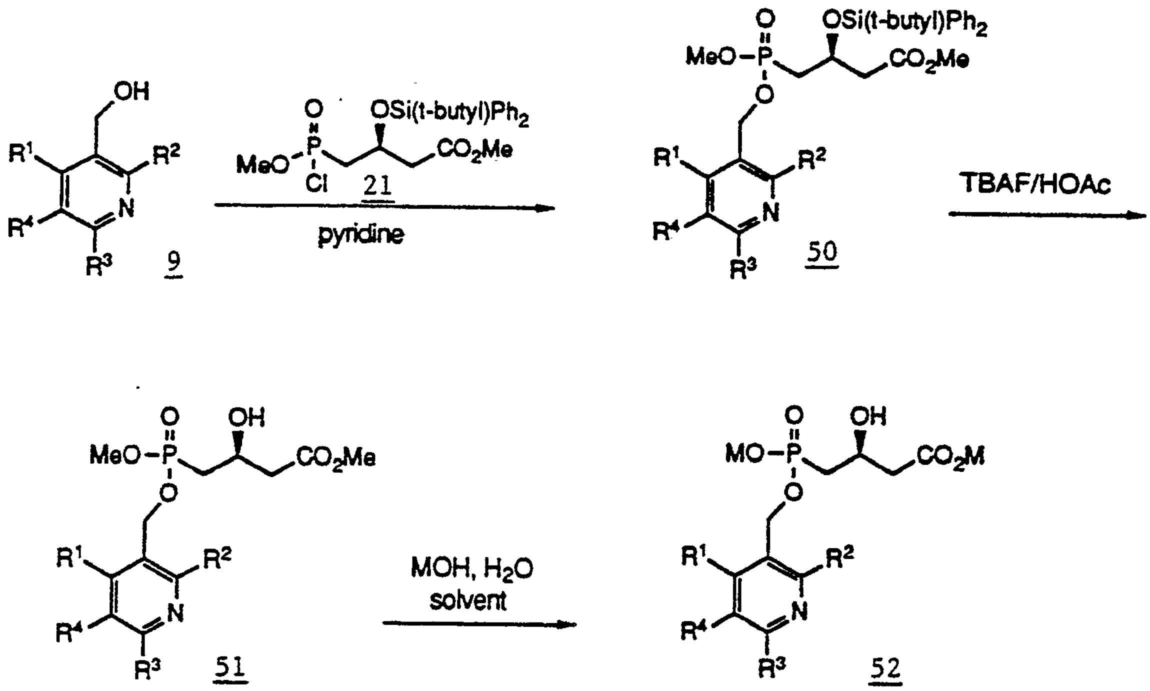


## Pharmaceutical Composition

A further aspect of the present invention is a pharmaceutical composition consisting of at least one of the compounds of the present invention in association with a pharmaceutical vehicle or diluent. The pharmaceutical composition can be formulated employing conventional solid or liquid vehicles of diluents and pharmaceutical additives of a type appropriate to the mode of desired administration. The compounds can be administered by an oral route, for example, in the form of tablets, capsules, granules or powders, or they can be administered by a parenteral route in the form of injectable preparations, such dosage forms containing from 1 to 2000 mg of active compound per dosage, for use in the treatment. The dose to be administered depends on the unitary dose, the symptoms, and the age and the body weight of the patient.

## Use and Utility

The compounds of the present invention can be administered in a similar manner as known compounds suggested for use in inhibiting cholesterol biosynthesis, such as lovastatin, in mammalian species such as humans, dogs, cats and the like. Thus, the compounds of the invention may be administered in an amount from about 4 to 2000 mg in a single dose or in the form of individual doses from 1 to 4 times per day, preferably 4 to 200 mg in divided dosages of 1 to 100 mg, suitably 0.5 to 50 mg 2 to 4 times daily or in sustained release form.

Compounds containing dihydroxy acid HMG-CoA binding domain side chains are prepared as racemic mixtures (3S*, 5R*) and may later be resolved to obtain the 3S, 5R isomer, which is preferred.

Phosphorus-containing HMG-CoA binding domain sidechains may be prepared as racemic mixtures and may later be resolved to obtain the S-isomer, which is preferred. However, these compounds may be prepared directly in the form of their S-isomers as described herein and in the working examples set out hereinafter.

The compounds of the invention are inhibitors of 3-hydroxy-3-methyl-glutaryl coenzyme A (HMG-CoA) reductase.

The following working Examples represent preferred embodiments of the present invention. Unless otherwise indicated, all temperatures are expressed in degrees Centrigrade.

Example 1

(S)-4-[[[4-(4-Fluorophenyl)-2-(1-methylethyl)-5H-indeno[1,2-b]pyridin-3-yl]ethynyl]hydroxyphosphinyl]-3-hydroxybutanoic acid, dilithium salt

A. β-(4-Fluorophenyl)-2,3-dihydro-α-(2-methyl-1-oxopropyl)-1-oxo-1H-indene-2-propanoic acid, ethyl ester

A mixture of 4-fluorobenzaldehyde (3.00 gm, 24 mmol), ethyl isobutyrylacetate (3.82 gm, 24 mmol), piperdine (240 μl), and HOAc (42 μl) was refluxed in benzene (15 ml) with removal of water (Dean-Stark trap) for 22 hours. The cooled mixture was diluted with $Et_2O$ and washed successively with 2% HCl, saturated $NaHCO_3$, $H_2O$, and brine, then dried ($Na_2SO_4$), filtered, and stripped to yield an oil. Distillation of the oil (bp 110-113 °C at 0.25 mm) afforded 2-[(4-fluorophenyl)methylene]-4-methyl-3-oxopentanoic acid, ethyl ester (5.32 gm, 83%) as a pale yellow liquid. This reaction has been successfully scaled up to 300 mmol.

TLC: $R_f$ 0.35 (20% EtOAc in hexane)

Micro Analyzed for $C_{15}H_{17}FO_3$:

| | | | |
|---|---|---|---|
| Calc'd: | C 68.17 | H 6.48 | F 7.19 |
| Found: | C 68.06 | H 6.65 | F 7.35 |

A -78 °C solution of $LiN(TMS)_2$ (1.0 M in THF, 19 ml, 19 mmol) in dry THF (25 ml) was treated with a solution of 1-indanone (2.50 gm, 19 mmol) in THF (3 ml) over a 2-minute period. After 50 minutes, a solution of the ethyl ester compound prepared above (5.00 g, 19 mmol) in THF (5 mL) was added dropwise to the above solution. After one hour, the mixture was quenched with saturated $NH_4Cl$ and warmed to room temperature. The mixture was diluted with $H_2O$ and subsequently extracted twice with $Et_2O$. The combined $Et_2O$ extracts were washed with brine, dried ($Na_2SO_4$), filtered, and stripped to yield a brown cloudy oil. Flash chromatography (Merck $SiO_2$, 20% EtOAc in hexane) afforded the impure title compound (4.671 gm, oil/solid) as a complex mixture of diastereomers. The material was used directly in the next reaction.

TLC: $R_f$ 0.30, 0.24, & 0.20 (20% EtOAc in hexane)

B. 4-(4-Fluorophenyl)-2(1-methylethyl)-5H-indeno[1,2-b]pyridine-3-carboxylic acid, ethyl ester

A mixture of the crude compound of part A (4.671 gm), $NH_4OAc$ (2.74 gm, 35.5 mmol), and $Cu(OAc)_2$ - (5.88 gm, 29.5 mmol) in glacial HOAc (30 ml) was gently refluxed for 6 hours. The solution was cooled to room temperature and subsequently poured into an ice cold mixture of concentrated $NH_4OH$ (50 ml) in $H_2O$ (100 ml). The mixture was extracted once with $Et_2O$ and once with EtOAc and the pooled organic extracts were washed with $H_2O$ and brine, then dried ($Na_2SO_4$), filtered and stripped to yield a dark brown oil. TLC showed the reaction had not gone to completion. The oil was flashed (Merck $SiO_2$, 10% EtOAc in hexane) to give the title compound as a pinkish oil (1.416 gm, 20% from compound 1).

TLC: $R_f$ 0.48 (20% EtOAc in hexane)

C. 4-(4-Fluorophenyl)-2-(1-methylethyl)-5H-indeno[1,2-b]pyridine-3-methanol

A solution of the ester from part B (1.410 gm, 3.76 mmol) in dry THF (50 ml) was treated with $LiAlH_4$ - (740 mg, 19.5 mmol). The deep purple reaction mixture was stirred at room temperature for 7.5 hours. The solution was then cooled to 0 °C and quenched in succession with $H_2O$ (1 ml), 10% NaOH (1 ml), and $H_2O$ (3 ml). The solution was filtered and the salts were washed with EtOAc and $Et_2O$. The filtrate was dried ($Na_2SO_4$), filtered and stripped of solvent to afford an oil. The oil was flashed (Merck $SiO_2$, 20% EtOAc in hexane) to provide the desired alcohol title compound (791 mg, 63%, 93% based on recovered ester) as a white solid and unreacted ester (454 mg). Analytically pure material was obtained by recrystallization from hot EtOAc/hexane.

m.p. 165.5-167 °C

TLC: $R_f$ 0.25 (20% EtOAc in hexane)

Microanalysis for $C_{22}H_{20}FNO * 0.12 H_2O$:

| | | | | | | | | |
|---|---|---|---|---|---|---|---|---|
| Calc'd: | C | 78.74 | H | 6.08 | N | 4.17 | F | 5.66 |
| Found: | C | 78.74 | H | 6.15 | N | 4.17 | F | 5.64 |

D. 4-(4-Fluorophenyl)-2-(1-methylethyl)-5H-indeno[1,2-b]pyridine-3-carboxaldehyde

A -78 °C solution of oxalyl chloride (280 ul, 407 mg, 3.21 mmol) in $CH_2Cl_2$ (10 ml) was treated dropwise with a solution of dry DMSO (455 ul, 501 mg, 6.4 mmol) in $CH_2Cl_2$ (1.5 ml). After 15 minutes, a solution of the alcohol from part C (764 mg, 2.29 mmol) in THF (3 ml) was added dropwise to the above mixture. Twenty minutes after the addition, TEA (2 ml) was added and the mixture was stirred at -78 °C for 5 minutes and then warmed to room temperature. The mixture was diluted with $Et_2O$ and washed twice with $H_2O$ and once with brine. The organic layer was dried ($Na_2SO_4$), filtered, and stripped to give a solid residue. The residue was flashed (Merck $SiO_2$, 10% EtOAc in hexane) providing the slightly impure aldehyde title compound (705 mg). The solid was recrystallized from hot hexane to give the title compound (637 mg, 84%) as white crystals.

m.p. 142.5-143.7 °C

TLC: $R_f$ 0.49 (20% EtOAc in hexane)

E. 3-(2,2-Dibromoethenyl)-4(4-fluorophenyl)-2-(1-methylethyl)-5H-indeno [1,2-b]pyridine

A solution of carbon tetrabromide (940 mg, 2.83 mmol) in $CH_2Cl_2$ (2 ml) was added over a 4-minute period to a cold (-7 °C) solution of the aldehyde from part D (626 mg, 1.89 mmol) and triphenylphosphine (1.486 gm, 5.67 mmol) in $CH_2Cl_2$ (11 ml). After the addition was complete, the cooling bath was removed and the mixture was stirred at room temperature for 30 minutes. The solution was quenched with saturated $NaHCO_3$ and extracted twice with $CH_2Cl_2$. The combined organic layers were dried ($Na_2SO_4$), filtered and concentrated. The concentrate was chromatographed (flash, Merck $SiO_2$, 20% $CH_2Cl_2$ in hexane) to give the impure dibromide title compound. The product was rechromatographed to provide the title pure compound (632 mg, 69%) as a white foam.

TLC: $R_f$ 0.32 (10% EtOAc in hexane)

F. (S)-4-[[[4-(4-Fluorophenyl)-2-(1-methylethyl)-5H-indeno[1,2-b]pyridin-3-yl]ethynyl]methoxyphosphinyl]-3-[-[(1,1-dimethylethyl)diphenylsilyl]oxy]butanoic acid, methyl ester

A solution of the dibromide from part E (622 mg, 1.28 mmol) in THF (8 ml) at -78 °C was treated with n-BuLi (2.5 M in hexane, 1.2 ml, 3 mmol) over a 1-minute period and the resulting deep purple-blue solution was stirred at -78 °C for 1 hour. The acetylenic anion solution was added dropwise via cannula over a 2-minute period to a -78 °C solution of the phosphonochloridate from Example 57, part G in THF (8 ml). The resulting mixture was stirred at -78 °C for 30 minutes, then quenched with 50% saturated $NH_4Cl$. The solution was warmed to 0 °C and poured into saturated $NaHCO_3$. The aqueous phase was extracted once with $Et_2O$. The $Et_2O$ layer was washed with brine, dried ($Na_2SO_4$), filtered and stripped to give an oil. The residue was chromatographed (flash, Merck $SiO_2$, 40% EtOAc in hexane) to afford the title compound as a colorless foam (305 mg, 31%).

TLC: $R_f$ 0.25 (40% EtOAc in hexane)

G. (S)-4-[[[4-(4-Fluorophenyl)-2-(1-methylethyl)-5H-indeno[1,2-b]pyridin-3-yl]ethynyl]methoxyphosphinyl]-3-hydroxy butanoic acid, methyl ester

A mixture of the compound of part F (304 mg, 0.40 mmol), tetra-n-butylammonium fluoride (1.0 M in THF, 1.6 ml, 1.6 mmol), and HOAc (115 ul, 121 mg, 2 mmol) in THF (8ml) was stirred at room temperature for 20 hours. The solution was diluted with EtOAc and washed three times with 5% $KHSO_4$. The aqueous layers were back-extracted once with EtOAc and the pooled EtOAc layers were dried ($Na_2SO_4$), filtered and stripped to afford a yellow oil. The oil was dissolved in $Et_2O$, cooled to 0 °C and treated with excess diazomethane for 10 minutes. The excess diazomethane was destroyed by the addition of HOAc and the solvent was removed *in vacuo*. The residue was chromatographed (flash, Merck $SiO_2$, 40% acetone in hexane) to afford the title compound (165 mg, 79%) as a yellow foam.

TLC: $R_f$ 0.31 (1:1 - acetone:hexane)

H. (S)-4-[[[4-(4-Fluorophenyl)-2-(1-methylethyl)-5H-indeno[1,2-b]pyridin-3-yl]ethynyl]hydroxyphosphinyl]-3-hydroxybutanoic acid, dilithium salt

A solution of the compound of part G (164 mg, 0.314 mmol) in dioxane (3 ml) was treated with 1 N LiOH (1.1 ml, 1.1 mmol) at room temperature and the mixture was subsequently heated at 65 °C under argon for 2 hours. The solvent was evaporated and the residue was chromatographed on HP-20, eluting in succession with $H_2O$ (200 ml), 25% MeOH in $H_2O$ (100 ml), and 50% MeOH in $H_2O$ (200 ml). The desired fractions were pooled and evaporated and the residue was taken up in $H_2O$ and lyophilized to give Example 1 (124mg, 75%) as a pale yellow solid.

TLC: $R_f$ 0.40 (7:2:1- i-propanol:$NH_4OH$:$H_2O$)

| | | | | | | |
|---|---|---|---|---|---|---|
| Anal. Calc'd for $C_{27}H_{23}FLi_2NO_5P$ * 1.31 $H_2O$: | | | | | | |
| | C 61.30 | H 4.88 | N 2.65 | F 3.59 | P 5.85 | |
| Found: | C 61.29 | H 4.74 | N 2.66 | F 3.57 | P 5.72 | |

Example 2

(S)-4-[[[6-(1,3-Benzodioxol-4-yl)-4-(4-fluorophenyl)-2-(1-methylethyl)-3-pyridinyl]ethynyl]hydroxyphosphinyl]-3-hydroxybutanoic acid, dilithium salt

A. 1,3-Benzodioxole-4-carboxaldehyde

A mixture of 2,3-dihydroxybenzaldehyde (25.0 g, 180 mmol; from Aldrich Chemical Co.) and potassium fluoride (49.3 g, 830 mmol) in DMF (500 ml) was heated at 50 °C for 20 minutes. The reaction mixture was cooled to room temperature and dibromomethane (44.5 g, 250 mmol) was added and the dark reaction mixture was heated at 120 °c for 3 hours. After filtration of the cooled mixture, the filtrate was poured into 1 liter $H_2O$ and extracted with ether (2x 200 ml). The combined ether extracts were washed with $H_2O$ (2x 200 ml), 0.5 N NaOH (2x 200 ml) and brine, then dried ($MgSO_4$) and concentrated, yielding the title compound I as a yellow oil (19.8 g, 73.0%).

$R_f$ 0.56 (40% EtOAc/hexane), UV

B. α-Methyl-1,3-benzodioxole-4-methanol

A -78 °C solution of the compound of part A (19.0 g, 126.6 mmol) in ether (400 ml) was treated with methylmagnesium bromide (3 M in ether, 55.0 ml, 165 mmol; from Aldrich Chemical Co.) over a 30-minute period using a syringe pump. After stirring for 1 hour the reaction mixture (heavy suspension) was carefully poured into saturated $NH_4Cl$ (250 ml). The layers were separated and the aqueous layer was extracted with ether (three times). The combined ether extracts were washed with $H_2O$ and brine, then dried ($MgSO_4$) and concentrated. The oily residue was purified by flash chromatography (Merck Silica gel, 10% EtOAc/hexane). The title compound was obtained as a colorless oil which slowly solidified on standing (13.3 g, 63.6%).

m.p. 45 - 46 °C

$R_f$ 0.39 ( 40% EtOAc/hexane), UV

| | | |
|---|---|---|
| Anal. Calc'd for $C_9H_{10}O_3$: | | |
| Calc'd: | C, 65.05; | H, 6.07; |
| Found: | C, 64.71; | H, 6.00 |

C. 1-(1,3-Benzodioxol-4-yl-1-ethanone

A -78° C solution of oxalyl chloride (8.68 ml, 99.6 mmol) in $CH_2Cl_2$ (120 ml) was treated dropwise with a solution of dry DMSO (14.2 ml, 199.2 mmol) in $CH_2Cl_2$ (20 ml). After 15 minutes, a solution of the alcohol from part B (13.5 g, 83.0 mmol) in $CH_2Cl_2$ (25 ml) was added dropwise to the above solution. After 20 minutes, triethylamine (34.7 ml) was added and the mixture was stirred at -78° C for 20 minutes and then warmed to room temperature. The mixture was diluted with ether and washed twice with $H_2O$ and brine, then dried ($MgSO_4$), filtered and concentrated under reduced pressure. The solid residue was recrystallized from hot hexane. The title compound was obtained as white crystals (13.5 g, 82.8%).

m.p. 94 - 95 °C
$R_f$ 0.49 (30% EtOAc/hexane), UV

Anal. Calc'd for $C_9H_8O$:

| | | |
|---|---|---|
| Calc'd: | C, 65.87; | H, 4.91; |
| Found: | C, 65.54; | H, 4.75 |

D. 1-(1,3-Benzodioxol-4-yl)-3-(4-fluorophenyl)-2-propen-1-one

A mixture of 4-fluorobenzaldehyde (8.78 ml, 81.8 mmol, from Aldrich Chemical Co.) and the compound of part C (13.4 g, 81.8 mmol) in absolute ethanol (110 ml) was treated with a solution of sodium ethoxide in ethanol (21% by weight solution; 3.10 ml, 8.18 mmol). A precipitate soon fell out of solution. After stirring at room temperature for 18 hours, the mixture was cooled to -10 °C and the precipitate was collected by filtration. The solid was washed with cold ethanol and dried *in vacuo* and recrystallized fom hot hexane. The title compound was obtained as a light yellow solid (12.5 g, 56.6%).

m.p. 248 - 250 °C
$R_f$ 0.68 (30% EtOAc/hexane), UV

Anal. Calc'd for $C_{16}H_{11}FO_3$ x 0.18 $H_2O$:

| | | | |
|---|---|---|---|
| Calc'd: | C, 70.27; | H, 4.19; | F, 6.95; |
| Found: | C, 70.27; | H, 3.87; | F, 6.65 |

E. β-(4-Fluorophenyl)-α-(2-methyl-1-oxopropyl)-Δ-oxo-1,3-benzodioxol-4-yl pentanoic acid, ethyl ester

A slurry of the compound of part D (12.5 g, 46.25 mmol) and ethyl isobutyrylacetate (7.46 ml, 46.25 mmol) in absolute ethanol (120 ml) was treated with a solution of sodium ethoxide in absolute EtOH (21% by weight solution, 1.70 ml, 4.62 mmol). A white precipitate soon fell out of solution. After stirring at room temperature overnight, the mixture was cooled to -10 °C and the precipitate was collected by filtration. The solid was washed with cold ethanol, dried in vacuo and recrystallized from hot hexane. The title compound was obtained as a white solid (14.6g, 75.0 %).

m.p. 109 - 110 °C
$R_f$ 0. 56 (30% EtOAc/hexane), UV

Anal. Calc'd for $C_{24}H_{25}FO_6$ x 0.19 $H_2O$:

| | | | |
|---|---|---|---|
| Calc'd: | C, 66.75; | H, 5.92; | F, 4.40; |
| Found: | C, 66.75; | H, 5.65; | F, 4.36 |

F. 6-(1,3-Benzodioxol-4-yl)-4-(4-fluorophenyl)-2-(1-methylethyl)-3-pyridinecarboxylic acid, ethyl ester

A mixture of the compound of part E (13.5 g, 31.5 mmol), ammonium acetate (7.28 g, 94.5 mmol) and copper (II) acetate (15.70 g, 78.75 mmol) in glacial acetic acid (90 ml) was heated at 110 °C for 18 hours (TLC indicated the absence of starting material). The solution was cooled to room temperature and poured into an ice cold mixture of concentrated $NH_4OH$ (110 ml) and $H_2O$ (150 ml). The mixture was extracted with ether and the ether extract was washed twice with $H_2O$ and brine, then dried ($MgSO_4$), filtered and concentrated to yield a yellow solid residue. The crude material was recrystallized from hot hexane, yielding the title compound as a pale yellow solid (7.52 g, 58.3%).

m.p. 148 - 150 °C

$R_f$ 0. 62 (30% EtOAc/hexane), UV

Anal. Calc'd for $C_{24}H_{22}NFO_4$:

| | | | | |
|---|---|---|---|---|
| Calc'd: | C, 70.75; | H, 5.44; | N, 3.44; | F, 4.66; |
| Found: | C, 70.92; | H, 5.53; | N, 3.49; | F, 4.62 |

G. 6-(1,3-Benzodioxol-4-yl)-4-(4-fluorophenyl)-2-(1-methylethyl)-3-pyridinemethanol

A cold (0 °C) solution of the ester from part F (7.0 g, 17.05 mmol) in dry THF (85 ml) was treated with $LiAlH_4$ (1.94 g, 51.15 mmol). Ten minutes after the addition, cooling bath was removed and the reaction mixture was stirred at room temperature for 3 hours (TLC indicated the absence of starting material). The dark mixture was cooled to 0 °C and, while stirring, carefully quenched first by a dropwise addition of 1.94 ml of $H_2O$ in THF (15 ml), then 1.94 ml 15 % NaOH and finally 4.0 ml $H_2O$. The precipitated aluminum salts were filtered and washed with EtOAc and ether. The filtrate was washed with $H_2O$ and brine, dried ($Na_2SO_4$) filtered and concentrated under reduced pressure. The solid residue was recrystallized from hot EtOAc/hexane, yielding the title compound as an off-white solid (6.0 g, 96.7%).

m.p. 114 - 115 °C

$R_f$ 0.44 (30% EtOAc/hexane), UV

Anal. Calc'd for $C_{22}H_{20}NFO_3$:

| | | | | |
|---|---|---|---|---|
| Calc'd: | C, 72.31; | H, 5.52; | N, 3.83; | F, 5.20; |
| Found: | C, 72.14; | H, 5.40; | N, 3.83; | F, 5.07 |

H. 6-(1,3-Benzodioxol-4-yl)-4-(4-fluorophenyl)-2-(1-methylethyl)-3-pyridinecarboxaldehyde

A -78 °C solution of oxalyl chloride (0.57 ml, 6.57 mmol) in $CH_2Cl_2$ (80 ml) was treated dropwise with a solution of dry DMSO (0.93 ml, 13.12 mmol) in $CH_2Cl_2$ (2 ml). After 15 minutes, a solution of the alcohol from part G (2.0g, 5.47 mmol) in $CH_2Cl_2$ (15 ml) was added dropwise to the above solution. After 20 minutes, triethylamine (4.57 ml) was added and the mixture was stirred at -78 °C for 20 minutes and then warmed to room temperature. The mixture was diluted with ether and washed twice with $H_2O$ and brine, dried ($MgSO_4$), filtered and concentrated under reduced pressure. The yellow solid residue was recrystallized from hot hexane. The title compound (two crops) was obtained as a pale yellow solid (1.65 g, 83.3%).

m.p. 121 - 122 °C

$R_f$ 0.57 (30% EtOAc/hexane), UV

Anal. Calc'd for $C_{22}H_{18}NFO_3$:

| | | | | |
|---|---|---|---|---|
| Calc'd: | C, 72.71; | H, 4.99; | N, 3.86; | F, 5.23; |
| Found: | C, 72.40; | H, 5.05; | N, 3.73; | F, 5.33 |

I. 6-(1,3-Benzodioxol-4-yl)-3-(2,2-dibromoethenyl)-4-(4-fluorophenyl)-2-(1-methylethyl)pyridine

A solution of carbon tetrabromide (3.28 g, 6.6 mmol) in $CH_2Cl_2$ (15 ml) was added dropwise over a 5-minute period to a cold (0 °C) solution of the aldehyde from part H (1.60 g, 4.4 mmol) and triphenylphosphine (3.46 g, 13.2 mmol,) in $CH_2Cl_2$ (60 ml). The mixture was stirred at 0 °C for 10 minutes and then at room temperature for 10 minutes. The solution was quenched with saturated $NaHCO_3$ and extracted twice with $CH_2Cl_2$. The combined organic layers were dried ($Na_2SO_4$), filtered, concentrated to half volume and applied on Merck silica gel column (50% $CH_2Cl_2$/hexane). Flash chromatography afforded the title compound as a colorless viscous oil·which slowly solidified on standing (1.65 g, 89.0%).

m.p. 129 - 131 °C

$R_f$ 0.63 (30% EtOAc/hexane), UV

Anal. Calc'd for $C_{23}H_{18}NO_2FBr_2$

| | |
|---|---|
| Calc'd: | C 53.20; H 3.49; N 2.70; F 3.66; Br 30.78; |
| Found: | C 53.14; H 34.5; N 2.66; F 3.49; Br 30.47 |

J. (S)-4-Chloromethoxyphosphonyl-3-[[(1,1-dimethylethyl)diphenylsilyl]oxy] butanoic acid, methyl ester

(S)-4-(Hydroxymethoxyphosphinyl)-3-[[(1,1-dimethylethyl)diphenylsilyl]oxy]butanoic acid, methyl ester, dicyclohexylamine (1:1) salt (3.88 g, 6.15 mmol was partitioned between EtOAc and 5% $KHSO_4$. The EtOAc layer was washed (3 x 5% $KHSO_4$. The EtOAc layer was washed (3 x 5% $KHSO_4$, then with brine), dried ($Na_2SO_4$), filtered and concentrated under reduced pressure to afford the phosphinic acid monomethyl ester as a colorless oil. The oil was dissolved in dry $CH_2Cl_2$ (15 ml) and treated with N,N-diethyltrimethylsilylamine (2.33 ml, 12.3 mmol). After stirring at room temperature for 1 hour, the solvent was removed in vacuo and the residue was azeotroped with dry benzene (30 ml). The residue was redissolved in dry $CH_2Cl_2$ (15 ml), cooled to 0 °C and treated with 2 drops of DMF and oxalyl chloride (610 µl, 7.0 mmol). After 15 minutes, the solution was warmed to room temperature and stirred for an additional 60 minutes. The solvent was removed under reduced pressure and the yellow residue (the phosphonochloridate compound J) was azeotroped with dryl benzene and dried in vacuo for 1 hour.

K. (S)-4-[[[6-(1,3-Benzodioxol-4-yl)-4-(4-fluorophenyl)-2-(1-methylethyl)-3-pyridinyl]ethynyl]-methoxyphosphinyl]-3-[[(1,1-dimethylethyl)diphenylsilyl]oxy]butanoic acid, methylester

A solution of the dibromide of part I (1.68 g, 3.17 mmol) in THF (10 ml) at -78 °C was treated with n-BuLi (2.5 M in hexane, 2.53 ml, 6.34 mmol) over a 1-minute period. The dark-green reaction mixture was stirred for 1 hour at -78 °C and then transferred via cannula to a -78 °C solution of the phosphonochloridate prepared in part J above in THF (10 ml). The green-brown reaction mixture was stirred at -78 °C for 30 minutes and quenched with 50% saturated $NH_4Cl$. After warming to room temperature, the solution was diluted with $H_2O$ and poured into saturated $NaHCO_3$. The layers were separated and the aqueous layer was extracted once with ether. The combined organic layers were washed with brine, dried ($Na_2SO_4$), filtered and concentrated under reduced pressure. The orange oily residue was purified by flash chromatography (Merck silica gel, 25% EtOAc/hexane) to afford the title compound as a pale yellow foam (0.68 9, 27%).

$R_f$ 0.31 (40% EtOAc/hexane), UV

L. (S)-4-[[[6-(1,3-Benzodioxol-4-yl)-4-(4-fluorophenyl)-2-(1-methylethyl)-3-pyridinyl]ethynyl]-methoxyphosphinyl]-3-hydroxybutanoic acid, methylester

To a solution of the acetylinic phosphonate of part K (0.94 g, 1.18 mmol) in THF (15 m) was added HOAc (0.27 ml, 4.72 mmol), followed by tetra-n-butylammonium fluoride (1.0 M in THF, 3.54 ml, 3.54 mmol). The reaction mixture was stirred at room temperature under argon for 20 hours. The solution was diluted with EtOAc and washed 3x 5 % $KHSO_4$ The aqueous layer was back-extracted twice with EtOAc

and the combined organic extracts were dried ($Na_2SO_4$), filtered and concentrated under reduced pressure. The yellow-orange oil was dissolved in ether (a few drops of THF were added to obtain a clear solution), cooled to 0 °C and treated with excess diazomethane for 30 minutes. The excess diazomethane was destroyed by the addition of HOAc. Solvent removal gave an orange oily residue, which was purified by flash chromatography (Merck silica gel, 40% acetone/hexane). The title compound was obtained as a colorless oil (0.24 g, 37.0%).

$R_f$ 0.30 (50% acetone/hexane) UV

M. (S)-4-[[[6-(1,3-Benzodioxol-4-yl)-4-(4-fluorophenyl)-2-(1-methylethyl)-3-pyridinyl]ethynyl]-hydroxyphosphinyl]-3-hydroxybutanoic acid, dilithium salt

The diester from part L (0.24 g, 0.43 mmol) in dioxane (5 ml) was treated with 1 N LiOH (1.51 ml, 1.51 mmol) at room temperature and then heated at 50 °C for 2.5 hours (TLC indicated the absence of starting material). The solvent was evaporated and the residue was chromatographed on HP-20 resin eluting first with $H_2O$ (200 ml), followed by 25% $MeOH/H_2O$ (200 ml), 50% $MeOH/H_2O$ (250 ml) and finally MeOH (100ml). The collected product fractions were evaporated, dissolved in $H_2O$, filtered, frozen and lyophilized to give Example 2 as a white lyophilate (0.22 g, 95.0%).

$R_f$ 0.55 (8:1:1 - $CH_2Cl_2$: $CH_3OH$ : HOAc), UV

Anal. Calc'd for $C_{27}H_{23}FLi_2NO_7$ x 1.24 $H_2O$

| | | | | |
|---|---|---|---|---|
| Calc'd: | C, 57.65; | H, 4.62; | N, 2.49; | F, 3.38; |
| | P, 5.51; | | | |
| Found: | C, 57.34; | H, 4.50; | N, 2.89; | F, 3.30; |
| | P, 5.41. | | | |

Example 3

(S)-4-[[[4-(4-Fluorophenyl)-2-(1-methylethyl)-benzo[6,7]cyclohepta[1,2-b]pyridin-3-yl]ethynyl]-hydroxyphosphinyl]-3-hydroxybutanoic acid, disodium salt

A. β-(4-Fluorophenyl)-6,7,8,9-tetrahydro-α-(2-methyl-1-oxopropyl)-5-oxo-5H-benzocycloheptene-6-propanoic acid, ethyl ester

A -78 °C solution of $LiN(TMS)_2$ (1.0 M in THF, 27.5 ml, 27.5 mmol) and THF (15 ml) was treated with a solution of benzosuberone (6.000 gm, 22.7 mmol) in THF (3 ml) over a 1-minute period. After 1 hour, the ester from Example 1, part A, first paragraph (4.08 g, 15.4 mmol) in THF (3 ml) was added dropwise to the above solution. The mixture was stirred at -78 °C for 30 minutes and then warmed to 0 °C. After 20 minutes, the mixture was quenched with HOAc (3.4 ml), diluted with saturated $NH_4Cl$ and $H_2O$ and subsequently extracted with $Et_2O$. The $Et_2O$ extract was washed with brine, then dried ($Na_2SO_4$), filtered, and stripped to yield a viscous pale yellow gum. The material was used directly in the next reaction.

B. 4-(4-Fluorophenyl)-6,7-dihydro-2-(1-methylethyl)-benzo[6,7]cyclohepta[1, 2-b]pyridine-3-carboxylic acid, ethyl ester

The aforementioned crude mixture of the compound of part A, $NH_4OAc$ (5.25 gm, 68.1 mmol), and $Cu(OAc)_2$ (11.33 gm, 57 mmol) in glacial HOAc (60 ml) was gently refluxed for 19 hours. The solution was cooled to room temperature and subsequently poured into an ice cold mixture of concentrated $NH_4OH$ (100 ml) in $H_2O$ (200 ml). The mixture was extracted once with $Et_2O$ the organic extract was washed with $H_2O$ and brine, then dried ($Na_2SO_4$), filtered and stripped to yield a dark red-purple oil. The residue was flashed (Merck $SiO_2$, 10% EtOAc in hexane) to give the impure title compound as an oil (6.707 gm, estimated purity of 90%).

TLC: $R_f$ 0.53 (20% EtOAc in hexane)

C. 4-(4-Fluorophenyl)-6,7-dihydro-2-(1-methylethyl)-benzo[6,7]cyclohepta[1, 2-b]pyridine-3-methanol

A cold (0 °C) solution of the impure ester of part B (6.692 gm) in dry THF (200 ml) was treated with $LiAlH_4$ (1.910 gm, 50 mmol). The ice bath was removed and the mixture was stirred at room temperature for 3.5 hours. The solution was then cooled to 0 °C and quenched in succession with $H_2O$ (2 ml), 10% NaOH (2 ml), and $H_2O$ (6 ml). The solution was filtered and the salts were washed with EtOAc and $Et_2O$. The filtrate was stripped of solvent to afford a gum, which was triturated with hexane to produce a solid. The solid was recrystallized twice from hot EtOAc/hexane to provide the title compound (3.800 gm, 68% from compound A from Example 53) as a white solid.

m.p. 160.5-161.8 °C
TLC: $R_f$ 0.24 (20% EtOAc in hexane)

D. 4-(4-Fluorophenyl)-6,7-dihydro-2-(1-methylethyl)-benzo[6,7]cyclohepta[1, 2-b]pyridine-3-carboxaldehyde

A -78 °C solution of oxalyl chloride (630 ul, 917 mg, 7.2 mmol) in $CH_2Cl_2$ (40 ml) was treated dropwise with a solution of dry DMSO (1.10 ml, 1.21 gm, 15.5 mmol) in $CH_2Cl_2$ (1 ml). After 10 minutes, a solution of the alcohol from part C (2.000 gm, 5.5 mmol) in THF (5 ml) was added dropwise to the above mixture. Fifteen minutes after the addition, TEA (4.6 ml) was added and the mixture was stirred at -78 °C for 5 minutes and then warmed to room temperature. The mixture was diluted with $Et_2O$ and washed twice with $H_2O$ and once with brine. The organic layer was dried ($Na_2SO_4$), filtered, and stripped to give a yellow oil, which solidified upon cooling to -78 °C in hexane. The mixture was then crystallized from hot hexane to give the aldehyde title compound (1.775 gm, 89%) as white needles.

m.p. 132-134 °C
TLC: $R_f$ 0.54 (20% EtOAc in hexane)

E. (3-(2,2-Dibromoethenyl)-4-(4-Fluorophenyl)-6,7-dihydro-2-(1-methylethyl)-benzo[6,7]cyclohepta[1,2-b]-pyridine

A solution of carbon tetrabromide (2.336 gm, 7.0 mmol) in $CH_2Cl_2$ (6 ml) was added over a 7-minute period to a cold (0 °C) solution of the aldehyde from part D (1.688, 4.7 mmol) and triphenylphosphine (3.698 gm, 14.1 mmol) in $CH_2Cl_2$ (20 ml). After the addition was complete, the cooling bath was removed and the mixture was stirred at room temperature for 25 minutes. The solution was quenched with saturated $NaHCO_3$ and extracted twice with $CH_2Cl_2$. The organic layers were dried ($Na_2SO_4$), filtered and concentrated. The concentrate was chromatographed (flash, Merck $SiO_2$, 40% $CH_2Cl_2$ in hexane) to give the dibromide title compound as a solid. Recrystallization of the material from hot EtOAc/hexane provided the pure title compound (2.257 gm, 93%) as a white solid.

m.p. 173-175 °C
TLC: $R_f$ 0.44 (10% EtOAc in hexane)

Microanalysis for $C_{25}H_{22}Br_2FN$:

| | | | | | |
|---|---|---|---|---|---|
| Calc'd: | C 58.27 | H 4.30 | N 2.72 | F 3.69 | Br 31.02 |
| Found: | C 58.27 | H 4.29 | N 2.69 | F 3.62 | Br 31.35 |

F. (S)-4-[[[4-(4-fluorophenyl)-2-(1-methylethyl)-benzo[6,7]cyclohepta[1,2-b]pyridin-3-yl]ethynyl]-methoxyphosphinyl]-3-[[(1,1-dimethylethyl)diphenylsilyl]-oxy]butanoic acid, methyl ester

A solution of the dibromide from part E (2.000 gm, 3.88 mmol) in THF (10 ml) at -78 °C was treated with n-BuLi (2.5 M in hexane, 3.3 ml, 8.2 mmol) over a 1-minute period, and the resulting clear green solution was stirred at -78 °C for 50 minutes. The acetylenic anion solution was added dropwise via cannula over a 10-minute period to a -78 °C solution of the phosphonochloridate from Example 2, part J in THF (12 ml). The resulting mixture was stirred at -78 °C for 30 minutes, then quenched with 50% saturated $NH_4Cl$. The solution was warmed to 0 °C and poured into saturated $NaHCO_3$. The aqueous phase was extracted once with $Et_2O$. The $Et_2O$ layer was washed with brine, dried ($Na_2SO_4$), filtered and stripped to give an oil. The residue was chromatographed (flash, Merck $SiO_2$, 40% EtOAc in hexane) to afford the title compound as a colorless foam (2.517 gm, 82%).

TLC: $R_f$ 0.31 (40% EtOAc in hexane)

G. (S)-4-[[[4-(4-fluorophenyl)-2-(1-methylethyl)-benzo[6,7]cyclohepta[1,2-b]pyridin-3-yl]ethynyl]-methoxyphosphinyl]-3-hydroxybutanoic acid, methyl ester

A mixture of the part F compound (2.487 gm, 3.15 mmol), tetra-n-butylammonium fluoride (1.0 M in THF, 11.0 ml, 11.0 mmol), and HOAc (810 ul, 850 mg, 14.1 mmol) in THF (40 ml) was stirred at room temperature for 18 hours. The solution was diluted with EtOAc and washed three times with 5% $KHSO_4$ and once with brine. The EtOAc layer was dried ($Na_2SO_4$), filtered and stripped to afford a yellow oil. The oil was dissolved in $Et_2O$, cooled to 0 °C and treated with excess diazomethane for 10 minutes. The excess diazomethane was destroyed by the addition of HOAc and the solvent was removed *in vacuo*. The residue was chromatographed (flash, Merck $SiO_2$, 40% acetone in hexane) to afford the title compound (1.534 gm, 89%) as a colorless foam.

TLC: $R_f$ 0.38 (1:1 - acetone:hexane)

H. (S)-4-[[[4-(4-Fluorophenyl)-2-(1-methylethyl)-benzo[6,7]cyclohepta[1,2-b]pyridin-3-yl]ethynyl]-hydroxyphosphinyl]-3-hydroxybutanoic acid, disodium salt

A solution of the part G compound (780 mg, 1.42 mmol) in dioxane (7 ml) was treated with 1 N NaOH (5.0 ml, 5.0 mmol) and the mixture was stirred at room temperature for 18 hours. The solvent was evaporated and the residue was chromatographed on HP-20, eluting in succession with $H_2O$ (200 ml), 50% MeOH in $H_2O$ (200 ml), and MeOH (100 ml). The desired fractions were pooled and evaporated and the residue was taken up in $H_2O$ and lyophilized to give Example 3 (744 mg, 90%) as a white solid.

TLC: $R_f$ 0.17 (8:1:1- $CH_2Cl_2$:HOAc:MeOH)

Analysis for $C_{29}H_{27}FNNa_2O_5P$ x 0.80 $H_2O$:

| | | | | | |
|---|---|---|---|---|---|
| | C 60.06 | H 4.97 | N 2.42 | F 3.28 | P 5.34 |
| Found: | C 59.98 | H 5.02 | N 2.50 | F 3.52 | P 5.55 |

Example 4

(S)-4-[[[6-(l-Adamantyl)-4-(4-fluorophenyl)-2-(l-methylethyl)-3-pyridinyl]ethynyl]hydroxyphosphinyl]-3-hydroxybutanoic acid, disodium salt

A. l-(l-Adamantyl)-3-(4-fluorophenyl)-2-propen-l-one

A mixture of 4-fluorobenzaldehyde (6.01 ml, 56.10 mmol, Aldrich) and 1-adamantyl methyl ketone (10.00 g, 56.10 mmol) in absolute ethanol (100 ml) was treated with a solution of sodium ethoxide in ethanol (21% by weight solution; 2.08 ml, 5.61 mmol). A precipitate soon fell out of solution. After stirring at room temperature for 48 hours, the mixture was cooled to -10 °C and the precipitate was collected by filtration. The solid was washed with cold ethanol, dried in vacuo and recrystallized from hexane. The title compound was obtained as an off-white solid (9.20 g, 57.8%).

m.p. 126 - 127 °C

$R_f$ 0.55 (25% EtOAc/hexane), UV

Analysis for $C_{19}H_{21}FO$:

| | | | |
|---|---|---|---|
| Calc'd | C, 80.25; | H, 7.44; | F, 6.68 |
| Found | C, 79.43; | H, 7.54; | F, 6.94 |

B. (4-Fluorophenyl)-α-(2-methyl-l-oxopropyl)-Δ-oxo-l-adamantanepentanoic

acid, ethyl ester A mixture of the part A compound (9.10 g, 32.00 mmol) and ethyl isobutyrylacetate (7.75ml, 48.00 mmol) in absolute ethanol (120 ml) was treated with a solution of sodium ethoxide in ethanol

(21% by weight solution, 5.95 ml, 16.00 mmol). The reaction mixture was stirred for 48 hours. The obtained heavy suspension was cooled to -10 °C, filtered and the off-white solids were washed with cold EtOH. The crude product was recrystallized from hot hexane, affording the title compound as an off-white solid (10.6 g, 74.8%, mixture of diastereomers).

m.p. 104 -105 °C

$R_f$ 0.42 + 0.44 (20% EtOAc/hexane), UV

Analysis for $C_{27}H_{35}FO_4$:

| | | | |
|---|---|---|---|
| Calc'd | C, 73.27; | H,7.97; | F, 4.29 |
| Found | C, 72.87; | H,7.93; | F, 4.25 |

C. l-(l-Adamantyl)-4-(4-fluorophenyl)-2-(l-methylethyl)-3-pyridinecarboxylic acid, ethyl ester

A mixture of the part B compound (10.50 g, 23.70 mmol), ammonium acetate (5.48 g, 71.10 mmol) and copper (II) acetate (11.83 g, 59.25 mmol) in glacial acetic acid (75 ml) was heated at 110 °C for 16 hours. The solution was cooled to room temperature and poured into an ice cold mixture of concentrated $NH_4OH$ (100 ml) and $H_2O$ (150 ml). The mixture was extracted with ether (twice) and the ether extracts were washed with $H_2O$ (twice) and brine, then dried ($MgSO_4$), filtered and concentrated into an oily residue that became a white hard foam upon drying in vacuo (high) (10.0 g, 100.0%). The crude title compound was used directly for the next reaction.

$R_f$ 0.61 (20% EtOAc/hexane), UV

D. l-(l-Adamantyl)-4-(4-fluorophenyl)-2-(l-methylethyl)-3-pyridinemethanol

A cold (0 °C) solution of the ester from part C (10.0 g, 23.70 mmol) in dry THF (100 ml) was treated with $LiAlH_4$ (2.70 g, 71.17 mmol). Ten minutes after the addition the cooling bath was removed and the reaction mixture stirred at room temperature for 2.5 hours. The reaction mixture was cooled to 0 °C and while stirring, carefully quenched by the dropwise addition of 2.7 ml of $H_2O$ in THF (15 ml), then 2.7 ml of 15% NaOH and finally 5.4 ml $H_2O$. The precipitated aluminum salts were filtered and washed with EtOAc and ether. The filtrate was washed with $H_2O$ and brine, dried ($Na_2SO_4$), filtered and concentrated under reduced pressure to give an oily residue. Purification by flash chromatography (Merck silica gel, 5% EtOAc/hexane) yielded the title compound as a white solid, which was recrystallized from hot hexane (6.7 g, 74.6%).

m.p. 143 -145 °C

$R_f$ 0.34 (20% EtOAc/hexane), UV

Analysis for $C_{25}H_{30}NFO$:

| | | | | |
|---|---|---|---|---|
| Calc'd | C, 79.12; | H, 7.97; | N, 3.69; | F, 5.01 |
| Found | C, 79.03; | H, 8.25; | N, 3.77; | F, 4.90 |

E. l-(l-Adamantyl)-4-(4-fluorophenyl)-2-(l-methylethyl)-3-pyridinecarboxaldehyde

A -78 °C solution of oxalyl chloride (1.85 ml, 21.21 mmol) in $CH_2Cl_2$ (150 ml) was treated dropwise with a solution of dry DMSO (3.01 ml, 42.40 mmol) in $CH_2Cl_2$ (25 ml). After 15 minutes, a solution of the alcohol from part D (6.70 g, 17.67 mmol) in $CH_2Cl_2$ (50 ml) was added dropwise to the above solution. After 20 minutes, triethylamine (14.78 ml, 106.02 mmol) was added and the mixture was stirred at -78 °C for 20 minutes and then warmed to room temperature. After 1.5 hours, the reaction was quenched with $H_2O$ and diluted with ether. The layers were separated and aqueous layer was extracted with ether (twice). The combined organic extracts were washed with $H_2O$ (twice) and brine, dried ($MgSO_4$), filtered and concentrated under reduced pressure. The oily residue was purified by flash chromatography (Merck silica gel, 5% EtOAc/hexane) to give the aldehyde title compound as a white solid. The solid was recrystallized from hexane yielding the title compound as white crystals (5.5 g, 83.5%).

m.p. 109 -110 °c
$R_f$ 0.66 (20% EtOAc/hexane), UV

| | | | | |
|---|---|---|---|---|
| Analysis for $C_{25}H_{28}NFO$: | | | | |
| Calc'd | C, 79.54; | H, 7.48; | N,3.71; | F, 5.03 |
| Found | C, 79.25; | H, 7.32; | N,3.61; | F, 4.80 |

F. l-(l-Adamantyl)-3-(2,2-dibromoethenyl)-2-(l-methylethyl)pyridine

A solution of carbon tetrabromide (2.63 g, 7.95 mmol) in $CH_2Cl_2$ (10 ml) was added dropwise over a 5-minute period to a cold (0° C) solution of the aldehyde from part E (2.00 g, 5.30 mmol) and triphenylphosphine (4.17 g, 15.90 mmol) in $CH_2Cl_2$ (60 ml). The mixture was stirred at 0 °C for 10 minutes and then at room temperature for 30 minutes. The solution was quenched with saturated $NaHCO_3$ and extracted twice with $CH_2Cl_2$. The combined organic layers were dried ($Na_2SO_4$), filtered, concentrated to 1/3 volume and applied on Merck silica gel column (50% $CH_2Cl_2$/hexane). Flash chromatography afforded the title compound as a white solid, which was recrystallized from hexane (2.3 g, 82.1%).
m.p. 176 -177 °C
$R_f$ 0.65 (15% EtOAc/hexane), UV

G. (S)-4-[[[6-(l-Adamantyl)-4-(4-fluorophenyl)-2-(l-methylethyl)-3-pyridinyl]ethynyl]methoxyphosphinyl]-3-[[(l,l-dimethylethyl)diphenylsilyl]oxy]butanoic acid, methyl ester

A solution of the dibromide from part F (2.2 g, 4.13 mmol) in THF (18 ml) at -78° C was treated with n-Buli (2.5 M in hexane, 3.46 ml, 8.67 mmol) over a 1 minute period. The green-blue reaction mixture was stirred for 45 minutes at -78° C and then transferred via cannula to -78° C solution of the phosphonochloridate from Example 2, part J in THF (10 ml). The green-brown reaction mixture was stirred at -78° C for 30 minutes and quenched with 50% saturated $NH_4Cl$. After warning to room temperature, the solution was diluted with $H_2O$ and poured into saturated $NaHCO_3$. The layers were separated and the aqueous layer was extracted once with ether. The combined organic layers were washed with brine, dried ($Na_2SO_4$), filtered and concentrated under reduced pressure. The dark oily residue was purified by flash chromatography (Merck silica gel, 15% EtOAc/hexane) to afford the title compound as a colorless oil (1.95 g, 59.0%).
$R_f$ 0.36 (30% EtOAc/hexane), UV

H. (S)-4-[[[6-(l-Adamantyl)-4-(4-fluorophenyl)-2-(l-methylethyl)-3-pyridinyl]ethynyl]methoxyphosphinyl]-3-hydroxybutanoic acid, methyl ester

To a solution of the acetylinic phosphonate of part G (1.9 g, 2.36 mmol) in THF (15 mmol) was added HOAc (0.53 ml, 9.44 mmol), followed by tetra-n-butylammonium fluoride (1.0 M in THF, 7.08 ml, 7.08 mmol). The reaction mixture was stirred at room temperature under argon for 24 hours. The solution was diluted with EtOAc and washed three times with 5% $KHSO_4$. The aqueous layer was back-extracted twice with EtOAc and the combined organic extracts were dried ($Na_2SO_4$), filtered and concentrated under reduced pressure. The yellow-orange oil was dissolved in ether (added a few drops of THF to obtain a clear solution), cooled to 0 °C and treated with excess diazomethane in ether for 20 minutes. The excess diazomethane was destroyed by the addition of HOAc. Solvent removal gave a dark oily residue, which was purified by flash chromatography (Merck silica gel, 20% acetone/hexane). The title compound was obtained as a colorless oil (0.69 g, 60.0%).
$R_f$ 0.59 (40% acetone/hexane) UV

I. (S)-4-[[[6-(l-Adamantyl)-4-(4-fluorophenyl)-2-(l-methylethyl)-3-pyridinyl]ethynyl]hydroxyphosphinyl]-3-hydroxybutanoic acid, disodium salt

The diester from part H (0.69 g, 1.21 mmol) in dioxane (15 ml) was treated with 1 N NaOH (4.23 ml, 4.23 mmol) at room temperature and then heated at 50 °C for 2.5 hours. The reaction mixture became opaque and dioxane was added until a clear solution was obtained (30 ml). After heating for an additional 16 hours the reaction mixture again became opaque with oily droplets present. To obtain a clear solution,

MeOH (15 ml) was added and the solution was treated with additional 1 N NaOH (1.21ml) and heated for 4 hours. Solvent was evaporated and the residue was chromatographed on HP-20 resin, eluting first with $H_2O$ (300 ml), followed by 25% $MeOH/H_2O$ (300 ml), 50% $MeOH/H_2O$ (300 ml) and finally MeOH (100 ml). The collected product fractions were evaporated, dissolved in $H_2O$, filtered, frozen and lyophilized to yield Example 4 as a white lyophilate (3.30g, 48.0%).

$R_f$ 0.45 (8:1:1 - $CH_2Cl_2$: $CH_3OH$ HOAc), UV

Analysis for $C_{30}H_{33}NFPO_5Na_2$ x 2.0 $H_2O$:

| | |
|---|---|
| Calc'd | C,58.15; H,6.02; N,2.26; F, 3.07; P,5.00 |
| Found | C,58.24; H,5.77; N,2.11; F, 3.04; P,4.79 |

Example 5

(S)-4-[[[5-Fluoro-4-(4-fluorophenyl)-2-(l-methylethyl)-6-phenyl-3-pyridinyl]ethynyl]hydroxyphosphinyl]-3-hydroxybutanoic acid, disodium salt

A. 2-Fluoroacetophenone

A mixture of 2-bromoacetophenone (20.00 gm, 100 mmol) and KF (8.82 gm, 152 mmol) in dry DMF (35 ml) was heated at 100 °C for 1 hour. Additional KF (2.26 gm) was added and heating of the mixture continued for 4 more hours. The dark-red solution was cooled, poured into $Et_2O$ and washed with $H_2O$ (twice) and brine. The ethereal solution was dried ($Na_2SO_4$), filtered, and stripped to yield a dark red oil. The oil was distilled to collect a fraction boiling between 62-64 °C at 1 mm Hg which was 91 molar percent the title compound and 9 molar percent acetophenone (6.00 gm). Flash chromatography (Merck $SiO_2$, 10% EtOAc in hexane) afforded the pure title compound (5.50 gm, 45%) as a liquid that solidifies near room temperature.

TLC $R_f$ 0.28 (20% EtOAc in hexane)

B. Δ-Fluoro-β-(4-fluorophenyl)-α-(2-methyl-1-oxopropyl)-Δ-oxobenzenepentanoic acid, ethyl ester

A -78 °C solution of $LiN(TMS)_2$ (1.0 M in THF, 28.7 ml, 28.7 mmol) in dry THF (17 ml) was treated with a solution of 2-fluoroacetophenone (A) (3.500 g, 28.65 mmol) in 3 mL) THF. After 50 minutes, the ester from Example 1, part A, first paragraph (6.310 gm, 23.9 mmol) in THF (4 ml) was added dropwise to the above solution. After 1.25 hours, the red-orange mixture was quenched with saturated $NH_4Cl$ and warmed to room temperature. The mixture was diluted with $H_2O$ and subsequently extracted with $Et_2O$. The $Et_2O$ extract was washed with brine, dried ($Na_2SO_4$), filtered, and stripped to give a yellow oil containing the title compound in crude form.

C. 5-Fluoro-4-(4-fluorophenyl)-2-(l-methylethyl)-6-phenyl-3-pyridinecarboxylic acid, ethyl ester

A mixture of the above crude oil, $NH_4OAc$ (5.52 gm, 71.6 mmol), and $Cu(OAc)_2$ (16.77 gm, 84 mmol) in glacial HOAc (80 ml) was heated at 100 °C for 2 hours. Additional $NH_4OAc$ (2.5 gm) was added and the temperature of the reaction mixture was raised to 110 °C and heating continued for 16 hours. The solution was cooled to room temperature and subsequently poured into an ice cold mixture of concentrated $NH_4OH$ (90 ml) in $H_2O$ (120 ml). The mixture was extracted with $Et_2O$ and the $Et_2O$ extract was washed with $H_2O$ and brine, then dried ($Na_2SO_4$), filtered and stripped to yield a brown oil. The oil was flashed (Merck $SiO_2$, 5% EtOAc in hexane) to give the title compound as a colorless oil (1.190 gm, approx. 11%), which was contaminated with an undetermined amount of the non-5-fluorinated pyridine ester 4-(4-fluorophenyl)-2-(l-methylethyl)-6-phenyl-3-pyridinecarboxylic acid, ethyl ester.

TLC $R_f$ 0.49 (20% EtOAc in hexane)

D. 5-Fluoro-4-(4-fluorophenyl)-2-(l-methylethyl)-6-phenyl-3-pyridinemethanol

A cold (0 °C) solution of the ester from part C (purity uncertain, 2.875 gm, assume 7.5 mmol) in dry THF (80 ml) was treated with $LiAlH_4$ (990 mg, 26 mmol). After the addition, the temperature of the reaction

was raised to 25 °C and stirring of the mixture continued for 2 hours. The solution was recooled to 0 °C and quenched in succession with $H_2O$ (1 ml), 10% NaOH (1.5 ml), and $H_2O$ (3 ml). The solution was filtered and the salts were washed with EtOAc. The filtrate was stripped to yield a mixture of the title compound and the non-fluorinated pyridine alcohol (the title compound where F on the pyridine ring is replaced with H) as a solid that could not be purified by recrystallization. The solid was dissolved in warm 40% EtOAc in hexane and flashed twice (Merck $SiO_2$, 20% EtOAc in hexane) to give the title compound (approx. 90% purity, 800 mg) and impure title compound (approx. 60% purity, 682 mg) as solids.

m.p. 163.5-165 °C
TLC $R_f$ 0.19 (20% EtOAc in hexane)

E. 5-Fluoro-4-(4-fluorophenyl)-2-(l-methylethyl)-6-phenyl-3-pyridinecarboxaldehyde

A -78 °C solution of oxalyl chloride (435 µl, 633 mg, 5.0 mmol) in $CH_2Cl_2$ (15 ml) was treated dropwise with a solution of dry DMSO (710 µl, 782 mg, 10.0 mmol) in $CH_2Cl_2$ (1 ml). After 15 minutes, a solution of the alcohol from part D (91% purity, 1.300 gm, assume 3.83 mmol) in $CH_2Cl_2$ (5 ml) and THF (2 ml) was added dropwise to the above mixture. Twenty minutes after the addition, TEA (3.4 ml) was added and the mixture was stirred at -78 °C for 5 minutes and then warmed to room temperature. The mixture was diluted with $Et_2O$ and washed twice with $H_2O$ and once with brine. The organic layer was dried ($Na_2SO_4$), filtered, and stripped to yield a solid. The solid was dissolved in $CH_2Cl_2$ and flashed (Merck $SiO_2$, 5% EtOAc in hexane) to give the desired product in approximately 92% purity. Recrystallization from hexane gave in two crops the title compound (1.153 gm, 89%) which was 97% pure by NMR analysis.

m.p. 135.5-137.2 °C
TLC $R_f$ 0.45 (20% EtOAc in hexane)

F. 3-(2,2-Dibromoethenyl)-5-fluoro-4-(4-fluorophenyl)-2-(1-methylethyl)-6-phenylpyridine

A solution of carbon tetrabromide (1.579 gm, 4.8 mmol) in $CH_2Cl_2$ (6 ml) was added over a 10-minute period to a cold (0 °C) solution of the aldehyde from part E (1.070 gm, 3.17 mmol) and triphenylphosphine (2.500 gm, 9.5 mmol) in $CH_2Cl_2$ (15 ml). After the addition was complete, the cooling bath was removed and the mixture was stirred for 30 minutes. The solution was quenched with saturated $NaHCO_3$ and extracted twice with $CH_2Cl_2$. The combined organic layers were dried ($Na_2SO_4$), filtered and concentrated. The concentrate was chromatographed (flash, Merck $SiO_2$, 30% $CH_2Cl_2$ in hexane) to give the dibromide title compound as a white foam that solidified. Recrystallization from-hexane gave the title compound (1.321 gm, 84%) as a white solid.

m.p. 104.5-107.0 °C
TLC $R_f$ 0.54 (20% EtOAc in hexane)

G. (S)-4-[[[5-Fluoro-4-(4-fluorophenyl)-2-(l-methylethyl)-6-phenyl-3-pyridinyl]ethynyl]methoxyphosphinyl]-3-[-[(l-dimethylethyl)diphenylsilyl]oxy]butanoic acid, methyl ester

A solution of the dibromide from part F (1.230 gm, 2.49 mmol) in THF (8 ml) at -78 °C was treated with n-BuLi (2.5 M in hexane, 2.5 ml, 6.25 mmol) over a 15-second period and the resulting dark brown-red solution was stirred for 40 minutes. The acetylenic anion solution was added dropwise via cannula over a 10-minute period to a -78 °C solution of the phosphonochloridate from Example 57, part G in THF (12 ml). The resulting mixture was stirred at -78 °C for 30 minutes, then quenched with 50% saturated $NH_4Cl$. The solution was warmed to 0 °C, diluted with $H_2O$, and poured into saturated $NaHCO_3$. The aqueous phase was extracted once with $Et_2O$. The $Et_2O$ layer was washed with brine, dried ($Na_2SO_4$),filtered and stripped to give an orange oil. The residue was chromatographed (flash, Merck $SiO_2$, 40% EtOAc in hexane) to afford the title compound as a yellow foam (1.254 gm, 66%).

TLC $R_f$ 0.29 (40% EtOAc in hexane)

H. (S)-4-[[[5-Fluoro-4-(4-fluorophenyl)-2-(l-methylethyl)-6-phenyl-3-pyridinyl]ethynyl]methoxyphosphinyl]-3-hydroxybutanoic acid, methyl ester

A mixture of the part G compound (1.227 gm, 1.60 mmol), tetra-n-butylammonium fluoride (1.0 M in THF 6.4 ml, 6.4 mmol), and HOAc (400 ul, 420 mg, 7 mmol) in THF (14 ml) was stirred at room temperature for 16 hours. The solution was diluted with EtOAc and washed 3 times with 5% $KHSO_4$. The EtOAc layer was dried ($Na_2SO_4$), filtered and stripped to afford an oil. The oil was dissolved in $Et_2O$, cooled to 0 °C and

treated with excess diazomethane for 5 minutes. The excess diazomethane was destroyed by the addition of HOAc and the solvent was removed in vacuo. The residue was chromatographed (flash, Merck $SiO_2$, 30% acetone in hexane followed by 40% acetone in hexane) to afford the title compound (669 mg, 79%) as a colorless oil.

TLC $R_f$ 0.38 (1:1 - acetone:hexane)

I. (S)-4-[[[5-Fluoro-4-(4-fluorophenyl)-2-(l-methylethyl)-6-phenyl-3-pyridinyl]ethynyl]hydroxyphosphinyl]-3-hydroxybutanoic acid, disodium salt

A solution of the part H compound (412 mg, 0.78 mmol) in dioxane (5 ml) was treated with 1 N NaOH (2.8 ml, 2.8 mmol) at room temperature and the mixture was subsequently heated at 60 °C under argon for 1.5 hours. The solvent was evaporated and the residue was chromatographed on HP-20 resin, eluting in succession with $H_2O$ (200 ml), 50% MeOH in $H_2O$ (200 ml), and MeOH (100 ml). The desired fractions were pooled and evaporated and the residue was taken up in $H_2O$ and lyophilized to give Example 5 (343 mg, 76%) as a white solid.

TLC $R_f$ 0.13 (8:1:1-$CH_2Cl_2$:HOAc:MeOH)

Analysis for $C_{26}H_{22}F_2Na_2NO_5P$ X 1.79 $H_2O$:

C, 54.25; H, 4.48; N, 2.43; F, 6.60; P, 5.38

Found: C, 54.23; H, 4.04; N, 2.45; F, 6.28; P, 5.33

Example 6

(S)-4-[[[6-Cyclohexyl-4-(4-fluorophenyl)-2-(l-methylethyl)-3-pyridinyl]ethynyl]hydroxyphosphinyl]-3-hydroxybutanoic acid, disodium salt

A. l-Cyclohexyl-3-(4-fluorophenyl)-2-propen-l-one

A sodium ethoxide solution (21% by weight in ethanol, 6.805 gm, 21 mmol) was added to a mixture of l-cyclohexyl-l-ethanone (18.749g, 140 mmol, from Aldrich) and 4-fluorobenzaldehyde (17.37g, 140 mmol, from Aldrich) in ethanol (100 ml), stirring under argon. The solution was allowed to stir 1.5 hours. The reaction mixture was then concentrated to afford an orange oil. The oil was dissolved in ether, washed with saturated $NH_4Cl$ and brine, then dried over $Na_2SO_4$, filtered and concentrated to an orange oil. The oil was purified by distillation (bp = 143 °C at 1.3 mm Hg). The resultant yellowish oil slowly solidified to afford the title compound as hard yellow crystals (22.640 gm, 65%).

m.p. 44-46 °C

TLC: Rf 0.43 (5% EtOAc in hexane)

Elemental Analysis for $C_{15}H_{17}FO$:

| | | | |
|---|---|---|---|
| Calculated: | C, 77.56; | H, 7.38; | F, 8.18 |
| Found: | C, 77.57; | H, 7.58; | F, 7.99 |

B. β-(4-Fluoro-2-phenyl-α-(2-methyl-l-oxopropyl)-Δ-oxocyclohexanepentanoic acid, ethyl ester

A sodium ethoxide solution (21% by weight in ethanol, 0.530 gm, 7.79 mmol) was added to a mixture of the enone from part A (12.930, 52 mmol) and ethyl isobutyrylacetate (12.32 gm, 77.9 mmol) in ethanol (200 ml). The solution was allowed to stir under argon at room temperature for 16 hours. Additional ethanol (30 ml) was added to the flask to make a slurry of the partially solidified reaction mixture. A white solid was then filtered out of solution and recrystallized from hot hexane to afford the title compound as a waxy white solid (19.574 gm, 92%).

m.p. 75-77° C
TLC: Rf 0.26 (10% EtOAc in hexane)

Elemental Analysis for $C_{23}H_{31}FO_4$:
Calculated: C, 70.74; H, 8.00; F, 4.87
Found: C, 70.82; H, 8.21; F, 4.82

C. 6-Cyclohexyl-4-(4-fluorophenyl)-2-(l-methylethyl)-3-pyridinecarboxylic acid, ethyl ester

$NH_4OAc$ (7.46 gm, 96.8 mmol) and $Cu(OAc)_2$ (14.49 gm, 72.6 mmol) were added to an acetic acid solution (100 ml) of the 1,5 diketone from part B (9.850 gm, 24.2 mmol). The solution was allowed to reflux 18 hours under argon. The solution was then poured into an ice-cold solution of $NH_4OH/H_2O$ (150 ml/200 ml). The mixture was extracted with ether (twice). The organic layers were combined and washed with water and brine, then dried over $Na_2SO_4$, and concentrated to afford a yellow oil. The oil was dissolved in hot hexane and cooled to afford the ester title compound as large yellowish crystals (4.300 gm, 48%).
m.p. 68-70° C
TLC: Rf (10% EtOAc in hexane)

Elemental Analysis for $C_{23}H_{27}NFO_2$:
Calculated: C, 73.86; H, 7.44; N, 3.75; F, 5.08
Found: C, 74.08; H, 7.52; N, 3.53; F, 5.00

D. 6-Cyclohexyl-4-(4-fluorophenyl)-2-(l-methylethyl)-3-pyridinemethanol

A THF solution (20 ml) of the ester from part C (4.000 gm, 10.8 mmol) was cooled to 0 ° C and treated with $LiAlH_4$ (1.23 gm, 55.7 mmol). The solution was allowed to stir 20 minutes at 0° C, then warmed to room temperature and stirred for 2 hours. Additional $LiAlH_4$ (0.885 gm, 23 mmol) was added to the solution at room temperature. The solution stirred 20 minutes. The solution was then cooled to 0° C and quenched by dropwise addition of 2.1 ml of water, followed by 2.1 ml of 15% NaOH, and then 6.3 ml water. The aluminum paste was filtered out of solution and the filtrate concentrated to afford a yellow oil. The oil was dissolved in hot hexane and the resultant solution cooled to give the title compound as fine white needles (3.077 gm, 87%).
m.p. 101-104° C
TLC: Rf 0.16 (5% EtOAc in hexane)

Elemental Analysis for $C_{21}H_{25}NFO*0.27\ H_2O$:
Calculated: C, 76.15; H, 7.77; N, 4.23; F, 5.74
Found: C, 76.43; H, 8.08; N, 3.95; F, 5.61

E. 6-Cyclohexyl-4-(4-fluorophenyl)-2-(l-methylethyl)-3-pyridinecarboxaldehyde

DMSO (0.929 gm, 11.9 mmol) was added to a solution of oxalyl chloride (0.756 gm, 5.95 mmol) in methylene chloride (40 ml), which was cooled to -78° C under argon. The solution was allowed to stir 10 minutes. Then, the alcohol from part D (1.50 gm, 4.58 mmol) was added to the flask as a methylene chloride (10 ml) solution. Gentle heating was required to get the alcohol into solution. Triethylamine (3.65

ml) was added twenty minutes later. The solution was stirred for an additional 10 minutes, then was warmed to room temperature. The solution was diluted with ether, washed with water and brine, then dried over $Na_2SO_4$, filtered and concentrated to afford a yellow-brown oil. The compound was purified by flash chromatography on Merck silica gel in 5% EtOAc in hexane. Fractions containing pure product were combined and concentrated. The resulting yellowish solid was recrystallized from hexane to give the title compound as hard, white crystals (1.239 gm, 83%).

m.p. 59-61 ° C

TLC: Rf 0.33 (10% EtOAc in hexane)

| | |
|---|---|
| Elemental Analysis for $C_{21}H_{23}NFO*0.21\ H_2O$: | |
| Calculated: | C, 76.87; H, 7.19; N, 4.27; F, 5.79 |
| Found: | C, 77.06; H, 7.56; N, 4.08; F, 5.78 |

F. 3-(2,2-Dibromoethenyl)-6-Cyclohexyl-4-(4-fluorophenyl)-2-(l-methylethyl)pyridine

$CBr_4$ (1.55 gm, 4.60 mmol) was added as a methylene chloride solution (10 ml) over 5 minutes to a mixture of triphenylphosphine (2.58, 9.85 mmol) and the aldehyde from part E (1.230 gm, 3.07 mmol) in methylene chloride (50 ml) that had been cooled to 0 ° 6. The solution was stirred for an additional 30 minutes. The reaction was quenched with saturated $NaHCO_3$ and the solution allowed to warm to room temperature. The aqueous layer was extracted with methylene chloride (twice) and the organic layers were combined and washed with brine, then dried over $Na_2SO_4$, filtered and concentrated to about 10 ml. The solution was subjected to flash chromatography on Merck silica gel in 30% methylene chloride in hexane. The desired fractions were pooled and concentrated to afford a clear oil, which solidified upon standing overnight. The white solid was recrystallized from hexane to afford the title compound as hard, white crystals (1.220 gm, 85%).

m.p. 98-100 ° C

TLC: Rf 0.89 (5% EtOAc in hexane)

| | |
|---|---|
| Elemental Analysis for $C_{20}H_{16}NBr_2FS$: | |
| Calculated: | C, 49.92; H, 3.35; N, 2.91; Br, 33.21; F, 3.95; S, 6.66 |
| Found: | C, 50.43; H, 3.25; N, 2.67; Br, 33.43; F, 3.95; S, 6.96 |

G. (S)-4-[[[6-Cyclohexyl-4-(4-fluorophenyl)-2-(l-methylethyl)-3-pyridinyl]ethynyl]methoxyphosphinyl]-3[[(l,l-dimethylethyl)diphenylsilyl]oxy]butanoic acid, methyl ester

2.5 M n-BuLi (1.68 ml, 4.2 mmol) was added dropwise to a solution of the dibromide from part F (3.976 gm, 2.10 mmol) in THF (15 ml) at -78 ° C under argon. The solution was allowed to stir l hour at -78 ° C, then cannulated into a THF solution (15 ml) of the chloridate from Example 2, part J which had also been cooled to -78 ° C. The resultant red-amber solution was stirred for 45 minutes, then quenched with saturated $NH_4Cl$ (15 ml) and warmed to 0 ° C. Then, saturated $NaHCO_3$ was added to the solution, which was subsequently warmed to room temperature. The mixture was diluted with ether and the organic layer was washed with brine, dried over $Na_2SO_4$, filtered and concentrated to give an orange oil. The oil was purified by flash chromatography on Merck silica gel in 40% EtOAc in hexane. The desired fractions were combined and evaporated to afford the title compound as a beige foam (1.018 gm, 68%).

TLC: Rf 0.35 (40% EtOAc in hexane)

H. (S)-4-[[[6-Cyclohexyl-4-(4-fluorophenyl)-2-(l-methylethyl)-3-pyridinyl]ethynyl]methoxyphosphinyl]-3-hydroxy]butanoic acid, methyl ester

$(Bu)_4NF$ (1.0 M in THF, 4.26 ml, 4.26 mmol) was added to a mixture of silyl ether from part G (1.018 gm, 1.42 mmol) and acetic acid (0.42 gm, 7.20 mmol) in THF (10 ml). The solution was allowed to stir 18 hours at room temperature under argon. The solution was diluted with EtOAc (20 ml) and then washed with 5% $KHSO_4$ (3 x 20 ml). The aqueous layers were back-extracted with EtOAc (3 times). The organic layers were pooled and washed with brine, then dried over $Na_2SO_4$, filtered and concentrated to afford a brown oil. The oil was dissolved in ether (15 ml) and treated with excess $CH_2N_2$. Excess $CH_2N_2$ was removed with a stream of argon, and the solution was concentrated to a brown oil. The oil was purified by flash chromatography on Merck silica gel in 35% acetone in hexane. The desired fractions were pooled and concentrated to afford the title compound as a beige foam (0.370 gm, 51%).

TLC: Rf 0.49 (40% acetone in hexane)

I. (S)-4-[[[6-Cyclohexyl-4-(4-fluorophenyl)-2-(l-methylethyl)-3-pyridinyl]ethynyl]hydroxyphosphinyl]-3-hydroxybutanoic acid, disodium salt

NaOH (1 M in water, 2.10 ml, 2.10 mmol) was added to a solution of the part H compound (0.370 gm, 0.72 mmol) in dioxane (10 ml). The solution was then heated to 55° C, allowed to stir 45 minutes and then concentrated to a white solid. The residue was dissolved in water and chromatographed on HP-20 resin, eluting first with water (200 ml), then with 50% MeOH in water (400 ml), then with MeOH (200 mol). The desired fractions were pooled and concentrated. The residue was taken up in water and lyophilized to give Example 6 as a fluffy white solid (0.374 gm, 98%).

TLC: Rf 0.67 (6:3:1, n-propanol: $NH_4OH$: $H_2O$)

Elemental Analysis for $C_{26}H_{29}NFNa_2PO_5$*1.60 $H_2O$:

| | |
|---|---|
| Calculated: | C, 55.74; H, 5.79; N, 2.50; F, 3.39; P, 5.53 |
| Found: | C, 55.74; H, 5.64; N, 2.66; F, 3.22; P 5.63 |

Example 7

(S)-4-[[[4-(4-Fluorophenyl)-2-(l-methylethyl)-6-(2-thienyl)-3-pyridinyl]ethynyl]hydroxyphosphinyl]-3-hydroxybutanoic acid, disodium salt

A. 3-(4-Fluorophenyl)-l-(2-thienyl)-2-propen-l-one

A solution of sodium ethoxide (21% by weight in EtOH, 0.68 gm, 10 mmol) was added to a solution of 2-acetylthiophene (12.60 gm, 100 mmol, from Aldrich) and 4-fluorobenzaldehyde (12.41 gm, 100 mmol, from Aldrich) in EtOH (50 ml) and the mixture stirred under argon for 15 minutes. More EtOH (25 ml) was then added to make a slurry of the reaction mixture, which had partially solidified. Acetic acid (0.60 gm, 11 mmol) was added and a yellow solid filtered out of solution. The solid was recrystallized from EtOAc and hexane to afford the title compound as long, pale yellow needles (17.679 gm, 71%).

m.p. 117-120° C

TLC: Rf 0.24 (10% EtOAc in hexane)

Elemental Analysis for $C_{13}H_9FSO$*0.21 $H_2O$:

| | |
|---|---|
| Calculated: | C, 66.13; H, 4.02; F, 8.05; S, 13.58 |
| Found: | C, 66.14; H, 3.95; F, 8.05; S, 13.58 |

B. β-(4-Fluoro-2-phenyl)-α-(2-methyl-l-oxopropyl)-Δ-oxo-2-thiophenepentanoic acid, ethyl ester

A solution of sodium ethoxide (21% by weight in EtOH, 0.530 gm, 7.79 mmol) was added to a solution of ethyl isobutyrylacetate (16.1 gm, 101 mmol) and the enone from part A (16.975 gm, 67.9 mmol) in EtOH (300 ml) and the mixture stirred under argon at room temperature for 17 hours. Acetic acid (0.667 gm, 11 mmol) was added to quench the reaction, and the solution was concentrated. The oily, yellow solid was dissolved in EtOAc and washed with saturated $NH_4Cl$, $H_2O$, and brine, then dried over $MgSO_4$, filtered and concentrated to give an oily, yellow solid. The solid was recrystallized from hexane to afford the title compound as fine white needles (21.413 gm, 77%).

m.p. 92-105 °C
TLC: Rf 0.25 & 0.30 (20% EtOAc in hexane)

Elemental Analysis for $C_{21}H_{23}FSO_4$:

| | |
|---|---|
| Calculated: | C, 64.59; H, 5.94; F, 4.87; S, 8.21 |
| Found: | C, 64.68; H, 5.98; F, 5.03; S, 8.27 |

C. 4-(4-Fluorophenyl)-2-(l-methylethyl)-6-(2-thienyl)-3-pyridinecarboxylic acid, ethyl ester

$NH_4OAc$ (8.176 gm, 106 mmol) and $Cu(OAc)_2$ (15.57 gm, 78 mmol) were added to an acetic acid solution (150 ml) of the 1,5 diketone from part B (10.025 gm, 26.5 mmol) and the mixture was allowed to reflux 18 hours under argon. The solution was then poured into an ice-cold solution of $NH_4OH/H_2O$ (200 ml/250 ml). The mixture was extracted with ether and EtOAc, washed with water and saturated NaCl, then dried over $Na_2SO_4$, and concentrated to afford a yellow-brown oil. Flash chromatography was performed on the oil in 5% EtOAc in hexane on Merck silica gel. Product fractions were pcoled and concentrated to a yellow solid. The solid was recrystallized from hexane to afford the title compound as hard yellow crystals (5.627 gm, 58%).

m.p. 114-115 °C
TLC: Rf 0.78 (20% EtOAc in hexane)

D. 4-(4-Fluorophenyl)-2-(l-methylethyl)-6-(2-thienyl)-3-pyridinemethanol

A THF solution (20 ml) of the ester from part C (5.620 gm, 15.2 mmol) was cooled to 0 °C and treated with $LiAlH_4$ (1.730 gm, 72 mmol). The solution was allowed to stir twenty minutes at 0 °C and then was warmed to room temperature. Additional $LiAlH_4$ (1.000 gm, 26.3 mmol) was added to the solution. The solution was allowed to stir 30 minutes, then cooled to 0 °C and quenched by dropwise addition of 2.73 ml of water followed by 2.73 ml of 15% NaOH, then 8.20 ml of water. The aluminum paste was filtered out of solution and the filtrate was concentrated to a yellow solid. The solid was recrystallized from hexane to afford the title compound as hard yellow crystals (4.067 gm, 82%).

m.p. 151-153 °C
TLC: Rf 0.42 (20% EtOAc in hexane)

Elemental Analysis for $C_{19}H_{18}NFSO*0.06\ H_2O$:

| | |
|---|---|
| Calculated: | C, 69.47; H, 5.56; N, 4.26; F, 5.78; S, 9.76 |
| Found: | C, 69.77; H, 5.50; N, 3.96; F, 5.67; S, 9.63 |

E. 4-(4-Fluorophenyl)-2-(l-methylethyl)-6-(2-thienyl)-3-pyridinecarboxaldehyde

DMSO (1.53 gm, 19.6 mmol) was added to a solution of oxalyl chloride (1.25 gm, 9.80 mmol) in methylene chloride (40 ml), which had been cooled to -78 °C under argon. The solution was allowed to stir 10 minutes, then the alcohol from part D (2.456 gm, 7.55 mmol) was added to the flask as a methylene

chloride (20 ml) solution. Triethylamine (5.25 ml, 38 mmol) was added 20 minutes later and the solution stirred for an additional 10 minutes, then warmed to room temperature. The solution was diluted with ether and washed with water and brine, then dried over $Na_2SO_4$, filtered and concentrated to afford a yellow solid. The solid was recrystallized from hexane to afford the title compound as hard, yellow crystals (2.255 gm, 93%).

m.p. 123-125° C
TLC: Rf 0.50 (20% EtOAc in hexane)

F. 3-(2,2-Dibromoethenyl)-4-(4-fluorophenyl)-2-(l-methylethyl)-6-(2-thienyl)pyridine

A solution of $CBr_4$ (3.36 gm, 10 mmol) in methylene chloride (15 ml) was added over 15 minutes to a solution of triphenylphosphine (5.59 gm, 21.3 mmol) and the aldehyde from part E (2.155 gm, 6.67 mmol) in methylene chloride (60 ml), which was stirring under argon at 0 ° C. The solution stirred for an additional 20 minutes at 0° C, then was warmed to room temperature and stirred 45 minutes. Saturated $NaHCO_3$ (15 ml) was added to quench the reaction. The aqueous layer was extracted with methylene chloride (twice), the organic layers were combined and washed with brine, then dried over $Na_2SO_4$, filtered and concentrated to about 15 ml. The viscous solution was purified by flash chromatography on Merck silica gel in 40% methylene chloride in hexane. Pure fractions were pooled and concentrated to afford a white foam. The foam was dissolved in hexane, and the solution cooled to afford the title compound as pale yellow crystals (2.942 gm, 92%)

m.p. 107-108 ° C
TLC: Rf 0.70 (10% EtOAc in hexane)

Elemental Analysis for $C_{20}H_{16}NBr_2FS$:

| | | |
|---|---|---|
| Calculated: | C, 49.92; H, 2.91; N, 2.91; Br, 33.21; | |
| | F, 3.95; S, 6.66 | |
| Found: | C, 50.43; H, 3.25; N, 2.67; Br, 33.43; | |
| | F, 3.95; S, 6.96 | |

G. (S)-4-[[[4-(4-Fluorophenyl)-2-(l-methylethyl)-6-(2-thienyl)-3-pyridinyl]ethynyl]methoxyphosphinyl]-3-[[(l,l-dimethylethyl)diphenylsilyl]oxy]butanoic acid, methyl ester

n-BuLi (2.5 M in hexane, 3.34 ml, 8.34 mmol) was added dropwise to a solution of the dibromide from part F (2.000 gm, 4.17 mmol) in THF (20 ml) at -78° C under argon. The solution was allowed to stir 1 hour at -78 ° C, then cannulated into a THF solution (15 ml) of the phosphonochloridate from Example 2, part J, which had also been cooled to -78° C. The resultant red-amber solution was stirred for 45 minutes, then quenched with saturated $NH_4Cl$, warmed to 0 ° C and then saturated $NaHCO_3$ was added to the solution. The mixture was diluted with ether, the organic layer was washed with brine, then dried over $Na_2SO_4$, filtered and concentrated to give an orange oil. The oil was purified by flash chromatography on Merck silica gel in 40% EtOAc in hexane. Pure product fractions were combined and concentrated to afford the title compound as a beige foam (1.018 gm, 68%).

TLC: Rf 0.35 (40% EtOAc in hexane)

H. (S)-4-[[[4-(4-Fluorophenyl)-2-(l-methylethyl)-6-(2-thienyl)-3-pyridinyl]ethynyl]methoxyphosphinyl]-3-hydroxybutanoic acid, methyl ester

A solution of $(Bu)_4NF$ (1 M in THF, 6.8 ml, 6.8 mmol) was added to a solution of the silyl ether from part G (1.640 gm, 2.2 mmol) and acetic acid (0.66 gm, 11.0 mmol) in THF (15 ml) and the solution allowed to stir at room temperature 16 hours. The solution was diluted with EtOAc, then washed with 5% $KHSO_4$ (3 times). The aqueous layers were extracted with EtOAc (3 times). The organic layers were pooled and washed with brine, then dried over $Na_2SO_4$, filtered and concentrated to afford a yellow oil. The oil was dissolved in ether and treated with excess $CH_2N_2$. Excess $CH_2N_2$ was removed with a stream of argon, and the solution was concentrated to give a yellow oil. The oil was purified by flash chromatography on Merck

silica gel in 35% acetone in hexane. Pure product fractions were pooled and concentrated to afford the title compound as a beige foam (0.870 gm, 75%).

TLC: Rf 0.63 (50% acetone in hexane)

I. (S)-4-[[[4-(4-Fluorophenyl)-2-(1-methylethyl)-6-(2-thienyl)-3-pyridinyl]ethynyl]hydroxyphosphinyl]-3-hydroxybutanoic acid, disodium salt

A solution of the part H compound (0.870 gm, 1.66 mmol) in dioxane (10 ml) was treated with a solution of NaOH (1 M in water, 4.95 ml, 4.95 mmol). The solution was then heated to 55 °C and allowed to stir 1.5 hours. The mixture was concentrated to a white solid. The residue was chromatographed on HP-20 resin, eluting first with water (200 ml), then with 50% MeOH in water (400 ml), then with MeOH (200 ml). The desired fractions were pooled and concentrated, the residue taken up in water and lyophilized to give Example 7 as a fluffy white solid (0.807 gm, 92%).

TLC: Rf 0.70 (6:3:1, n-propanol: $NH_4OH$: $H_2O$)

Elemental Analysis for $C_{24}H_{21}NFNa_2PSO_5$*1.55 $H_2O$:

| | |
|---|---|
| Calculated: | C, 51.53; H, 4.34; N, 2.50; F, 3.40; P, 5.54 |
| Found: | C, 51.58; H, 4.25; N, 2.58; F, 3.72; P, 5.71 |

Example 8

(S)-4-[[[6-Cyclopropyl-4-(4-fluorophenyl)-2-(1-methylethyl)-3-pyridinyl]ethynyl]hydroxyphosphinyl]-3-hydroxybutanoic acid, disodium salt

A. 1-Cyclopropyl-3-(4-fluorophenyl)-2-propen-1-one

A mixture of 4-fluorobenzaldehyde (9.30 g, 75.00 mmol, Aldrich) and cyclopropyl methyl ketone (6.30 g, 75.00 mmol) in absolute ethanol (100 ml) was treated with a solution of sodium ethoxide in ethanol (21% by weight solution; 2.79 ml, 7.50 mmol). After stirring for 2 hours at room temperature, additional 4-fluorobenzaldehyde was added (1.86 g, 15.00 mmol). The reaction mixture was stirred for 2 hours, concentrated to 1/3 volume and partitioned between 50% saturated $NH_4Cl$/EtOAc (100 ml/250 ml). The layers were separated and the aqueous portion was extracted with EtOAc (twice) and the combined organics were washed with $H_2O$ (twice) and brine, dried ($Na_2SO_4$) and concentrated. The yellow oily residue was distilled (Vigroux column, bp 115 - 120 °C, 0.6 mm Hg) to afford the title compound as a colorless oil (12.8 g, 89.7%).

$R_f$ 0.37 (15% EtOAc/hexane), UV

B. β-(4-Fluorophenyl)-α-(2-methyl-1-oxopropyl)-Δ-oxocyclopropylpentanoic acid, ethyl ester

A mixture of the compound from part A (6.65 g, 35.00 mmol) and ethyl isobutyrylacetate (8.47 ml, 52.50 mmol) in absolute ethanol (50 ml) was treated with a solution of sodium ethoxide in ethanol (21% by weight solution, 6.51 ml, 17.00 mmol). The reaction mixture was stirred at room temperature for 48 hours, then concentrated to 1/3 volume and partitioned between 50% saturated $NH_4Cl$/EtOAc (100 ml/250 ml). The layers were separated and the aqueous portion was extracted with EtOAc (twice) and the combined organics were washed with $H_2O$ (twice) and brine, then dried ($Na_2SO_4$) and concentrated. The dark oily residue was purified by flash chromatography (Merck silica gel, 5% EtOac/hexane) to afford the title compound as a colorless oil (9.0 g, 74.3%, mixture of diastereomers).

$R_f$ 0.29 + 0.31 (25% EtOAc/hexane), UV

C. 6-(Cyclopropyl-4-(4-fluorophenyl)-2-(1-methylethyl)-3-pyridinecarboxylic acid, ethyl ester

A mixture of the part B compound (9.00 g, 25.7 mmol), ammonium acetate (5.94 g, 77.10 mmol) and

copper (II) acetate (12.82 g, 64.25 mmol) in glacial acetic acid (80 ml) was heated at 115 °C for 16 hours (TLC after 30 minutes indicated the presence of two products and almost no starting material). The reaction mixture was cooled and carefully poured into an ice cold mixture of concentrated $NH_4OH$ (100 ml) and $H_2O$ (200 ml). The mixture was extracted with ether (twice) and the ether extracts were washed with $H_2O$ (twice) and brine, then dried ($MgSO_4$), filtered and concentrated. The dark oily residue was purified by flash chromatography (10% EtOAc/hexane) to afford Compound III as a colorless oil (3.45 g, 41.0%).

$R_f$ 0.62 (20% EtOAc/hexane), UV

D. 6-(Cyclopropyl-4-(4-fluorophenyl)-2-(1-methylethyl)-3-pyridinemethanol

A cold (0 °C) solution of the ester from part C (3.40 g, 11.03 mmol) in dry THF (50 ml) was treated with $LiAlH_4$ (1.25 g, 33.00 mmol). Ten minutes after the addition the cooling bath was removed and the reaction mixture stirred at room temperature for 3 hours and then refluxed for 24 hours. The reaction mixture was cooled to 0 °C and carefully quenched in succession with 1.25 ml of $H_2O$ in THF (10 ml), then 1.25 ml 15% NaOH and finally 3.0 ml $H_2O$. The precipitated aluminum salts were filtered and washed with EtOAc and ether. The filtrate was washed with $H_2O$ and brine, dried ($Na_2SO_4$), filtered and concentrated under reduced pressure to give an oily residue. Purification by flash chromatography (Merck silica gel, 5% EtOAc/hexane) yielded the title compound as a colorless oil which solidified on standing under high vacuum. Recrystallization from hot hexane afforded the title compound as a white solid (2.32 g, 79.3%).

m.p. 94 - 95 °C

$R_f$ 0.36 (20% EtOAc/hexane), UV

Analysis for $C_{18}H_{20}NFO$:

| | | | | |
|---|---|---|---|---|
| Calc'd: | C, 75.76; | H, 7.06; | N, 4.91; | F, 6,66 |
| Found: | C, 75.23; | H, 7.13; | N, 4.92; | F, 6.71 |

E. 6-(Cyclopropyl-4-(4-fluorophenyl)-2-(1-methylethyl)-3-pyridinecarboxaldehyde

A -78 °C solution of oxalyl chloride (0.85 ml, 9.67 mmol) in $CH_2Cl_2$ (70 ml) was treated dropwise with a solution of dry DMSO (1.37 ml, 19.34 mmol) in $CH_2Cl_2$ (10 ml). After 15 minutes, a solution of the alcohol from part D (2.30 g, 8.06 mmol) in $CH_2Cl_2$ (10 ml) was added dropwise to the above solution. After 20 minutes, triethylamine (6.75 ml, 48.36 mmol ) was added and the mixture was stirred at -78 °C for 20 minutes and then warmed to room temperature. After 1 hour, the reaction was quenched with $H_2O$ and diluted with ether. The layers were separated and aqueous layer was extracted with ether (twice). The combined organic solutions were washed with $H_2O$ (twice) and brine, then dried ($MgSO_4$), filtered and concentrated under reduced pressure. The oily residue was purified by flash chromatography (Merck silica gel, 10% EtOAc/hexane), affording the aldehyde title compound as a colorless oil, which solidified on standing under high vacuum (1.74 g, 76.3%).

m.p. 67 - 68 °C

$R_f$ 0.56 (20% EtOAc/hexane), UV

Analysis for $C_{18}H_{18}NFO$:

| | | | | |
|---|---|---|---|---|
| Calc'd: | C, 76.30; | H, 6.40; | N, 4.94; | F, 6.71 |
| Found: | C, 75.99; | H, 6.43; | N, 4.69; | F, 6.66 |

F. 6-Cyclopropyl-3-(2,2-dibromoethenyl)-4-(4-fluorophenyl)-2-(1-methylethyl)-pyridine

A solution of carbon tetrabromide (2.89 g, 8.73 mmol) in $CH_2Cl_2$ (10 ml) was added dropwise over a 5-minute period to a cold (0 °C) solution of the aldehyde from part E (1.65 g, 5.82 mmol) and triphenylphosphine (4.58 g, 17.46 mmol) in $CH_2Cl_2$ (60 ml). The mixture was stirred at 0 °C for 10 minutes and then at room temperature for 1 hour. The solution was quenched with saturated $NaHCO_3$ and extracted twice with $CH_2Cl_2$. The combined organic layers were dried ($Na_2SO_4$), filtered , concentrated to 1/3 volume and

applied on Merck silica gel column (50% $CH_2Cl_2$/hexane). Flash chromatography afforded the title compound as a colorless oil (2.36 g, 93.0%).

$R_f$ 0.58 (10% EtOAc/hexane), UV

G. (S)-4-[[[6-Cyclopropyl-4-(4-fluorophenyl)-2-(1-methylethyl)-3-pyridinyl]ethynyl]methoxyphosphinyl]-3-[[-(1,1-dimethylethyl)diphenylsilyl]oxy]butanoic acid, methyl ester

A solution of the dibromide from part F (2.00 g, 4.56 mmol) in THF (15 ml) at -78°C was treated with n-BuLi (2.5 M in hexane, 3.82 ml, 9.57 mmol) over a 1-minute period. The brown-red reaction mixture was stirred for 30 minutes at -78°C and then transferred via cannula to - 78°C solution of the phosphonochloridate from Example 2, part J in THF (15 ml). The dark-brown reaction mixture was stirred -78°C for 45 minutes and then quenched with 50% saturated $NH_4Cl$. After warming to room temperature, the solution was diluted with $H_2O$ and poured into saturated $NaHCO_3$. The layers were separated and the aqueous layer was extracted once with ether. The combined organic layers were washed with brine, dried ($Na_2SO_4$), filtered and concentrated under reduced pressure. The dark oily residue was purified by flash chromatography (Merck silica gel, 20% EtOAc/hexane) to afford Compound G as a pale yellow oil (2.11 g, 65.0%).

$R_f$ 0.18 (30% EtOAc/hexane), UV

H. (S)-4-[[[6-Cyclopropyl-4-(4-fluorophenyl)-2-(1-methylethyl)-3-pyridinyl]ethynyl]methoxyphosphinyl]-3-hydroxybutanoic acid, methyl ester

To a solution of the acetylinic phosphonate from part G (2.10 g, 2.95 mmol) in THF (15 ml) was added HOAc (0.67 ml, 11.8 mmol), followed by tetra-n-butylammonium fluoride (1.0 M in THF, 8.85 ml, 8.85 mmol). The reaction mixture was stirred at room temperature under argon for 24 hours. The solution was diluted with EtOAc and washed three times with 5% $KHSO_4$. The aqueous layer was back-extracted twice with EtOAc and the combined organic extracts were dried ($Na_2SO_4$), filtered and concentrated under reduced pressure. The dark oil was dissolved in ether (added a few drops of THF to obtain a clear solution), cooled to 0 °C and treated with excess diazomethane in ether for 20 minutes. The excess diazomethane was destroyed by the addition of HOAc. Solvent removal gave a dark oily residue, which was purified by flash chromatography (Merck silica gel, 30% acetone/hexane). The product was obtained as a colorless oil (0.77 g, 55.7%).

$R_f$ 0.30 (40% acetone/hexane) UV

I. (S)-4-[[[6-Cyclopropyl-4-(4-fluorophenyl)-2-(1-methylethyl)-3-pyridinyl]ethynyl]hydroxyphosphinyl]-3-hydroxybutanoic acid, disodium salt

The diester from part H (0.76 g, 1.60 mmol) in dioxane (15 ml) was treated with 1 N NaOH (5.60 ml, 5.60 mmol) at room temperature and then heated at 50 °C for 2.5 hours. The reaction mixture was concentrated and the residue was chromatographed on HP-20 resin, eluting first with $H_2O$ (300 ml), followed by 25% $MeOH/H_2O$ (300 ml), 50% $MeOH/H_2O$ (300 ml) and finally MeOH (100ml). The collected product fractions were evaporated, dissolved in $H_2O$, filtered, frozen and lyophilized to give Example 8 as a white lyophilate (0.61 g, 78.2%).

$R_f$ 0.27 (8:1:1 - $CH_2Cl_2$: $CH_3OH$ : HOAc), UV

Analysis for $C_{23}H_{23}NFPO_5Na_2$ x 2.67 $H_2O$:

| | | | | | |
|---|---|---|---|---|---|
| Calc'd: | C, 51.40; | H, 5.31; | N, 2.61; | F, 3.54; | P, 5.76 |
| Found: | C, 51.70; | H, 5.13; | N, 2.31; | F, 3.39; | P, 5.55 |

Example 9

(S,E)-4-[[2-[4-(4-Fluorophenyl)-6,7-dihydro-2-(l-methylethyl)-5H-benzo[6,7]cyclohepta[l,2-b]pyridin-3-yl]-ethyl]hydroxyphosphinyl]-3-hydroxybutanoic acid, disodium salt

A. 3-Ethynyl-4-(4-fluorophenyl)-6,7-dihydro-2-(l-methylethyl)-5H-benzo-[6,7]cyclohepta[l,2-b]pyridine

The vinyl dibromide compound D from Example 3 (3.520 gm, 6.8 mmol) was added as a solution of THF (4 ml) over 2.5 minutes to a -78° C solution of n-BuLi (2.5 M in hexane, 5.46 ml, 13.66 mmol), in THF (20 ml). The solution was allowed to stir at -78 °C for 1.5 hours. Saturated $NH_4Cl$ was added to quench the reaction and the mixture warmed to 0° C and saturated $NaHCO_3$ added. The mixture was then warmed to room temperature, diluted with ether and the aqueous layer back-extracted with ether (twice). The organic layers were combined and washed with brine, then dried over $MgSO_4$, filtered and concentrated to afford a yellow oil, The yellow oil was purified by flash chromatography on Merck silica gel in 1% EtOAc in hexane. Fractions containing the product were pooled and concentrated to give a white solid. The solid was recrystallized from hexane as hard white crystals (2.108 gm, 87%).

m.p. 131-132 °C

Rf 0.43 (1% EtOAc in hexane)

B. (E)-3-(2-Iodoethenyl)-4-(4-fluorophenyl)-6,7-dihydro-2-(l-methylethyl)-5H-benzo-[6,7]cyclohepta[l,2-b]-pyridine

A mixture of the acetylene prepared in part A (2.108 gm, 6.68 mmol), $Bu_3SnH$ (3.507 gm, 12.03 mmol), and AIBN (0.0408 gm) was heated to 140° C for 1.5 hours. Ether (20 ml) was added to the mixture after it had cooled to room temperature. The solution was then treated with $I_2$ (3.49 gm) and stirred at room temperature for 18 hours. The solution was diluted with ether, washed with 10% $Na_2SO_4$ in saturated $NaHCO_3$ and brine, then dried over $MgSO_4$, filtered and concentrated to a yellow oil. The oil was purified by flash chromatography on Merck silica gel in 1% EtOAc in hexane. Fractions containing product compound B were pooled and concentrated to give a yellow solid. The solid was recrystallized from hexane to afford product compound B as yellow crystals (2.363 gm, 80%).

m.p. 169-170° C

TLC: Rf 0.55 (2% EtOAc in hexane)

C. (S)-4-[[2-[6-(l,l-Dimethylethyl)-4-(4-fluorophenyl)-2-(l-methylethyl)-3-pyridinyl]ethyl]methoxyphosphinyl]-3-[[(l,l-dimethylethyl)diphenylsilyl]oxy]butanoic acid, methyl ester

The vinyl iodide from part B (2.363 gm, 5.0 mmol) was added as a THF solution (5 ml) over 3 minutes to a solution of t-BuLi (1.7 M in pentane, 5.90 ml, 10 mmol) in THF (10 ml) at -78° C. The solution was allowed to stir 1 hour at -78° C, then cooled to -100 °C and cannulated over 1.5 minutes into a THF solution (20 ml) of the Example 2, part J chloridate also cooled to -100° C. The resultant red-amber solution was stirred for 45 minutes, then quenched with saturated $NH_4Cl$ (15 ml). The solution was warmed to 0° C and saturated $NaHCO_3$ was added. The mixture was diluted with ether and the aqueous layer back-extracted with ether (twice). The organic layers were combined and washed with brine, then dried over $Na_2SO_4$, filtered and concentrated to give an orange oil. The oil was purified by flash chromatography on Merck silica gel in 40% EtOAc in hexane. Desired fractions were combined and evaporated to afford the product as a beige foam (1.408 gm, 39%).

TLC: Rf 0.36 (40% EtOAc in hexane)

D. (S)-4-[[2-[6-(l,l-Dimethylethyl)-4-(4-fluorophenyl)-2-(l-methylethyl)-3-pyridinyl]ethyl]methoxyphosphinyl]-3-hydroxybutanoic acid, methyl ester

A THF solution (20 ml) of compound C (1.408 gm, 1.88 mmol) was treated with acetic acid (0.45 gm, 7.55 mmol) and then $(Bu)_4NF$ (1.0 M in THF, 5.64 ml, 5.64 mmol) and allowed to stir 16 hours at room temperature. The solution was diluted with EtOAc, then washed with saturated $NaHCO_3$, water, 1.5 M HCl, water, and brine, then dried over $MgSO_4$, filtered and concentrated to give a yellow oil. The oil was purified by flash chromatography on Merck silica gel in 50% acetone in hexane. Pure product fractions were pooled and concentrated to afford compound D as a beige foam (0.726 gm, 71%).

TLC: Rf 0.48 (50% acetone in hexane)

E. (S,E)-4-[[2-[4-(4-Fluorophenyl)-6,7-dihydro-2-(l-methylethyl)-5H-benzo[6,7]-cyclohepta[l,2-b]pyridin-3-yl]-ethyl]hydroxyphosphinyl]-3-hydroxybutanoic acid, disodium salt

A solution of NaOH (1.0 M in water, 2.1 ml, 2.1 mmol) was added to a solution of compound D in

dioxane (5 ml) and allowed to stir 19 hours at room temperature. The solution was then concentrated to a white solid. The solid was dissolved in water and chromatographed on HP-20 resin, eluting first with water (200 ml), then with 50% MeOH in water (400 ml), then with MeOH (200 mol). The desired fractions were pooled and concentrated. The residue was taken up in water and lyophilized to give Example 9 as a fluffy white solid (0.375 gm, 97%).

TLC: Rf 0.68 (6:3:1, n-propanol: $NH_4OH$: $H_2O$)

Elemental Analysis for $C_{29}H_{29}NFNa_2PO_5$ X 2.50 $H_2O$:

| | |
|---|---|
| Calculated: | C, 56.87; H, 5.59; N, 2.29; F, 3.10; P, 5.06 |
| Found: | C, 56.53; H, 5.48; N, 2.38; F, 3.40; P, 5.25 |

Example 10

(S)-4-[[2-[4-(4-Fluorophenyl)-6,7-dihydro-2-(l-methylethyl)-5H-benzo[6,7]cyclohepta[l,2-b]pyridin-3-yl]ethyl]-hydroxyphosphinyl]-3-hydroxybutanoid acid, disodium salt

A. (S)-4-[[2-[4-(4-Fluorophenyl)-6,7-dihydro-2-(l-methylethyl)-5H-benzo[6,7]-cyclohepta[l,2-b]pyridin-3-yl]-ethyl]methoxyphosphinyl]-3-hydroxybutanoid acid, methyl ester

A mixture of compound D from Example 9 (0.347 g, 0.63 mmol) and 10% Pd/C (0.067 g, 0.063 mmol) in MeOH (50 ml) was purged with argon for fifteen minutes, then placed under a hydrogen pressure of 50 psi on a Parr apparatus for 7 days. The solution was then filtered through a pad of Celite® which was washed repeatedly with MeOH. The solution was concentrated and the white residue purified by flash chromatography on Merck silica gel in 40% acetone in hexane. After combining and concentrating the desired fractions, the product A was obtained as a white foam (0.295 gm, 85%).

TLC: Rf 0.44 (50% acetone in hexane)

B. (S)-4-[[2-[4-(4-Fluorophenyl)-6,7-dihydro-2-(l-methylethyl)-5H-benzo[6,7]cyclohepta[l,2-b]pyridin-3-yl]-ethyl]hydroxyphosphinyl]-3-hydroxybutanoid acid, disodium salt

A solution of the part A compound (0.295 g, 0.533 mmol) in dioxane (7 ml) was treated with a solution of NaOH (1.0 M in water, 1.60 ml, 1.60 mmol) and allowed to stir at 50° C for 3 hours. The solution was cooled to room temperature and concentrated to a white solid. The residue was dissolved in water and chromatographed on HP-20 resin, eluting first with water (200 ml), then with 50% MeOH in water (400 ml), and then with MeOH (200 mol). The desired fractions were pooled and concentrated, the residue was taken up in water and lyophilized to give Example 10 as a fluffy white solid (0.285 gm, 94%).

TLC: Rf 0.66 (6:3:1, n-propanol: $NH_4OH$: $H_2O$)

Elemental Analysis for $C_{29}H_{31}NFNa_2PO_5$ x 2.04 $H_2O$:

| | |
|---|---|
| Calculated: | C, 57.46: H, 5.83; N, 2.31; F, 3.13; P, 5.11 |
| Found: | C, 57.59; H, 6.16; N, 2.33; F, 3.18; P, 5.38 |

Example 11

(S)-4-[[[4-(4-Fluorophenyl)-5,6-dihydro-2-(1-methylethyl)benzo[h]quinolin-3-yl]ethynyl]hydroxyphosphinyl]-3-hydroxybutanoic acid, disodium salt

A. $\beta$-(4-Fluorophenyl)-1,2,3,4-tetrahydro-$\alpha$-(2-methyl-1-oxopropyl)-1-oxo-2-naphthalenepropanoic acid, ethyl ester

A -78 °C solution of lithium bis(trimethylsilyl)amide (1 M in THF, 33.5 ml, 33.6 mmol) in THF (70 ml) was treated dropwise with a solution of 3,4-dihydro-1(2H)-naphthalenone (4.46 ml, 33.6 mmol, Aldrich) in THF (10 ml). After one hour, a solution of the compound from Example 53, part A (6.96 gm, 28.0 mmol) in THF (5 ml) was added to the solution. The mixture was stirred at -78 °C for one hour, then at 0 °C for 1.5 hours. The reaction mixture was treated with glacial HOAc (8 ml), then quenched with 50% saturated $NH_4Cl$ and extracted with EtOAc. The aqueous layer was back-extracted with EtOAc. The organic layers were combined and washed with $H_2O$ and brine, then dried over $Na_2SO_4$,filtered and concentrated to afford a dark oily residue. The residue was dried under high vacuum and used in the next step without further purification.

B. 1-(4-Fluorophenyl)-9,10-dihydro-3-(1-methylethyl)-2-phenanthrenecarboxylic acid, ethyl ester

A mixture of crude 1,5 diketone A, ammonium acetate (6.47 gm, 84.0 mmol) and copper (II) acetate (13.97 gm, 70.0 mmol) in glacial acetic acid (75 ml) was allowed to reflux for 16 hours. The reaction mixture was then cooled and poured into an ice cold mixture of $NH_4OH/H_2O$ (120 ml/150 ml). The mixture was extracted with ether (twice) and the ether extracts were washed with water (twice) and brine, then dried over $MgSO_4$, filtered and concentrated. The dark oily residue was purified by flash chromatography (10% ether/hexane) to afford compound B as a pink solid (5.2 gm, 46% from the naphthalenone), which was contaminated with a higher Rf component.

TLC: Rf 0.5 (10% ether/hexane), UV + Rf 0.62 (10% ether/hexane), UV, fluorescence

C. 1-(4-Fluorophenyl)-9,10-dihydro-3-(1-methylethyl)-2-phenanthrenemethanol

A 0 °C solution of ester B in dry THF (60 ml) was treated with LAH (1.46 gm, 38.52 mmol). Ten minutes after the addition the solution was warmed to room temperature and stirred for two hours. The reaction mixture was then cooled to 0 °C and quenched by dropwise addition of 1.46 ml of $H_2O$ in THF (10 ml), then 1.46 ml 15% NaOH, and finally 3.0 ml $H_2O$. The precipitated aluminum salts were filtered out of solution and washed with EtOAc and ether. The filtrate and washings were combined and washed with water and brine, then dried over $Na_2SO_4$, filtered and concentrated to give an oily residue. Purification by flash chromatography (Merck silica gel, 10% ether/hexane, followed by 10% EtOAc/hexane) afforded a colorless oil which solidified on standing under high vacuum. Recrystallization of the solid from hexane afforded compound C as a white solid (4.0 gm, 89.8%).

m.p. 138 - 139 °C
Rf 0.42 (20% EtOAc/hexane), UV

Analysis for $C_{23}H_{22}NFO*0.18\ H_2O$:

| | | | | |
|---|---|---|---|---|
| Calc'd: | C 78,79 | H 6.43 | N 4.00 | F 5.42 |
| Found: | C 78.80 | H 6.45 | N 3.99 | F 5.24 |

D. 1-(4-Fluorophenyl)-9,10-dihydro-3-(1-methylethyl)-2-phenanthrenecarboxaldehyde

A solution of NMO (0.989 gm, 8.44 mmol) in $cH_2Cl_2$ (30 ml) was allowed to stir over $MgSO_4$ for 30 minutes before being added to a mixture of compound C (1.647 gm, 4.56 mmol) and 4 angstrom sieves (2.67 gm) in $CH_2Cl_2$ (20 ml). The mixture was allowed to stir 5 minutes at room temperature, then TPAP (0.087 gm, 0.25 mmol) was added to the flask and the solution stirred for an additional hour. The mixture was diluted with ether, then filtered through a Celite® pad and washed with ether and EtOAc. The filtrate and washings were combined and concentrated to a dark green solid. The solid was purified by flash chromatography on Merck silcia gel in 15% EtOAc in hexane. The desired fractions were combined and concentrated to a white solid. The white solid was recrystallized from hexane to afford compound D as hard white crystals (1.364 gm, 83%).

m.p. 129-130 °C
TLC: Rf 0.62 (25% EtOAc in hexane)

E. 2-(2,2-Dibromoethenyl)-1-(4-fluorophenyl)-3-(1-methylethyl)phenanthrene

A solution of $CBr_4$ (0.281 gm, 0.836 mmol) in $CH_2Cl_2$ (5 ml) was added dropwise to a 0 °C solution of aldehyde D (0.200 gm, 0.557 mmol) and triphenylphosphine (0.470 gm, 1.78 mmol) in $CH_2Cl_2$ (20 ml). The solution was allowed to stir 30 minutes at 0 °C, then warmed to room temperature over 15 minutes. The solution was then quenched with $NaHCO_3$ and diluted with EtOAc and ether. The organic layer was washed with brine, then dried over $Na_2SO_4$, filtered and concentrated to a yellow solid. The solid was purified by flash chromatography on Merck silica gel in 15% EtOAc in hexane. The desired fractions were combined and concentrated to a white solid. The solid was recrystallized from hexane to afford compound E as large clear crystals (0.254 gm, 88.5%).

m.p. 121-122 °C

Elemental Analysis dor $C_{24}H_{20}NBr_2F$*0.18 $H_2O$:

| | | | | | |
|---|---|---|---|---|---|
| Calc'd: | C 57.14 | H 4.07 | N 2.78 | Br 31.68 | F 3.77 |
| Found: | C 57.53 | H 3.92 | N 2.72 | Br 31.21 | F 3.83 |

F. (S)-4-[[[4-(4-Fluorophenyl)-5,6-dihydro-2-(1-methylethyl)benzo[h]quinolin-3-yl]ethynyl]-methoxyphosphinyl]-3-[[(1,1-dimethylethyl)diphenylsilyl]oxy]butanoic acid, methyl ester

N-BuLi (2.5 M in hexane, 1.31 ml, 3.27 mmol) was added dropwise to a solution of vinyl dibromide E (0.780 gm, 1.56 mmol) in THF (10 ml) at -78 °C under argon. The solution was allowed to stir 1 hour at -78 °C, then cannulated into a THF solution (15 ml) of the chloridate from Example 2, part J, which had also been cooled to -78 °C. The solution was stirred 30 minutes at -78 °C, then quenched with saturated $NH_4Cl$ and warmed to 0 °C, and then saturated $NaHCO_3$ was added. The solution was diluted with ether and the organic layer was washed with brine, then dried over $Na_2SO_4$, filtered and concentrated to a dark orange oil. The oil was purified by flash chromatography on Merck silica gel in 45% EtOAc in hexane. The desired fractions were combined and concentrated to afford compound F as a brown oil (0.385 gm, 33%).

TLC: Rf 0.62 (1:1, EtOAc:hexane)

G. (S)-4-[[[4-(4-Fluorophenyl)-5,6-dihydro-2-(1-methylethyl)benzo[h]quinolin-3-yl]ethynyl]-methoxyphosphinyl]-3-hydroxybutanoic acid, methyl ester

$(Bu)_4NF$ (1 M in THF, 1.55 ml, 1.55 mmol) was added to a solution of compound F (0.385 gm, 0.52 mmol) and HOAc (0.125 gm, 2.08 mmol) in THF (10 ml) under argon and allowed to stir 17 hours at room temperature. The reaction mixture was diluted with EtOAc, then washed with 5% $KHSO_4$ (three times). The aqueous layers were back-extracted with EtOAc (twice). The organic layers were pooled and washed with brine, then dried over $Na_2SO_4$, filtered and concentrated to afford an orange oil. The oil was dissolved in ether (10 ml) and treated with excess $CH_2N_2$. Excess $CH_2N_2$ was removed with a stream of argon, and the solution was concentrated to give an orange oil. The oil was purified by flash chromatography on Merck silica gel in 35% acetone in hexane. The desired fractions were pooled and concentrated to afford compound G as a clear oil (0.195 gm, 70%).

TLC: Rf 0.80 (8:1:1, $CH_2Cl_2$:MeOH:HOAc)

H. (S)-4-[[[4-(4-Fluorophenyl)-5,6-dihydro-2-(1-methylethyl)benzo[h]quinolin-3-yl]ethynyl]hydroxyphosphinyl]-3-hydroxybutanoic acid, disodium salt

A solution of compound G (0.190 gm, 0.357 mmol) in dioxane (10 ml) was treated with NaOH (1 M in $H_2O$, 1.10 ml 1.10 mmol), then heated to 55 °C and allowed to stir 3 hours. The solution was cooled to room temperature and concentrated to a white solid. The solid was dissolved in water and chromatographed on HP-20 resin, eluting first with water (200 ml), then with 50% MeOH in water (400 ml), and finally with MeOH (200 ml). The desired fractions were pooled and concentrated. The resultant white solid was dissolved in water and lyophilized to give Example 11 as a fluffy white solid (0.150 gm, 76%).

m.p. 282 °C (decomp)

**Elemental Analysis for $C_{28}H_{25}FNO_5P$*2 Na*2.25 $H_2O$:**

| | | | | | |
|---|---|---|---|---|---|
| Calc'd: | C 56.81 | H 5.02 | N 2.37 | F 3.21 | P 5.23 |
| Found: | C 56.84 | H 4.83 | N 2.48 | F 3.44 | P 4.84 |

Optical Rotation: $[a]_D$ = +11.4° (MeOH, C = 4.0 mg/ml)

## Example 12

(S)-4-[[[4-(4-Fluoro-2-methylphenyl)-2-(l-methylethyl)-6-phenyl-3-pyridinyl]ethynyl]hydroxyphosphinyl]-3-hydroxybutanoic acid, disodium salt

### A. 4-Fluoro-2-methylbenzaldehyde

n-BuLi (2.6 M in hexane, 57 ml, 143 mmol) was added over 15 minutes to a THF solution (200 ml) of 2-methyl-4-fluorophenylbromide, cooled to -78° C and allowed to stir 1 hour at -78 °C . DMF (26.61 gm, 364 mmol) was then added over 2 minutes, and the solution was allowed to stir another hour. The reaction was quenched with $NH_4Cl$ and warmed to room temperature. 10% HCl was added until the solution became acidic. The mixture was diluted with ether and the organic layer washed with water and brine, then dried over $MgSO_4$, filtered and concentrated to give an orange oil. The oil was purified by distillation (bp = 69 °C at 7 mm Hg) to afford aldehyde A as a clear liquid (13.284 gm, 74%).

TLC: Rf 0.30 (10% EtOAc in hexane)

### B. 3-(4-Fluoro-2-methylphenyl)-l-phenyl-2-propen-l-one

A solution of sodium ethoxide in ethanol (21% by weight, 3.08 gm, 9.50 mmol) was added to a mixture of aldehyde A (13.084 gm, 95 mmol) and acetophenone (11.38 gm, 95 mmol, from Aldrich) in ethanol (125 ml). After stirring at room temperature for 17 hours, the solution was concentrated to about 30 ml to give an orange oil. The oil was dissolved in ether and EtOAc, washed with water and brine, then dried over $MgSO_4$, filtered and concentrated to afford an orange oil. The oil was purified by flash chromatography on Merck silica gel in 5-10% EtOAc in hexane. Fractions containing product B, were pooled and concentrated to afford compound B as a pale yellow oil (17.285 gm, 76%).

TLC: Rf 0.39 (10% EtOAc in hexane)

### C. β-(4-Fluoro-2-methylphenyl)-α-(2-methyl-l-oxopropyl)-Δ-oxobenzenepentanoic acid, ethyl ester

A solution of sodium ethoxide in ethanol (21% by weight, 3.49 gm, 10.8 mmol) was added to a mixture of enone B (17.280 gm, 72 mmol) and ethylisobutyrylacetate (17.086 gm, 108 mmol) in ethanol (300 ml) and stirred at room temperature for 6 hours, the solution was concentrated to about 50 ml, dissolved in ether and EtOAc, washed with saturated $NH_4Cl$, water and brine, then dried over $MgSO_4$, filtered and concentrated to afford an orange oil. The oil was purified by flash chromatography on Merck silica gel in 5-10% EtOAc in hexane. Fractions containing the product were pooled and concentrated to give compound C as a white, crystalline solid (23.905 gm, 87%).

m.p. = 73-77 °C

TLC: Rf 0.38, 0.43 (15% EtOAc in hexane)

### D. 4-(4-Fluoro-2-methylphenyl)-2-(l-methylethyl)-6-phenyl-3-pyridinecarboxylic acid, ethyl ester

$NH_4OAc$ (14.47 gm, 188 mmol) and $Cu(OAc)_2$ (31.23 gm, 156 mmol) were added to an acetic acid solution (100 ml) of 1,5 diketone C (23.905 gm, 62.6 mmol). The resulting mixture was allowed to reflux for 13 hours while stirring under argon. The solution was then poured into an ice-cold solution of $NH_4OH/H_2O$ (125 ml/150 ml). The mixture was extracted with EtOAc (twice), washed with water and saturated NaCl, then dried over $MgSO_4$, filtered and concentrated to afford an orange oil. The oil was purified by flash chromatography on Merck silica in 5% EtOAc in hexane. Those fractions containing pure product D were pooled and concentrated to give a clear oil (19.140 gm, 81%).

TLC: Rf 0.63 (10% EtOAc in hexane)

E. 4-(4-Fluoro-2-methylphenyl)-2-(l-methylethyl)-6-phenyl-3-pyridinemethanol

A THF solution (20 ml) of ester D (19.140 gm, 50.8 mmol) was cooled to 0° C and treated with $LiAlH_4$ - (5.78 gm, 152 mmol). After stirring 15 minutes at 0° C, the solution was warmed to room temperature and stirred for an additional 3 hours. The solution was cooled to 0° C and quenched by dropwise addition of 5.8 ml of water followed by 5.8 ml of 15% NaOH, then 17.0 ml water. The aluminum paste was filtered out of solution and the filtrate concentrated to a white solid. Recrystallization of the solid from EtOAc/hexane gave compound E as hard, white crystals (15.072 gm, 94%).

m.p. 134-135 ° C
TLC: Rf 0.62 (27% EtOAc in hexane)

| Elemental Analysis for $C_{22}H_{22}NFO$: | |
|---|---|
| Calc'd | C, 78.78; H, 6.61; N, 4.18; F, 5.66 |
| Found | C, 78.85; H, 6.68; N, 4.18; F, 5.54 |

F. 4-(4-Fluoro-2-methylphenyl)-2-(l-methylethyl)-6-phenyl-3-pyridinecarboxaldehyde

DMSO (9.66 gm, 124 mmol) was added to a -78° C solution of oxalyl chloride (7.85 gm, 61.9 mmol) in methylene chloride (200 ml). The mixture was allowed to stir 20 minutes at -78° C under argon. Compound E (15.072 gm, 47.5 mmol) was then added dropwise to the flask as a methylene chloride (50 ml) solution. Twenty minutes later, triethyl amine (33 ml, 0.327 mmol) was added dropwise to the cloudy white solution, and the mixture was stirred for 20 minutes, then warmed to room temperature. The solution was diluted with ether and the organic layer washed with water and brine, then dried over $Na_2SO_4$, filtered and concentrated to afford a yellow oil. The compound was purified by flash chromatography on Merck silica gel in 10% EtOAc in hexane. The desired fractions were combined and concentrated to afford product F as a yellow, crystalline solid (13.70 gm, 92%).

m.p. 90-93 ° C
TLC: Rf 0.62 (15% EtOAc in hexane)

| Elemental Analysis for $C_{22}H_{20}NFO$: | |
|---|---|
| Calc'd | C, 79.26; H, 6.05; N, 4.20; F, 5.70 |
| Found | C, 79.27; H, 6.06; N, 4.20; F, 5.82 |

G. 3-(2,2-Dibromoethenyl)-4-(4-fluoro-2-methylphenyl)-2-(l-methylethyl)-6-phenylpyridine

A solution of $CBr_4$ (21.95 gm, 65.2 mmol) in methylene chloride (75 ml) was added over 20 minutes to a 0° C solution of aldehyde F (13.70 gm, 43.5 mmol) and triphenyl phosphine (36.51 gm, 139 mmol) in methylene chloride (250 ml). The solution stirred at 0° C for an additional 30 minutes, then warmed to room temperature over 40 minutes. Saturated $NaHCO_3$ was then added to quench the reaction. The organic layer was washed with brine, then dried over $Na_2SO_4$, filtered and concentrated to about 100 ml. The solution was purified by flash chromatography on Merck silica gel in 15% EtOAc in hexane. The desired fractions were pooled and concentrated to afford a yellowish solid. The solid was recrystallized from hexane to afford product G as hard, clear crystals (16.560 gm, 81%).

m.p. 128-129° C.
TLC: Rf 0.37 (5% EtOAc in hexane)

| Elemental Analysis for $C_{23}H_{20}NBr_2F$: | |
|---|---|
| Calc'd | C, 56.47; H, 4.12; N, 2.86; Br, 32.67; F, 3.88 |
| Found | C, 56.71; H, 4.00; N, 2.82; Br, 32.46; F, 3.86 |

H. 3-Ethynyl-4-(4-fluoro-2-methylphenyl)-2-(l-methylethyl)-6-phenylpyridine

A solution of vinyl dibromide G (8.280 gm, 17.6 mmol) in THF (10 ml) was added dropwise to a solution of n-BuLi (2.5 M in hexane, 14.7 ml, 36.9 mmol) in THF (20 ml) stirring at -78° C under argon. The solution was allowed to stir 1 hour at -78° C, then quenched with saturated $NH_4Cl$ and warmed to 0° C, then saturated $NaHCO_3$ was added to the solution. The mixture was diluted with ether, and the organic layer was washed with brine, dried over $Na_2SO_4$, filtered and concentrated to give yellow oil. The oil was purified by flash chromatography on Merck silica gel in 1% EtOAc in hexane. The desired fractions were combined and concentrated to afford compound H as a white solid (5.514 gm, 95%).

m.p. 72-74° C.
TLC: Rf 0.32 (1% EtOAc in hexane)

| Elemental Analysis for $C_{23}H_{20}NF$: | |
|---|---|
| Calc'd | C, 83.86; H, 6.12; N, 4.25; F, 5.77 |
| Found | C, 83.72; H, 6.02; N, 4.21; F, 5.68 |

I. (S)-4-[[[4-(4-Fluoro-2-methylphenyl)-2-(l-methylethyl)-6-phenyl-3-pyridinyl]ethynyl]methoxyphosphinyl]-3-[-[(l,l-dimethylethyl)diphenylsilyl]oxy]butanoic acid, methyl ester

n-BuLi (2.5 M in hexane, 0.67 ml, 1.67 mmol) was added dropwise to a solution of acetylene H (0.500 gm, 1.52 mmol) in THF (10 ml) at -78° C under argon. The solution was allowed to stir 1 hour at -78° C, then cannulated into a THF solution (15 ml) of the chloridate from Example 2, part J, which had also been cooled to -78° C. The solution was stirred 30 minutes at -78° C, then quenched with saturated $NH_4Cl$ and warmed to 0° C, then saturated $NaHCO_3$ was added to the solution. The solution was diluted with ether and the organic layer was washed with brine, then dried over $Na_2SO_4$, filtered and concentrated to a dark orange oil. The oil was purified by flash chromatography on Merck silica gel in 45% EtOAc in hexane. The desired fractions were combined and concentrated to afford compound I as a beige foam (0.878 gm, 76%).

TLC: Rf 0.47 (40% EtOAc in hexane)

J. (S)-4-[[[4-(4-Fluoro-2-methylphenyl)-2-(l-methylethyl)-6-phenyl-3-pyridinyl]ethynyl]methoxyphosphinyl]-3-hydroxybutanoic acid, methyl ester

$(Bu)_4NF$ (1 M in THF, 3.86 ml, 3.86 mmol) was added to a solution of silyl ether I (0.878 gm, 1.28 mmol) and HOAc (0.307 gm, 5.12 mmol) in THF (20 ml) under argon and allowed to stir 16 hours at room temperature. The reaction mixture was diluted with EtOAc, then washed with 5% $KHSO_4$ (3 times). The aqueous layers were back-extracted with EtOAc (twice). The organic layers were pooled and washed with brine, then dried over $Na_2SO_4$, filtered and concentrated to afford a brown oil. The oil was dissolved in ether (15 ml) and treated with excess $CH_2N_2$. Excess $CH_2N_2$ was removed with a stream of argon, and the solution was concentrated to give a brown-yellow oil. The oil was purified by flash chromatography on Merck silica gel in 50% acetone in hexane. The desired fractions were pooled and concentrated to afford compound J as a clear oil (0.325 gm, 48%).

TLC: Rf 0.63 (50% acetone in hexane)

K. (S)-4-[[[4-(4-Fluoro-2-methylphenyl)-2-(l-methylethyl)-6-phenyl-3-pyridinyl]ethynyl]hydroxyphosphinyl]-3-hydroxybutanoic acid, disodium salt

A solution of compound J (0.325 gm, 0.62 mmol) in dioxane (10 ml) was treated with NaOH (1 M in $H_2O$, 2.20 ml, 2.20 mmol), then heated to 55° C and allowed to stir 2 hours. The solution was then cooled to room temperature and concentrated to a white solid. The solid was dissolved in water and chromatographed on HP-20 resin, eluting first with water (200 ml), then with 50% MeOH in water (400 ml), and finally with MeOH (200 ml). The desired fractions were pooled and concentrated. The resultant white solid was dissolved in water and lyophilized to give Example 12 as a fluffy white solid (0.300 gm, 90%).

m.p. 305° C (decomp).

TLC: Rf 0.64 (6:3:1, n-propanol:$NH_4OH$;$H_2O$)

Elemental Analysis for $C_{27}H_{25}NFNa_2PO_{5x}3.0\ H_2O$:

| | |
|---|---|
| Calc'd | C, 56.41; H, 5.06; N, 2.27; F, 3.08; P, 5.02 |
| Found | C, 56.37; H, 4.89; N, 2.30; F, 3.12; P, 5.36 |

Optical Rotation: $[a]_D$ = +8.8° (MeOH, c = 3.8 mg/ml)

Example 13

(S)-4-[[[2-Cyclopropyl-4-(4-fluorophenyl)-6-phenyl-3-pyridinyl]ethynyl]hydroxyphosphinyl]-3-hydroxybutanoic acid, disodium salt

A. 3-Cyclopropyl-3-oxopropanoic acid, ethyl ester

To a slurry of NaH (60% in mineral oil, 19.200 gm, 0.48 mol) in dry diethyl carbonate (80 ml) was added a solution of methyl cyclopropyl ketone (23.5 ml, 20.00 gm, 0.238 mol) in $Et_2O$ (30 ml). After the addition of approximately 10% of the ketone solution, EtOH (0.25 ml) was added and addition of the ketone continued. Soon after the addition was complete, the reaction became quite exothermic with vigorous $H_2$ evolution. Periodic cooling with an ice bath was required. After one hour, $H_2$ evolution had ceased and the mixture was diluted with $Et_2O$ (200 ml) and hexane (185 ml). The solution was cooled in an ice bath and carefully treated with 5% HCl (10 ml), at which time a thick slurry developed. The solid was collected by filtration and washed with hexane. The solid was then partitioned between $Et_2O$ and 5% HCl until all of the solid had dissolved and the aqueous layer remained acidic. The organic layer was separated and washed with $H_2O$, saturated $NaHCO_3$, and brine, then dried ($MgSO_4$), filtered and stripped. The liquid residue was subjected to flash chromatography (Merck $SiO_2$, 20% EtoAc in hexane) to provide compound A (20.357 gm, 55%) as a yellow liquid.

TLC: $R_f$ 0.24 (20% EtOAc in hexane)

B. 3-Fluorophenyl-l-phenyl-2-propen-l-one

A solution of benzaldehyde (19.220 gm, 181 mmol) and p-fluoroacetophenone (25.000 gm, 181 mmol) in ethanol (200 ml) was treated with sodium methoxide (1.972 gm, 36.5 mmol). A precipitate fell out of solution after 30 minutes. After stirring at room temperature for 15 hours, the solution was treated with 50 ml of $H_2O$, cooled in an ice bath, and filtered. The solid was rinsed with cold ethanol and dried under high vacuum to yield compound B (29.730 gm, 73%) as a yellow crystalline solid.

m.p. 76.3-77.5° C

TLC $R_f$ 0.46 (20% EtOAc in hexane)

Microanalysis for $C_{15}H_{11}FO$:

| | |
|---|---|
| Calc'd | C, 79.63; H, 4.90; F, 8.40 |
| Found | C, 79.57; H, 4.77; F, 8.30 |

C. α-(2-Cyclopropyl-l-oxoethyl)-β-(4-fluorophenyl)-Δ-oxophenylpentanoic acid, ethyl ester

A mixture of compound B (7.280 gm, 32.2 mmol) and compound A (6.00 gm, 38.4 mmol) in absolute EtOH (100 ml) was treated with a solution of EtONa in EtOH (21% by weight solution, 1.28 gm, 4.8 mmol). After stirring at room temperature for 30 minutes, a thick precipitate fell out of solution. Additional EtOH (40 ml) was added and stirring continued for an hour. The solution was treated with acetic acid (0.28 ml) and cooled to 0 °C. The solid was collected by filtration, washed with cold EtOH and hexane, and dried *in vacuo* to give compound C, a mixture of diastereomers, as a white amorphous solid (10.295 gm, 84%).

m.p. 114-117 °C

TLC $R_f$ 0.21 (20% EtOAc in hexane)

D. 2-Cyclopropyl-4-(4-fluorophenyl)-6-phenyl-3-pyridinecarboxylic acid, ethyl ester

A mixture of compound C (10.113 gm, 26.4 mmol), $NH_4OAc$ (6.142 gm, 79.7 mmol), and $Cu(OAc)_2$ - (13.920 gm, 70.0 mmol) in glacial HOAc (70 ml) was heated at 110 °C for 0.5 hours. Additional $NH_4OAc$ (2.10 gm) was added and heating continued for four more hours. The solution was cooled to room temperature and subsequently poured into an ice cold mixture of concentrated $NH_4OH$ (85 ml) in $H_2O$ (150 ml). The mixture was extracted with $Et_2O$ and the $Et_2O$ extract was washed with $H_2O$ (twice) and brine, then dried ($Na_2SO_4$), filtered and stripped to yield a yellow oil that solidified on standing. Recrystallization from EtOAc/hexane afforded compound D as a pale yellow solid (7.810 gm). The mother liquor was flashed (Merck $SiO_2$, 10% EtOAc in hexane) to give additional product, which was recrystallized from hexane (477 mg). Total pooled solids were 8.287 gm, 87% yield.

m.p. 89.5-92 °C

TLC $R_f$ 0.50 (20% EtOAc in hexane)

Microanalysis for $C_{23}H_{20}FNO_2$:

| | |
|---|---|
| Calc'd | C, 76.43; H, 5.58; N, 3.88; F, 5.26 |
| Found | C, 76.40; H, 5.58; N, 3.70; F, 5.15 |

E. 2-Cyclopropyl-4-(4-fluorophenyl)-6-phenyl-3-pyridinemethanol

A cold (0 °C) solution of ester D (7.798 gm, 21.6 mmol) in dry THF (200 ml) was treated with $LiAlH_4$ - (2.378 gm, 62.7 mmol). Ten minutes after the addition, the cooling bath was removed and the mixture was stirred at room temperature for one hour. The solution was recooled to 0 °C and quenched in succession with $H_2O$ (2.5 ml), 10% NaOH (4.0 ml), and $H_2O$ (7.5 ml). The solution was filtered and the salts were washed with EtOAc and $Et_2O$. Removal of the filtrate solvent afforded a solid. The solid was recrystallized from EtOAc/hexane to provide compound E (5.866 gm, 85%) as an amorphous white solid.

m.p. 176-177 °C

TLC $R_f$ 0.53 (40% EtOAc in hexane)

Microanalysis for $C_{21}H_{18}FNO$:

| | |
|---|---|
| Calc'd | C, 78.97; H, 5.68; N, 4.39; F, 5.95 |
| Found | C, 78.72; H, 5.67; N, 4.23; F, 5.67 |

F. 2-Cyclopropyl-4-(4-fluorophenyl)-6-phenyl-3-pyridinecarboxaldehyde

A solution of 4-methylmorpholine-N-oxide (2.982 gm, 25.5 mmol) in $CH_2Cl_2$ (80 ml) was dried over $MgSO_4$ for 15 minutes. The solution was filtered directly into a 500 ml flask, using approximately 20 ml $CH_2Cl_2$ to effect the transfer. The flask was then charged with dry 4 angstrom molecular sieves (10.4 gm), alcohol E (4.012 gm, 12.56 mmol), and tetrapropylammonium perruthenate (222 mg, 0.63 mmol). After stirring at room temperature for 40 minutes, the black solution was diluted with $Et_2O$ (120 ml), stirred for 5

minutes, then filtered through Celite®. The filtrate was stripped and the dark residue was dissolved in $CH_2Cl_2$ and flashed (Merck $SiO_2$, 20% EtoAc in hexane) to give the product as a solid. The solid was recrystallized from EtOAc/hexane (2 crops) to give compound F (3.115 gm, 78%) as a yellow solid.

m.p. 110-113 °C

TLC $R_f$ 0.52 (20% EtOAc in hexane)

G. 3-(2,2-Dibromoethenyl)-2-cyclopropyl-4-(4-fluorophenyl)-6-phenylpyridine

A solution of carbon tetrabromide (4.245 gm, 12.8 mmol) in $CH_2Cl_2$ (10 ml) was added over a 9-minute period to a cold (0 °C) solution of aldehyde F (2.725 gm, 8.6 mmol) and triphenylphosphine (6.761 gm, 25.8 mmol) in $CH_2Cl_2$ (35 ml). After the addition was complete, the cooling bath was removed and the mixture was stirred at room temperature for 25 minutes. The solution was quenched with saturated $NaHCO_3$ and extracted twice with $CH_2Cl_2$. The combined organic layers were dried ($Na_2SO_4$), filtered and concentrated. The concentrate was chromatographed (flash, Merck $SiO_2$, 35% $CH_2Cl_2$ in hexane) to give impure dibromide G as a solid. Recrystallization from EtOAc/hexane (2 crops) provided compound G (3.588 gm, 88%) as fine white needles.

m.p. 170-172 °C

TLC $R_f$ 0.58 (20% EtOAc in hexane)

Microanalysis for $C_{22}H_{16}Br_2FN$:

| | |
|---|---|
| Calc'd | C, 55.84; H, 3.41; N, 2.96; F, 4.02; Br, 33.77 |
| Found | C, 55.95; H, 3.20; N, 2.77; F, 4.21; Br, 33.59 |

H. (S)-4-[[[2-Cyclopropyl-4-(4-fluorophenyl)-6-phenyl-3-pyridinyl]ethynyl]methoxyphosphinyl]-3-[[(l,l-dimethylethyl)diphenylsilyl]oxy]butanoic acid, methyl ester

A solution of dibromide G (1.000 gm, 2.11 mmol) in THF (7 ml) was added dropwise to a solution of n-BuLi (2.5 M in hexane, 1.8 ml, 4.5 mmol) in THF (10 ml) at -78 °C over a 6-minute period. The resulting clear yellow solution was stirred at -78 °C for 40 minutes, then added dropwise via cannula over a 10-minute period to a -78 °C solution of the phosphonochloridate from Example 2, part J, in THF (15 ml). The resulting greenish mixture was stirred at -78 °C for 20 minutes, then quenched with 50% saturated $NH_4Cl$. The solution was warmed to 0 °C, diluted with $H_2O$, and poured into saturated $NaHCO_3$. The aqueous phase was extracted once with $Et_2O$. The $Et_2O$ layer was washed with brine, dried ($Na_2SO_4$),filtered and stripped to give a yellow oil. The residue was chromatographed (flash, Merck $SiO_2$, 40% EtOAc in hexane) to afford compound H as a white foam (1.270 gm, 81%).

TLC $R_f$ 0.25 (40% EtOAc in hexane)

I. (S)-4-[[[2-Cyclopropyl-4-(4-fluorophenyl)-6-phenyl-3-pyridinyl]ethynyl]methoxyphosphinyl]-3-hydroxybutanoic acid, methyl ester

A mixture of compound H (1.255 gm, 1.68 mmol) and HOAc (490 µl, 514 mg, 8.6 mmol) in THF (25 ml) was treated with tetra-n-butylammonium fluoride (1.0 M in THF, 8.2 ml, 8.2 mmol). After stirring at room temperature for 16 hours, the solution was diluted with EtOAc and washed 3 times with 5% $KHSO_4$. The EtOAc layer was washed with brine, dried ($Na_2SO_4$), filtered and stripped to afford a yellow oil. The oil was dissolved in $Et_2O$, cooled to 0 °C and treated with excess diazomethane for 15 minutes. The excess diazomethane was destroyed by the addition of HOAc and the solvent was removed in vacuo. The residue was chromatographed (flash, Merck $SiO_2$, 1:1-acetone: hexane) to afford compound I (746 mg, 87%) as a white foam.

TLC $R_f$ 0.30 (1:1 - acetone:hexane)

J. (S)-4-[[[2-Cyclopropyl-4-(4-fluorophenyl)-6-phenyl-3-pyridinyl]ethynyl]hydroxyphosphinyl]-3-hydroxybutanoic acid, disodium salt

A solution of compound I (730 mg, 1.44 mmol) in dioxane (6 ml) was treated with 1 N NaOH (5.0 ml, 5.0 mmol) at room temperature and the mixture was subsequently heated at 60 °C under argon for 0.5 hour. The solvent was evaporated and the residue was chromatographed on HP-20 resin, eluting in succession with $H_2O$ (200 ml) and 50% MeOH in $H_2O$ (200 ml). The desired fractions were pooled and evaporated and the residue was taken up in $H_2O$ and lyophilized to give Example 13 (674 mg, 84%) as a white solid.

m.p. decomposition at 285 °C
TLC $R_f$ 0.07 (8:1:1- $CH_2Cl_2$:HOAc:MeOH)

**Analysis for $C_{26}H_{21}FNNa_2O_5P$ X 2.0 $H_2O$:**
**Calc'd C, 55.82; H, 4.51; N, 2.50; F, 3.40; P, 5.54**

**Found: C, 55.84; H, 4.28; N, 2.46; F, 3.33; P, 5.50**
**Rotation $[a]_D$ = + 5.6° (MeOH, 9.8 mg in 1.21 ml)**

Example 14

(S)-4-[[[2-Cyclopropyl-4-(4-fluorophenyl)-5-methyl-6-phenyl-3-pyridinyl]ethynyl]hydroxyphosphinyl]-3-hydroxybutanoic acid, disodium salt

A. γ-Ethyl-β-(4-fluorophenyl)-α-(2-methyl-1-oxopropyl)-Δ-oxobenzenepentanoic acid, ethyl ester

A -78 °C solution of $LiN(TMS)_2$ (1.0 M in THF, 23 ml, 23 mmol) and THF (15 ml) was treated with a solution of butyrophenone (3.365 gm, 22.7 mmol) in THF (2 ml) over a 1-minute period. After 60 minutes, a solution of the compound prepared in Example 53, part A (5.000 gm, 18.9 mmol) in THF (5 ml) was added dropwise to the above solution. The mixture was stirred at -78 °C for 45 minutes and then warmed to 0 °C. After 25 minutes, the mixture was quenched with HOAc (1.3 ml), diluted with saturated $NH_4Cl$ and $H_2O$ and subsequently extracted with $Et_2O$. The $Et_2O$ extract was washed with brine, then dried ($Na_2SO_4$), filtered, and stripped to yield a yellow oil. The material was used directly in the next reaction.

TLC: $R_f$ 0.43, 0.33, & 0.30 (20% EtOAc in hexane)

B. 5-Ethyl-4-(4-fluorophenyl)-2-(1-methylethyl)-6-phenyl-3-pyridinecarboxylic acid, ethyl ester

The aforementioned crude mixture of the compound of part A, $NH_4OAc$ (4.455 gm, 58 mmol), and $Cu(OAc)_2$ (9.92 gm, 49.7 mmol) in glacial HOAc (50 ml) were gently refluxed for 25 hours. The solution was cooled to room temperature and subsequently poured into an ice cold mixture of concentrated $NH_4OH$ (78 ml) in $H_2O$ (150 ml). The mixture was extracted once with $Et_2O$ the organic extract was washed with $H_2O$ and brine, then dried ($Na_2SO_4$), filtered and stripped to yield a brown oil. The residue was flashed (Merck $SiO_2$, 10% EtOAc in hexane) to give the title compound as an oil (5.350 gm) which was contaminated with butyrophenone.

TLC: $R_f$ 0.48 (20% EtOAc in hexane)

C. 5-Ethyl-4-(4-fluorophenyl)-2-(1-methylethyl)-6-phenyl-3-pyridinemethanol

A cold (0 °C) solution of the impure ester from part B (5.335 gm) in dry THF (150 ml) was treated with $LiAlH_4$ (1.645 gm, 43.3 mmol). The ice bath was removed and the mixture was stirred at room temperature for 4 hours. The solution was then cooled to 0 °C and quenched in succession with $H_2O$ (1.5 ml), 10% NaOH (2 ml), and $H_2O$ (3.5 ml). The solution was filtered and the salts were washed with EtOAc and THF. The filtrate was stripped of solvent to afford a solid. The residue was recrystallized from hot EtOAc/hexane to provide the title compound (3.540 gm 53% from the compound from Example 53, part A as a white solid.

m.p. 228.5-230 °C

TLC: $R_f$ 0.16 (20% EtOAc in hexane)

| Microanalysis for $C_{23}H_{24}FNO$: | | | | | | | | |
|---|---|---|---|---|---|---|---|---|
| Calc'd: | C | 79.05 | H | 6.92 | N | 4.01 | F | 5.44 |
| Found: | C | 78.98 | H | 6.87 | N | 4.08 | F | 5.28 |

D. 5-Ethyl-4-(4-fluorophenyl)-2-(1-methylethyl)-6-phenyl-3-pyridinecarboxaldehyde

A -78 °C solution of oxalyl chloride (600 μl, 873 mg, 6.9 mmol) in $CH_2Cl_2$ (30 ml) was treated dropwise with a solution of dry DMSO (1.00 ml, 1.10 gm, 14.1 mmol) in $CH_2Cl_2$ (1 ml). After 15 minutes, a solution of the alcohol from part C (2.000 gm, 5.7 mmol) in THF (15 ml) was added dropwise to the above mixture. Fifteen minutes after the addition, TEA (4.5 ml) was added and the mixture was stirred at -78 °C for 3 minutes and then warmed to room temperature. The mixture was diluted with $Et_2O$ and washed twice with $H_2O$ and once with brine. The organic layer was dried ($Na_2SO_4$), filtered, and stripped to give a solid residue. The residue was flashed (LPS-1, $CH_2Cl_2$) to obtain a solid which was recrystallized from hot hexane to give the aldehyde title compound (1.715 gm, 86%) as an amorphous white solid.

m.p. 122-123.8 °C

TLC: $R_f$ 0.51 (20% EtOAc in hexane)

| Microanalysis for $C_{23}H_{22}FNO$: | | | | | | | | |
|---|---|---|---|---|---|---|---|---|
| Calc'd: | C | 79.51 | H | 6.38 | N | 4.03 | F | 5.47 |
| Found: | C | 79.35 | H | 6.40 | N | 4.12 | F | 5.36 |

E. 3-(2,2-Dibromoethenyl)-5-ethyl-4-(4-fluorophenyl)-2-(1-methylethyl)-6-phenylpyridine

A solution of carbon tetrabromide (2.35 gm, 7.1 mmol) in $CH_2Cl_2$ (8 ml) was added over a 7-minute period to a cold (-5 °C) solution of the aldehyde from part D (1.630, 4.7 mmol) and triphenylphosphine (3.750 gm, 14.3 mmol) in $CH_2Cl_2$ (20 ml). After the addition was complete, the cooling bath was removed and the mixture was stirred at room temperature for 25 minutes. The solution was quenched with saturated $NaHCO_3$ and extracted twice with $CH_2Cl_2$. The organic layers were dried ($Na_2SO_4$), filtered and concentrated. The concentrate was chromatographed (flash, Merck $SiO_2$, 30% $CH_2Cl_2$ in hexane followed by 30% hexane in $CH_2Cl_2$) to give the dibromide title compound as a foam, which solidified on standing. Recrystallization of the material from $CH_2Cl_2$/hexane provided the pure title compound (2.237 gm, 95%) as white needles.

m.p. 155.5-157 °C

TLC: $R_f$ 0.40 (10% EtOAc in hexane)

Microanalysis for $C_{24}H_{22}Br_2FN$:

F. 5-Ethyl-3-ethynyl-4-(4-fluorophenyl)-2-(1-methylethyl)-6-phenylpyridine

To a solution of compound E (2.001 gm, 4.0 mmol) in dry THF (15 ml) at -78 °C was added n-BuLi (2.5 M in hexanes, 3.80 ml, 9.5 mmol) over a 1-minute period. After stirring at -78 °C for 50 minutes, the clear green solution was quenched with saturated $NH_4Cl$ and warmed to room temperature. The mixture was diluted with $H_2O$ $Et_2O$, and EtOAc and the mixture was shaken in a separatory funnel. The organic layer contained a finely divided white solid. The organic layer was washed with brine and filtered to give approximately 1 gram of solid. Additional solid was obtained after evaporation of the organic filtrate. The pooled solids were refluxed in EtOAc/acetone, diluted with hexane, and cooled to give the acetylene title compound (1.253 gm, 91%) as a white solid that is poorly soluble in most common organic solvents.

m.p. 245 °C

TLC $R_f$ 0.44 (10% EtOAc in hexane)

| | | | | |
|---|---|---|---|---|
| Analysis for $C_{24}H_{22}FN$: | | | | |
| Calc'd: | C 83.93 | H 6.46 | N 4.08 | F 5.53 |
| Found: | C 83.53 | H 6.28 | N 4.06 | F 5.68 |

G. 5-Ethyl-4-(4-fluorophenyl)-3-(2-iodoethynyl)-2-(1-methylethyl)-6-phenylpyridine

A mixture of compound F (1.222 gm, 3.56 mmol) and AIBN (20 mg) in tri-n-butylstannyl hydride (2.3 ml) was rapidly heated to 140 °C by immersion in a preheated oil bath. Approximately 20 mg of AIBN was added to the reaction mixture one and two hours after heating was initiated. After 3 hours, the mixture was cooled to room temperature, diluted with $Et_2O$ (40 ml) and treated with solid $I_2$ (3.40 gm, 13.4 mmol). The dark reaction mixture was stirred for 50 minutes, then poured into a 50% saturated $NaHCO_3$ solution containing 6.8 gm $Na_2S_2O_3$. The layers were shaken and separated. The ethereal layer was washed successively $H_2O$, 1.7 M $NH_4OH$, $H_2O$ and brine, then dried ($Na_2SO_4$), filtered and stripped to yield an oil. The residue was subjected to flash chromatography (Merck $SiO_2$, 10% EtOAc in hexane) to afford the crude title compound as a solid. Recrystallization from hexane gave the title compound (1.389 gm, 87%) as pale yellow crystals.

m.p. 116.8-118.8 °C
TLC $R_f$ 0.36 (4% EtOAC in hexane)

| | | | | | |
|---|---|---|---|---|---|
| Microanalysis for $C_{24}H_{23}FNI$: | | | | | |
| Calc'd: | C 61.15 | H 4.92 | N 2.97 | F 4.03 | I 26.92 |
| Found: | C 60.34 | H 4.92 | N 2.69 | F NA | I 26.53 |

H. (S,E)-4-[[2-[5-Ethyl-4-(4-fluorophenyl)-2-(1-methylethyl)-6-phenyl-3-pyridinyl]ethenyl]methoxyphosphinyl]-3-[[(1,1-dimethylethyl)diphenylsilyl]oxy]butanoic acid, methyl ester

A solution of the part G vinyl iodide (1.300 gm 2.76 mol) in THF (8 ml) was added over a 5-minute period to a -100 °C solution of t-butyllithium (1.7 M in pentane, 3.40 ml, 5.8 mmol) in THF (8 ml). The resulting solution was stirred for 15 minutes, then added over a 10-minute period to a -100 °C solution of the phosphonochloridate from Example 57, part G in THF (15 ml). The resulting yellow mixture was stirred at minus 100 °C for 10 minutes, then at -78 °C for 30 minutes, then quenched with 50% saturated $NH_4Cl$. The solution was warmed to room temperature, diluted with $H_2O$, and poured into saturated $NaHCO_3$. The aqueous phase was extracted with $Et_2O$. The $Et_2O$ extract was washed with brine, dried ($Na_2SO_4$), filtered and stripped. The resulting yellow-orange oil was chromatographed (flash, LPS-1, 50% EtOAc in hexane) to afford the title compound as a tan foam (1.261 gm, 59%).

TLC $R_f$ 0.21 (40% EtOAc in hexane)

I. (S)-4-[[2-[5-Ethyl-4-(4-fluorophenyl)-2-(l-methylethyl)-6-phenyl-3-pyridinyl]ethyl]methoxyphosphinyl]-3-hydroxybutanoic acid, methyl ester

A mixture of compound H (467 mg, 0.865 mmol) and 10% palladium on carbon (100 mg) in MeOH (20 ml) was shaken under 50 psi of $H_2$ for 3 days. The solution was filtered, stripped, and chromatographed (flash, Merck $SiO_2$, 50% acetone in hexane) to give compound I (411 mg, 88%) as a colorless oil.

TLC $R_f$ 0.39 (1:1 - acetone:hexane)

J. 3-Cyclopropyl-2-[(4-fluorophenyl)methylene]-3-oxopropanoic acid, ethyl ester

A mixture of 4-fluorobenzaldehyde (4.768 gm, 38.4 mmol), ethyl 3-cyclopropyl-3-oxopropionate from part I (6.000 gm, 38.4 mmol), piperidine (380 $\mu$l), and HOAc (75 $\mu$l) was refluxed in benzene (40 ml) with removal of water (Dean-Stark trap) for 16 hours. The cooled mixture was diluted with $Et_2O$ and washed successively with 5% HCl, saturated $NaHCO_3$, $H_2O$, and brine, then dried ($Na_2SO_4$), filtered, and stripped to yield an oil. Distillation of the oil (bp 127-135 °C at 0.2 mm Hg) afforded compound J (8.299 gm, 82%) as a

pale yellow liquid.

TLC $R_f$ 0.31 (20% EtOAc in hexane)

Microanalysis for $C_{15}H_{15}FO_3$:

| | |
|---|---|
| Calcd | C, 68.69; H, 5.76; F, 7.24 |
| Found | C, 68.92; H, 5.90; F, 7.25 |

K. α-(l-Cyclopropyl-l-oxomethyl)-β-(4-fluorophenyl)-γ-methyl-Δ-oxophenylpentanoic acid, ethyl ester

A -78° C solution of $LiN(TMS)_2$ (1.0 M in THF, 38 ml, 38 mmol) in dry THF (40 ml) was treated with a solution of propiophenone (5.015 gm, 37.4 mmol) in THF (6 ml) over a 4-minute period. After 30 minutes, a solution of compound J (7.842 gm, 29.9 mmol) in THF (15 ml) was added dropwise to the above solution. After one hour, the mixture was quenched with acetic acid (2.5 ml) and saturated $NH_4Cl$ and warmed to room temperature. The mixture was diluted with $H_2O$ and subsequently extracted once with $Et_2O$. The $Et_2O$ extract was washed with brine, dried ($Na_2SO_4$), filtered, and stripped to give an oil. The oil was transferred to a round bottom flask which was fitted with a short path distillation apparatus and the oil was heated at 65° C and 0.2 mm Hg to remove the volatiles and excess propiophenone. The pot residue, crude compound K, was used directly in the next reaction.

L. 2-Cyclopropyl-4-(4-fluorophenyl)-5-methyl-6-phenyl-3-pyridinecarboxylic acid, ethyl ester

A mixture of crude compound K, $NH_4OAc$ (9.210 gm, 119.5 mmol), and $Cu(OAc)_2$ (17.921 gm, 89.8 mmol) in glacial HOAc (80 ml) was heated at 110° C for 21 hours. The solution was cooled to room temperature and subsequently poured into an ice cold mixture of concentrated $NH_4OH$ (100 ml) in $H_2O$ (200 ml). The mixture was extracted twice with $Et_2O$ and the pooled $Et_2O$ extracts were washed with $H_2O$ and brine, then dried ($Na_2SO_4$), filtered and stripped to yield a brown solid. The solid was dissolved in $CH_2Cl_2$ and flashed (Merck $SiO_2$, 20% EtOAc in hexane) to give compound C as a reddish oil which readily solidified. Recrystallization from EtOAc/hexane (2 crops) afforded compound L (9.615 gm, 86%) as white crystals.

m.p. 113-114.5° C

TLC $R_f$ 0.46 (20% EtOAc in hexane)

**Microanalysis for $C_{24}H_{22}FNO_2$:**

| | |
|---|---|
| Calc'd | C, 76.78; H, 5.91; N, 3.73; F, 5.06 |
| Found | C, 76.68; H, 5.92; N, 3.63; F, 5.06 |

M. 2-Cyclopropyl-4-(4-fluorophenyl)-5-methyl-6-phenyl-3-pyridinemethanol

A cold (0° C) solution of ester L (8.942 gm, 23.8 mmol) in dry THF (200 ml) was treated with $LiAlH_4$ - (2.701 gm, 71.2 mmol). Ten minutes after the addition, the cooling bath was removed and the mixture was stirred at room temperature for 1.5 hours. The solution was recooled to 0° C and quenched in succession with $H_2O$ (3.0 ml), 10% NaOH (4.5 ml), and $H_2O$ (8.5 ml). The solution was filtered and the salts were washed with EtOAc and $Et_2O$. Removal of the filtrate solvent afforded an oily solid residue. The residue was dissolved in $Et_2O$, diluted with hexane, heated to remove the $Et_2O$, and cooled to provide compound M (6.548 gm, 83%) as an amorphous white solid.

m.p. 140.5-142° C

TLC $R_f$ 0.18 (20% EtOAc in hexane)

Microanalysis for $C_{22}H_{20}FNO$:

Calc'd C 79.25 H 6.05 N 4.20 F 5.70

Found C 79.14 H 5.99 N 4.15 F 5.73

N. 2-Cyclopropyl-4-(4-fluorophenyl)-5-methyl-6-phenyl-3-pyridinecarboxaldehyde

A solution of 4-methylmorpholine-N-oxide (4.002 gm, 34.2 mmol) in $CH_2Cl_2$ (130 ml) was dried over $MgSO_4$ for 15 minutes. The solution was filtered directly into a 500-ml flask, using approximately 30 ml $CH_2Cl_2$ to effect the transfer. The flask was then charged with dry 4 angstrom molecular sieves (16 gm), alcohol M (5.686 gm, 17.05 mmol), and tetrapropylammonium perruthenate (301 mg, 0.86 mmol). After stirring at room temperature for 30 minutes, the black solution was diluted with $Et_2O$ (200 ml), stirred for 5 minutes, then filtered through a plug of silica gel (Merck, 65 x 30 mm), washing with $Et_2O$. The filtrate was stripped to give a pale yellow solid. The solid was recrystallized from EtOAc/hexane to give compound N (3.982 gm) as white crystals. Flashing the mother liquor (Merck $SiO_2$, 20% EtOAc in hexane) gave additional product, which was recrystallized from hexane (499 mg). Total pooled solids were 4.481 gm (79%).

m.p. 137-139 °C

TLC $R_f$ 0.50 (20% EtOAc in hexane)

Microanalysis for $C_{22}H_{18}FNO$:

Calc'd C, 79.74; H, 5.47; N, 4.23; F, 5.73

Found C, 79.32; H, 5.49; N, 4.13; F, 5.65

O. 2-Cyclopropyl-3-(2,2-dibromoethenyl)-4-(4-fluorophenyl)-5-methyl-6-phenylpyridine

A solution of carbon tetrabromide (5.810 gm, 17.5 mmol) in $CH_2Cl_2$ (9 ml) was added over a 12-minute period to a cold (0 °C) solution of aldehyde N (3.864 gm, 11.7 mmol) and triphenylphosphine (9.191 gm, 35.0 mmol) in $CH_2Cl_2$ (60 ml). After the addition was complete, the cooling bath was removed and the mixture was stirred at room temperature for 20 minutes. The solution was quenched with saturated $NaHCO_3$ (40 ml) and extracted twice with $CH_2Cl_2$. The combined organic layers were dried ($Na_2SO_4$), filtered and concentrated. The concentrate was chromatographed (flash, Merck $SiO_2$, 40% $CH_2Cl_2$ in hexane) and the pure desired fractions were stripped to give a solid. Recrystallization from EtOAc/hexane (2 crops) provided compound 0 (5.432 gm, 96%) as white crystals.

m.p. 155-157 °C

TLC $R_f$ 0.58 (20% EtOAc in hexane)

Microanalysis for $C_{23}H_{18}Br_2FN$:

Calc'd C, 56.70; H, 3.72; N, 2.88; F, 3.90; Br, 32.80

Found C, 56.55; H, 3.68; N, 2.82; F, 3.89; Br, 32.72

P. (S)-4-[[[2-Cyclopropyl-4-(4-fluorophenyl)-5-methyl-6-phenyl-3-pyridinyl]ethynyl]methoxyphosphinyl]-3-[[(1,1-dimethylethyl)diphenylsilyl]oxy]butanoic acid, methyl ester

A solution of dibromide O (1.000 gm, 2.05 mmol) in THF (5 ml) was added dropwise to a solution of n-BuLi (2.5 M in hexane, 1.73 ml, 4.3 mmol) in THF (10 ml) at -78 °C over a 10-minute period. The resulting

pale yellow solution was stirred at -78 °C for one hour, then added dropwise via cannula over a 12-minute period to a -78 °C solution of the phosphonochloridate from Example 2, part J, in THF (15 ml). The resulting mixture was stirred at -78 °C for 30 minutes, then quenched with 50% saturated $NH_4Cl$. The solution was warmed to 0 °C, diluted with $H_2O$, and poured into saturated $NaHCO_3$. The aqueous phase was extracted once with $Et_2O$. The $Et_2O$ layer was washed with brine, dried ($Na_2SO_4$), filtered and stripped to give an oil. The residue was chromatographed (flash, Merck $SiO_2$, 50% EtOAc in hexane) to afford compound P as a white foam (1.158 gm, 74%).

TLC $R_f$ 0.27 (40% EtOAc in hexane)

Q. (S)-4-[[[2-Cyclopropyl-4-(4-fluorophenyl)-5-methyl-6-phenyl-3-pyridinyl]ethynyl]methoxyphosphinyl]-3-hydroxybutanoic acid, methyl ester

A mixture of compound P (1.148 gm, 1.51 mmol) and HOAc (460 µl 482 mg, 8.0 mmol) in THF (25 ml) was treated with tetra-n-butylammonium fluoride (1.0 M in THF, 7.6 ml, 7.6 mmol). After stirring at room temperature for 17 hours, the solution was diluted with EtOAc and washed 3 times with 5% $KHSO_4$. The pooled aqueous extracts were back-extracted once with EtOAc and the combined EtOAc layers were washed with brine, dried ($Na_2SO_4$), filtered and stripped to afford a yellow oil. The oil was dissolved in $Et_2O$, cooled to 0 °C and treated with excess diazomethane for 15 minutes. The excess diazomethane was destroyed by the addition of HOAc and the solvent was removed in vacuo. The residue was chromatographed (flash, Merck $SiO_2$, 40% acetone in hexane followed by 40% hexane in acetone) to afford compound Q (684 mg, 87%) as a white foam.

TLC $R_f$ 0.37 (1:1 - acetone:hexane)

R. (S-)4-[[[2-Cyclopropyl-4-(4-fluorophenyl)-5-methyl-6-phenyl-3-pyridinyl]ethynyl]hydroxyphosphinyl]-3-hydroxybutanoic acid, disodium salt

A solution of compound Q (672 mg, 1.29 mmol) in dioxane (7 ml) was treated with 1 N NaOH (4.5 ml, 4.5 mmol) at room temperature and the mixture was subsequently heated at 60 °C under argon for one hour. The solvent was evaporated and the solid residue was dissolved in warm $H_2O$ and chromatographed on HP-20, eluting in succession with $H_2O$ (150 ml) and 50% MeOH in $H_2O$ (200 ml). The desired fractions were pooled and evaporated and the residue was taken up in $H_2O$ and lyophilized to give Example 14 (640 mg, 89%) as a white solid.

m.p. decomposition at 295 °C

TLC $R_f$ 0.07 (8:1:1- $CH_2Cl_2$:HOAc:MeOH)

Calc'd for $C_{27}H_{23}FNNa_2O_5P$ X 1.25 $H_2O$:

| | |
|---|---|
| Calc'd | C, 57.92; H, 4.59; N, 2.50; F, 3.39; P, 5.53 |
| Found | C, 58.05; H, 4.50; N, 2.35; F, 3.38; P, 5.72 |

Rotation $[a]_D$ = + 8.8° (MeOH, 7.6 mg in 1.22 ml)

## Example 15

(S,E)-4-[[2-[4-(4-Fluoro-2-methylphenyl)-2-(1-methylethyl)-6-phenyl-3-pyridinyl]ethenyl]hydroxyphosphinyl]-3-hydroxybutanoic acid, disodium salt

A. (E)-3-(2-Iodoethenyl)-4-fluoro-2-methylphenyl)-2-(1-methylethyl)-6-phenylpyridine

A mixture of (E)-3-[2-(Tributylstannyl)ethenyl]-4-(4-fluoro-2-methylphenyl)-2-(1-methylethyl)-6-phenylpyridine (7.18 gm, 24.7 mmol), acetylene 1 (4.500 gm, 13.7 mmol) and AIBN (0.084 gm, 5.1 mmol) were heated at 140 °C for 1.5 hours. The solution was cooled to room temperature and diluted with ether (30

ml). Iodine (7.16 gm, 27.1 mmol) was added and an unexpectedly strong exothermic reaction occurred and approximately half the solution foamed out of the flask. The flask was recapped and allowed to stir 16 hours. The solution was then quenched with 10% $Na_2S_2O_3$ insaturated $NaHCO_3$. The solution was diluted with ether, washed with 10% $Na_2S_2O_3$ in saturated $NaHCO_3$ (twice) and brine, then dried over $Na_2SO_4$, filtered and concentrated to a yellow oil. The oil was purified by flash chromatography on Merck silica gel in 1% EtOAc in hexane. The desired fractions were combined and concentrated to afford a white solid, which was recrystallized from hexane to give product A as hard, off-white crystals (2.830 gm, 45%).

m.p. 115-117 °C

Elemental Analysis for $C_{23}H_{21}FIO_5$:

| | | | | |
|---|---|---|---|---|
| Calc'd: | C 60.4 | H 4.63 | N 3.06 | F 4.15 |
| | I 27.75 | | | |
| Found: | C 60.87 | H 4.58 | N 2.93 | F 4.12 |
| | I 27.51 | | | |

B. (S,E)-4-[[2-[4-(4-Fluoro-2-methylphenyl)-2-(1-methylethyl)-6-phenyl-3-pyridinyl]ethenyl]-methoxyphosphinyl]-3-[[(1,1-dimethylethyl)diphenylsilyl]oxy]butanoic acid, methyl ester

Vinyl iodide A (2.380 gm, 5.19 mmol) was added dropwise to a -78 °C solution of t-BuLi (1.7 M in pentane, 6.11 ml, 10.4 mmol) in THF (10 ml) stirring under argon. The solution was allowed to stir 15 minutes at -78 °C, then cooled to -100 °C and cannulated into a THF solution (15 ml) of the chloridate from Example 2, part J, also cooled to -100 °C. The solution was stirred 15 minutes at -100 °C, quenched with saturated $NH_4Cl$ and warmed to 0 °C, after which saturated $NaHCO_3$ was added. The solution was diluted with ether and the organic layer washed with brine, dried over $Na_2SO_4$, filtered and concentrated to give a dark orange oil. The oil was purified by flash chromatography on Merck silica gel in 60% EtOAc in hexane. Pure product fractions were combined and evaporated to afford compound B as a white, fluffy foam (1.230 gm, 35%).

TLC: Rf 0.39 (40% EtOAc in hexane)

C. (S,E)-4-[[2-[4-(4-Fluoro-2-methylphenyl)-2-(1-methylethyl)-6-phenyl-3-pyridinyl]ethenyl]-methoxyphosphinyl]-3-hydroxybutanoic acid, methyl ester

$(Bu)_4NF$ (1 M in THF, 5.40 ml, 5.40 mmol) was added to a solution of silyl ether B (1.230 gm, 1.80 mmol) and HOAc (0.54 ml, 9.43 mmol) in THF (20 ml) and allowed to stir 17 hours at room temperature. Additional TBAF (1.80 ml, 1.80 mmol and HoAC (0.172 ml, 3.2 mmol) were added to the reaction mixture, which was allowed to stir under argon another 3 hours. The solution was diluted with EtOAc and washed with 5% $KHSO_4$ (three times). The aqueous layers were extracted with EtOAc (twice) and the organic layers were combined and washed with brine, then dried over $Na_2SO_4$, filtered and concentrated to give a clear oil. The oil was purified by flash chromatography on Merck silica gel in 35% acetone in hexane. Product fractions were pooled and concentrated to afford compound C as a clear oil (0.785 gm, 83%).

TLC: Rf 0.54 (50% acetone in hexane)

D. (S,E)-4-[[2-[4-(4-Fluoro-2-methylphenyl)-2-(1-methylethyl)-6-phenyl-3-pyridinyl]ethenyl]-hydroxyphosphinyl]-3-hydroxybutanoic acid, disodium salt

NaOH (1 M in $H_2O$, 2.26 ml, 2.26 mmol) was added dropwise to a solution of compound C (0.335 gm, 0.64 mmol) in dioxane (10 ml), allowed to stir at room temperature for 1.5 hours, and then heated to 60 °C and stirred for 1 hour. The mixture was then concentrated to a white solid. The solid was dissolved in water and chromatographed on HP-20 resin, eluting first with water (200 ml), then with 50% MeOH in water (400 ml), and finally with MeOH (200 ml). The desired fractions were pooled, concentrated, and the resultant white solid dissolved in water and lyophilized to give Example 15 as a fluffy white solid (0.300 gm, 90%).

m.p. 312 °C (decomp)

TLC: Rf 0.61 (6:3:1, n-propanol: $NH_4$:OH:$H_2O$)

Elemental Analysis for $C_{27}H_{27}NFPO_5Na_2$ X 2.20 $H_2O$:

| | | | | |
|---|---|---|---|---|
| Calc'd: | C 55.81 | H 5.45 | N 2.41 | F 3.27 |
| | P 5.33 | | | |
| Found: | C 55.78 | H 5.40 | N 2.44 | F 3.12 |
| | P 5.32 | | | |

Optical Rotation: $[a]_D$ = +1.6° (MeOH, c = 4.1 mg/ml)

Example 16

(S)-4-[[2-[4-(4-Fluoro-2-methylphenyl)-2-(1-methylethyl)-6-phenyl-3-pyridinyl]ethyl]hydroxyphosphinyl]-3-hydroxybutanoic acid, disodium salt

A. (S)-4-[[2-[4-(4-Fluoro-2-methylphenyl)2-(1-methylethyl)-6-phenyl-3-pyridinyl]ethyl]methoxyphosphinyl]-3-hydroxybutanoic acid, methyl ester

10% Pd/C (0.185 gm, 0.174 mmol) was added to a Parr bottle containing an argon-purged, MeOH (50 ml) solution of compound C from Example 15 (0.450 gm, 0.854 mmol). The bottle was then placed under 50 psi of hydrogen and shaken for 3 hours. The solution was then filtered through a Celite® pad, which was washed repeatedly with methanol. The filtrate was concentrated to give a clear oil. The oil was purified by flash chromatography on Merck silica gel in 60% acetone in hexane. After concentrating the desired fractions, product A was obtained as a white foam (0.300 gm, 67%).

TLC: Rf 0.50 (50% acetone in hexane)

B. (S)-4-[[2-[4-(4-Fluoro-2-methylphenyl)2-(1-methylethyl)-6-phenyl-3-pyridinyl]ethyl]hydroxyphosphinyl]-3-hydroxybutanoic acid, disodium salt

Di-ester A (0.300 gm, 0.57 mmol) was dissolved in dioxane (10 ml) and treated with NaOH (1 M in $H_2O$ 1.71 ml, 1.71 mmol). The solution was heated to 55 °C and allowed to stir 3 hours. The solution was concentrated to a white solid. The solid was dissolved in water and chromatographed on HP-20 resin, eluting first with water (200 ml), then with 50% MeOH in water (400 ml), and finally, MeOH (200 ml). The desired fractions were pooled and concentrated, and the resultant white solid was dissolved in water and lyophilized to give Example 16 as a fluffy white solid (0.290 gm, 94%).

m.p. 312 °C (decomp)

TLC: Rf 0.67 (6:3:1, n-propanol: $NH_4OH$, $H_2O$)

Elemental Analysis for $C_{27}H_{29}NFNa_2PO_5$*1.75 $H_2O$:

| | | | | | |
|---|---|---|---|---|---|
| Calc'd: | C 56.39 | H 5.70 | N 2.44 | F 3.30 | P 5.39 |
| Found: | C 56.71 | H 5.52 | N 2.35 | F 3.11 | P 5.23 |

Optical Rotation: $[a]_D$ = -1.9° (MeOH, c = 4.3 mg/ml)

Example 17

(S)-4-[[[2-Cyclopropyl-4-(4-fluorophenyl)-6-phenyl-3-pyridinyl]ethynyl]hydroxyphosphinyl]-3-hydroxybutanoic acid, disodium salt

A. 3-Cyclopropyl-3-oxopropanoic acid, ethyl ester

To a slurry of NaH (60% in mineral oil, 19.200 g, 0.48 mol) in dry diethyl carbonate (80 ml) was added a solution of methyl cyclopropyl ketone (23.5 ml, 20.00 g, 0.238 mol) in $Et_2O$ (30 ml). After the addition of approximately 10% of the ketone solution, EtOH (0.25 ml) was added and the addition of the ketone continued. Soon after the addition was complete, the reaction became quite exothermic with vigorous $H_2$ evolution. Periodic cooling with an ice bath was required. After one hour, $H_2$ evolution had ceased and the mixture was diluted with $Et_2O$ (200 ml) and hexane (185 ml). The solution was cooled in an ice bath and carefully treated with 5% HCl (10 ml), at which time a thick slurry developed. The solid was collected by filtration and washed with hexane. The solid was then partitioned between $Et_2O$ and 5% HCl until all of the solid had dissolved and the aqueous layer remained acidic. The organic layer was separated and washed with $H_2O$, saturated $NaHO_3$, and brine, then dried ($MgSO_4$), filtered and stripped. The liquid residue was subjected to flash chromatography (Merck $SiO_2$, 20% EtOAc in hexane) to provide compound A (20.357 g, 55%) as a yellow liquid.

TLC: $R_f$ = 0.24 (20% EtOAc in hexane).

B. 3-(4-Fluorophenyl)-1-phenyl-2-propen-1-one

A solution of benzaldehyde (19.220 g, 181 mmol) and p-fluoroacetophenone (25.000 g, 181 mmol) in ethanol (200 ml) was treated with sodium methoxide (1.972 g, 36.5 mmol). A precipitate fell out of solution after 30 minutes. After stirring at room temperature for 15 hours, the solution was treated with 50 ml of $H_2O$, cooled in an ice bath, and filtered. The solid was rinsed with cold ethanol and dried under high vacuum to yield compound B (29.730 g, 73%) as a yellow crystalline solid.

Melting point: 76.3-77.5°

TLC: $R_f$ = 0.46 (20% EtOAc in hexane)

**Microanalysis for $C_{15}H_{11}FO$:**

| | | | |
|---|---|---|---|
| **Calc'd:** | **C 79.63,** | **H 4.90,** | **F 8.40** |
| **Found:** | **C 79.57,** | **H 4.77,** | **F 8.30** |

C. α-Cyclopropylcarbonyl-β-(4-fluorophenyl)-Δ-oxobenzenepentanoic acid, ethyl ester

A mixture of compound B (7.280 g, 32.2 mmol) and compound A (6.00 g, 38.4 mmol) in absolute EtOH (100 ml) was treated with a solution of EtONa in EtOH (21% by weight solution, 1.28 g, 4.8 mmol). After stirring at room temperature for 30 minutes, a thick precipitate fell out of solution. Additional EtOH (40 ml) was added and stirring continued for an hour. The solution was treated with acetic acid (0.28 ml) and cooled to 0° C. The solid was collected by filtration, washed with cold EtOH and hexane, and dried *in vacuo* to give compound C, a mixture of diastereomers, as a white amorphous solid (10.295 g, 84%).

m.p. 114-117° C

TLC: $R_f$ 0.21 (20% EtOAc in hexane)

D. 2-Cyclopropyl-4-(4-fluorophenyl)-6-phenyl-3-pyridinecarboxylic acid, ethyl ester

A mixture of compound C (10.113 g, 26.4 mmol), $NH_4OAC$ (6.142 g, 79.7 mmol) and $Cu(OAc)_2$ (13.920 g, 70.0 mmol) in glacial HOAc (70 ml) was heated at 110° C for 0.5 hours. Additional $NH_4OAc$ (2.10 g) was added, and heating continued for four more hours. The solution was cooled to room temperature and subsequently poured into an ice cold mixture of concentrated $NH_4OH$ (85 ml) in $H_2O$ (150 ml). The mixture was extracted with $Et_2O$ and the $Et_2O$ extract was washed with $H_2O$ and brine, then dried ($Na_2SO_4$), filtered and stripped to yield a yellow oil which solidified on standing. Recrystallization from EtOAc/hexane afforded compound D as a pale yellow solid (7.810 g). The mother liquor was flashed (Merck $SiO_2$, 10% EtOAc in hexane) to five additional product, which was recrystallized from hexane (477 mg). Total pooled solids (8.287 g, 87%).

m.p. 89.5-92° C

TLC: $R_f$0.50 (20% EtOAc in hexane)

**Microanalysis for $C_{23}H_{20}FNO_2$:**

| | | | | |
|---|---|---|---|---|
| Calc'd: | C 76.43 | H 5.58 | N 3.88 | F 5.26 |
| | C 76.40 | H 5.58 | N 3.70 | F 5.15 |

E. 2-Cyclopropyl-4-(4-fluorophenyl)-6-phenyl-3-pyridinemethanol

A cold (0 °C) solution of ester D (7.798 g, 21.6 mmol) in dry THF (200 ml) was treated with $LiAlH_4$ - (2.378 g, 62.7 mmol). Ten minutes after the addition, the cooling bath was removed and the mixture was stirred at room temperature for one hour. The solution was recooled to 0 °C and quenched in succession with $H_2O$ (2.5 ml), 10% NaOH (4.0 ml), and $H_2O$ (7.5 ml). The solution was filtered and the salts were washed with EtOAc. Removal of the filtrate solvent afforded a solid. The solid was recrystallized from EtOAc and $Et_2O$. Removal of the filtrate solvent afforded a solid. The solid was recrystallized from EtOAc/hexane to provide compound E (5.866 g, 85%) as an amorphous white solid.

m.p. 176 - 177 °C

TLC: $R_f$ 0.53 (40% EtOAc in hexane)

**Microanalysis for $C_{21}H_{18}FNO$:**

| | | | | |
|---|---|---|---|---|
| Calc'd: | C 78.97 | H 5.68 | N 4.39 | F 5.95 |
| Found: | C 78.72 | H 5.67 | N 4.23 | F 5.67 |

F. 2-Cyclopropyl-4-(4-fluorophenyl)-6-phenyl-3-pyridinecarboxaldehyde

A solution of 4-methylmorpholine N-oxide (2.982 g, 25.5 mmol) in $CH_2Cl_2$ (80 ml) was dried over $MgSO_4$ for 15 minutes. The solution was filtered directly into a 500 ml flask, using approximately 20 ml $CH_2Cl_2$ to effect transfer. The flask was then charged with dry 4A molecular sieves (10.4 g), alcohol E (4.012 g, 12.56 mmol), and tetrapropylammonium perruthenate (222 mg, 0.63 mmol). After stirring at room temperature for 40 minutes, the black solution was diluted with $Et_2O$ (120 ml), stirred for 5 minutes, then filtered through Celite®. The filtrate was stripped and the dark residue was dissolved in $CH_2Cl_2$ and flashed (Merck $SiO_2$, 20% EtOAc in hexane) to give the product as a solid. The solid was recrystallized from EtOAc/hexane (2 crops) to give compound F (3.115 g, 78%) as a yellow solid.

m.p. 110-113 °C

TLC: $R_f$0.52 (20% EtOAc in hexane)

G. 2-Cyclopropyl-3-(2,2-dibromoethenyl)-4-(4-fluorophenyl)-6-phenylpyridine

A solution of carbon tetrabromide (4.245 g, 12.8 mmol) in $CH_2Cl_2$ (10 ml) was added over a 9 minute period to a cold (0 °C) solution of aldehyde F (2.725 g, 8.6 mmol) and triphenylphosphine (6.761 g, 25.8 mmol) in $CH_2Cl_2$ (35 ml). After the addition was complete, the cooling bath was removed and the mixture was stirred at room temperature for 25 minutes. The solution was quenched with saturated $NaHCO_3$ and extracted 2 times with $CH_2Cl_2$. The combined organic layers were dried ($Na_2SO_4$), filtered and concentrated. The concentrate was chromatographed (flash, Merck $SiO_2$, 35% $CH_2Cl_2$ in hexane) to give impure dibromide G as a solid. Recrystallization from EtOAc/hexane (2 crops) provided compound G (3.588 g, 88%) as fine white needles.

H. (S)-4-[[[2-Cyclopropyl-4-(4-fluorophenyl)-6-phenyl-3-pyridinyl]ethynyl]methoxyphosphinyl]-3-[[(1,1-dimethylethyl)diphenylsilyl]oxy]butanoic acid, ethyl ester

A solution of dibromide G (1.000 g, 2.11 mmol) in THF (7 ml) was added dropwise to a solution of n-BuLi (2.5 M in hexane, 1.8 ml, 4.5 mmol) in THF (10 ml) at -78 °C over a 6-minute period. The resulting clear yellow solution was stirred at -78 °C for 40 minutes, then added dropwise via cannula over a 10-minute period to a -78 °C solution of the phosphonochloridate from Example 2, part J in THF (15 ml). The resulting greenish mixture was stirred at -78 °C for 20 minutes, then quenched with 50% saturated $NH_4Cl$.

The solution was warmed to 0° C, diluted with $H_2O$, and poured into saturated $NaHCO_3$. The aqueous phase was extracted once with $Et_2O$. The $Et_2O$ layer was washed with brine, dried ($Na_2SO_4$), filtered and stripped to give a yellow oil. The residue was chromatographed (flash, Merck $SiO_2$, 40% EtOAc in hexane) to afford compound H as a white foam (1.270 g, 81%).

TLC: $R_f$ = 0.25 (40% EtOAc in hexane).

I. (S)-4-[[[2-Cyclopropyl-4-(4-fluorophenyl)-6-phenyl-3-pyridinyl]ethynyl]methoxyphosphinyl]-3-hydroxybutanoic acid, ethyl ester

A mixture of compound H (1.255 g, 1.68 mmol) and HOAc (490 µl, 5.14 mg, 8.6 mmol) in THF (25 ml) was treated with tetra-n-butylammonium fluoride (1.0 M in THF 8.2 ml, 8.2 mmol). After stirring at room temperature for 16 hours, the solution was diluted with EtOAc and washed three times with 5% $KHSO_4$. The EtOAc layer was washed with brine, dried ($Na_2SO_4$), filtered and stripped to afford a yellow oil. The oil was dissolved in $Et_2O$, cooled to 0° C and treated with excess diazomethane for 15 minutes. The excess diazomethane was destroyed by the addition of HOAc and the solvent was removed in vacuo. The residue was chromatographed (flash, Merck $SiO_2$, 1:1-acetone:hexane) to afford intermediate I (746 mg, 87%) as a white foam.

TLC: $R_f$ = 0.30 (1:1 - acetone:hexane)

J. (S)-4-[[[2-Cyclopropyl-4-(4-fluorophenyl)-6-phenyl-3-pyridinyl]ethynyl]hydroxyphosphinyl]-3-hydroxybutanoic acid, disodium salt

A solution of intermediate I (730 mg, 1.44 mmol) in dioxane (6 ml) was treated with 1 N NaOH (5.0 ml, 5.0 mmol) at room temperature and the mixture was subsequently heated at 60° C under argon for 0.5 hour. The solvent was evaporated and the residue was chromatographed on HP-20 eluting in succession with $H_2O$ (200 ml) and 50% MeoH in $H_2O$ (200 ml). The desired fractions were pooled and evaporated and the residue was taken up in $H_2O$ and lyophilized to give Example 17 (674 mg, 84%) as a white solid.

m.p. decomp. ≧285° C

TLC: $R_f$ = 0.07 (8:1:1-$CH_2Cl_2$:HOAc:MeOH)

Microanalysis for $C_{26}H_{21}FNNa_2O_5P$* 2.0 $H_2O$:
Calc'd: C 55.82, H. 4.51; N 2.50; F 3.40; P 5.54
Found: C 55.84, H. 4.28; N 2.46; F 3.33; P 5.50
Rotation $[\alpha]_D$ = +5.6° (MeOH, 9.8 mg in 1.21 ml)

## Example 18

(S)-4-[[[2-Cyclopropyl-4-(4-fluorophenyl)-5-methyl-6-phenyl-3-pyridinyl]ethynyl]hydroxyphosphinyl]-3-hydroxybutanoic acid, disodium salt

A. 2-Cyclopropylcarbonyl-3-(4-fluorophenyl)-2-propenoic acid, ethyl ester

A mixture of 4-fluorobenzaldehyde (4.768 g, 38.4 mmol), the compound from Example 17, part A (6.000 g, 38.4 mmol), piperidine (380 µl), and HOAc (75 µl) was refluxed in benzene (40 ml) with removal of water (Dean-Stark trap) for 16 hours. The cooled mixture was diluted with $Et_2O$ and washed successively with 5% HCl, saturated $NaHCO_3$, $H_2O$, and brine, then dried ($Na_2SO_4$), filtered, and stripped to yield an oil. Distillation of the oil (bp 127-135° C at 0.2 mm Hg) afforded compound A (9.299 g, 82%) as a pale yellow liquid.

TLC: $R_f$ = 0.31 (20% EtOAc in hexane)

Microanalysis for $C_{15}H_{15}FO_3$:
Calc'd: C 68.69; H 5.76; F 7.24
Found: C 68.92; H 5.90; F 7.25

B. α-Cyclopropylcarbonyl-β-(4-fluorophenyl)-γ-methyl-Δ-oxobenzenepentanoic acid, ethyl ester

A -78 °C solution of $LiN(TMS)_2$ (1.0 M in THF, 38 ml, 38 mmol) in dry THF (40 ml) was treated with a solution of propiophenone (5.015 g, 37.4 mmol) in THF (6 ml) over a 4-minute period. After 30 minutes, a solution of compound A (7.842 g, 29.9 mmol) in THF (15 ml) was added dropwise to the above solution. After one hour, the mixture was quenched with acetic acid (2.5 ml) and saturated $NH_4Cl$ and warmed to room temperature. The mixture was diluted with $H_2O$ and subsequently extracted once with $Et_2O$. The $Et_2O$ extract was washed with brine, dried ($Na_2SO_4$), filtered and stripped to give an oil. The oil was transferred to a round bottom flask which was fitted with a short path distillation apparatus and the oil was heated at 65 °C at 0.2 mm Hg to remove the volatiles and excess propiophenone. The pot residue, crude compound B was used directly in the next reaction.

C. 2-Cyclopropyl-4-(4-fluorophenyl)-5-methyl-6-phenyl-3-pyridinecarboxylic acid, ethyl ester

A mixture of crude compound B, $NH_4OAc$ (9.210 g, 119.5 mmol), and $Cu(OAc)_2$ (17.921 g, 89.8 mmol) in glacial HOAc (80 ml) was heated at 110 °C for 21 hours. The solution was cooled to room temperature and subsequently poured into an ice cold mixture of concentrated $NH_4OH$ (100 ml) in $H_2O$ (200 ml). The mixture was extracted twice with $Et_2O$ and the pooled $Et_2O$ extracts were washed with $H_2O$ and brine, then dried ($Na_2SO_4$), filtered and stripped to yield a brown solid. The solid was dissolved in $CH_2Cl_2$ and flashed (Merck $SiO_2$, 20% EtOAc in hexane) to give compound C as a reddish oil which readily solidified. Recrystallization from EtOAc/hexane (2 crops) afforded ester compound C (9.615 g, 86%) as white crystals.

m.p. 113-114.5 °C

TLC: $R_f$ = 0.46 (20% EtOAc in hexane)

**Microanalysis for $C_{24}H_{22}FNO_2$:**

| | |
|---|---|
| Calc'd: | C 76.78; H 5.91; N 3.73; F 5.06 |
| Found: | C 76.68; H 5.92; N 3.63; F 5.06 |

D. (S)-4-[[[2-Cyclopropyl-4-(4-fluorophenyl)-5-methyl-6-phenyl-3-pyridinyl]ethynyl]hydroxyphosphinyl]-3-hydroxybutanoic acid, disodium salt

A cold (0 °C) solution of ester C (8.942 g, 23.8 mmol) in dry THF (200 ml) was treated with $LiAlH_4$ - (2.701 g, 71.2 mmol). Ten minutes after the addition, the cooling bath was removed and the mixture was stirred at room temperature for 1.5 hours. The solution was recooled to 0 °C and quenched in succession with $H_2O$ (3.0 ml), 10% NaOH (4.5 ml), and $H_2O$ (8.5 ml). The solution was filtered and the salts were washed with EtOAc and $Et_2O$. Removal of the filtrate solvent afforded an oily solid residue. The residue was dissolved in $Et_2O$, diluted with hexane, heated to remove the $Et_2O$, and cooled to provide compound D (6.548 g, 83%) as an amorphous white solid.

m.p. 140.5-142 °C

TLC: $R_f$ = 0.18 (20% EtOAc in hexane)

**Microanalysis for $C_{22}H_{20}FNO$:**

| | |
|---|---|
| Calc'd: | C 79.25; H 6.05; N 4.20; F 5.70 |
| Found: | C 79.14; H 5.99; N 4.15; F 5.73 |

E. 2-Cyclopropyl-4-(4-fluorophenyl)-5-methyl-6-phenyl-3-pyridinecarboxaldehyde

A solution of 4-methylmorpholine N-oxide (4.002 g, 34.2 mmol) in $CH_2Cl_2$ (130 ml) was dried over $MgSO_4$ for 15 minutes. The solution was filtered directly into a 500-ml flask, using approximately 30 ml $CH_2Cl_2$ to effect the transfer. The flask was then charged with dry 4A molecular sieves (16 g), alcohol D (5.686 g, 17.05 mmol), and tetrapropylammonium perruthenate (301 mg, 0.86 mmol). After stirring at room

temperature for 30 minutes, the black solution was diluted with $Et_2O$ (200 ml), stirred for 5 minutes, then filtered through a plug of silica gel (Merck, 65 x 30 mm) washed with $Et_2O$. The filtrate was stripped to give a pale yellow solid. The solid was recrystallized from EtOAc/hexane to give compound E (3.982 g) as white crystals. Flashing the mother liquor (Merck $SiO_2$, 20% EtOAc in hexane) gave additional product, which was recrystallized from hexane (499 mg). Total pooled solids, 4.481 g (79%).

m.p. 137-139 °C
TLC: $R_f$ = 0.50 (20% EtOAc in hexane)

Microanalysis for $C_{22}H_{18}FNO$:
Calc'd: C 79.74; H 5.47; N 4.23; F 5.73
Found: C 79.32; H 5.49; N 4.13; F 5.65

F. 2-cyclopropyl-3-(2,2-dibromoethenyl)-4-(4-fluorophenyl)-5-methyl-6-phenylpyridine

A solution of carbon tetrabromide (5.810 g, 17.5 mmol) in $CH_2Cl_2$ (9 ml) was added over a 12-minute period to a cold (0 °C) solution of aldehyde E (3.864 9, 11.7 mmol) and triphenylphosphine (9.191 g, 35.0 mmol) in $CH_2Cl_2$ (60 ml). After the addition was complete, the cooling bath was removed and the mixture was stirred at room temperature for 20 minutes. The solution was quenched with saturated $NaHCO_3$ (40 ml) and extracted twice with $CH_2Cl_2$. The combined organic layers were dried ($Na_2SO_4$), filtered and concentrated. The concentrate was chromatographed (flash, Merck $SiO_2$, 40% $CH_2Cl_2$ in hexane) and the pure desired fractions were stripped to give a solid. Recrystallization from EtOAc/hexane (2 crops) provided compound F (5.432 g, 96%) as white crystals.

m.p. 155-157 °C
TLC: $R_f$ = 0.58 (20% EtOAc in hexane).

Microanalysis for $C_{23}H_{18}Br_2FN$:
Calc'd: C 56.70; H 3.72; N 2.88; F 3.90; Br 32.80
Found: C 56.55; H 3.68; N 2.82; F 3.89; Br 32.72

G. (S)-4-[[[2-Cyclopropyl-4-(4-fluorophenyl)-5-methyl-6-phenyl-3-pyridinyl]ethynyl]methoxyphosphinyl]-3-[[-(1,1-dimethylethyl)diphenylsilyl]oxy]butanoic acid, ethyl ester

A solution of dibromide F (1.000 g, 2.05 mmol) in THF (5 ml) was added dropwise to a solution of n-BuLi (2.5 M in hexane, 1.73 ml, 4.3 mmol) in THF (10 ml) at -78 °C over a 10-minute period. The resulting pale yellow solution was stirred at -78 °C for one hour, then added dropwise via cannula over a 12-minute period to a -78 °C solution of the phosphonochloridate from Example 2, part J in THF (15 ml). The resulting mixture was stirred at -78 °C for 30 minutes, then quenched with 50% saturated $NH_4Cl$. The solution was warmed to 0 °C, diluted with $H_2O$, and poured into saturated $NaHCO_3$. The aqueous phase was extracted once with $Et_2O$. The $Et_2O$ layer was washed with brine, dried ($Na_2SO_4$), filtered and stripped to give an oil. The residue was chromatographed (flash, Merck $SiO_2$, 50% EtOAc in hexane) to afford compound G as a white foam (1.158 g, 74%).

TLC: $R_f$ = 0.27 (40% EtOAc in hexane)

H. (S)-4-[[[2-Cyclopropyl-4-(4-fluorophenyl)-5-methyl-6-phenyl-3-pyridinyl]ethynyl]methoxyphosphinyl]-3-hydroxybutanoic acid, ethyl ester

A mixture of compound G (1.148 g, 1.51 mmol) and HOAc (460 µl, 482 mg, 8.0 mmol) in THF (25 ml) was treated with tetra-n-butylammonium fluoride (1.0 M in THF, 7.6 ml, 7.6 mmol). After stirring at room temperature for 17 hours, the solution was diluted with EtOAc and washed three times with 5% $KHSO_4$. The pooled aqueous extracts were back-extracted once with EtOAc and the combined EtOAc layers were washed with brine, dried ($Na_2SO_4$), filtered and stripped to afford a yellow oil. The oil was dissolved in $Et_2O$, cooled to 0 °C and treated with excess diazomethane for 15 minutes. The excess diazomethane was

destroyed by the addition of HOAc and the solvent was removed in vacuo. The residue was chromatographed (flash, Merck $SiO_2$, 40% acetone in hexane, followed by 40% hexane in acetone) to afford compound H (684 mg, 87% as a white foam.

TLC: $R_f$ = 0.37 (1:1 - acetone:hexane)

I. (S)-4-[[[2-Cyclopropyl-4-(4-fluorophenyl)-5-methyl-6-phenyl-3-pyridinyl]ethynyl]hydroxyphosphinyl]-3-hydroxybutanoic acid, disodium salt

A solution of compound H (672 mg, 1.29 mmol) in dioxane (7 ml) was treated with 1 N NaOH (4.5 ml, 4.5 mmol) at room temperature and the mixture was subsequently heated at 60°C under argon for one hour. The solvent was evaporated and the solid residue was dissolved in warm $H_2O$ and chromatographed on HP-20, eluting in succession with $H_2O$ (150 ml) and 50% MeOH in $H_2O$ (200 ml). The desired fractions were pooled and evaporated and the residue was taken up in $H_2O$ and lyophilized to give Example 18 (640 mg, 89%) as a white solid.

m.p. decomp. ≧295°C

TLC: $R_f$ = 0.07 (8:1-1-$CH_2Cl_2$:HOAc:MeOH)

Microanalysis for $C_{27}H_{23}FNNa_2O_5P$ * 1.25 $H_2O$

Calc'd: C 57.92; H 4.59; N 2.50; F 3.39; P 5.53

Found: C 58.05; H 4.50; N 2.35; F 3.38; P 5.72

Rotation: $[\alpha]_D$ = +8.8° (MeOH, 7.6 mg in 1.22 ml)

## Example 19

(S,E)-4-[[2-[2-Cyclopropyl-4-(4-fluorophenyl)-5-methyl-6-phenyl-3-pyridinyl]ethenyl]hydroxyphosphinyl]-3-hydroxybutanoic acid, disodium salt

A. 2-Cyclopropyl-3-(2-ethynyl)-4-(4-fluorophenyl)-5-methyl-6-phenylpyridine

The compound from Example 18, part F (4.000 g, 8.21 mmol) in dry THF (12 ml) was added to a solution of n-BuLi (2.5 M in hexanes, 7.40 ml, 18.5 mmol) in THF (35 ml) over a 6-minute period. After stirring at -78°C for 50 minutes, the lime-green solution was quenched with saturated $NH_4Cl$ and warmed to room temperature. The colorless mixture was diluted with $H_2O$ and extracted with $Et_2O$. The organic layer was washed with brine, dried ($Na_2SO_4$), filtered and stripped to give a white solid. The solid was recrystallized from hexane to give very pale lavender-colored needles (1st crop 2.062). From the mother liquor was obtained a second (392 mg) and third crop (151 mg) of pure product. Total pooled solids (compound A) were 2.064 g, 97%.

m.p.: 140-142°C

TLC: $R_f$ = 0.55 (EtOAc in hexane)

Analysis for $C_{23}H_{18}FN$:

Calc'd: C 84,38; H 5.54; N 4.28; F 5.80

Found: C 84.42; H 5.45, N 4.44; F 5.88

B. 2-Cyclopropyl-3-(2-iodoethenyl)-4-(4-fluorophenyl)-5-methyl-6-phenylpyridine

A mixture of compound A (1.400 g, 4.28 mmol) and AIBN (20 mg) in tri-n-butylstannyl hydride (2.4 ml, 2.60 g, 8.9 mmol) was rapidly heated to 140°C by immersion in a pre-heated oil bath. Approximately 20 mg of AIBN was added to the reaction mixture after 0.5 hour and again one hour after heating was initiated. After 1.5 hours, the dark orange mixture was cooled to room temperature, diluted with $Et_2O$ (40 ml) and treated with 2,6-lutidine (1.30 ml, 1.19 g, 11.2 mmol) followed by solid $I_2$ (3.27 g, 12.9 mmol). The dark

reaction mixture was stirred for 60 minutes, then poured into a 50% saturated $NaHCO_3$ solution containing 3.6 g $Na_2S_2O_3$. The layers were shaken and separated. The ethereal layer was washed successively with $H_2O$, 4.5 M $NH_4OH$, $H_2O$, and brine, then dried ($Na_2SO_4$), filtered and stripped to yield an oil. The residue was subjected to flash chromatography (Merck $SiO_2$, 10% EtOAc in hexane ) to afford crude compound B as a solid. Recrystallization from hexane gave compound B (1.288 g) as a yellow solid. The mother liquor was flashed (Merck $SiO_2$, 5% EtOAc in hexane) and the desired fractions were pooled, stripped, and recrystallized from hexane to give additional product (235 mg). Total yield of pure compound C 1.523 g, 78%.

m.p.: 134-136° C
TLC: $R_f$ = 0.45 (10% EtOAC in hexane)

Microanalysis for $C_{23}H_{19}FIN$:
Calc'd: C 60.67; H 4.21; N 3.08; F 4.17; I 27.87
Found: C 60.76; H 4.19; N 2.92; F 4.26; I 27.90

C. (S)-4-[[[2-Cyclopropyl-4-(4-fluorophenyl)-5-methyl-6-phenyl-3-pyridinyl]ethenyl]methoxyphosphinyl]-3-[[-(1,1-dimethylethyl)diphenylsilyl]oxy]butanoic acid, methyl ester

A solution of the vinyl iodide from part B (1.300 g 2.85 mmol) in THF (6 ml) was added over a 5-minute period to a -100° C solution of t-butylithium (1.7 M in pentane, 3.50 ml, 5.95 mmol) in THF (8 ml). The resulting deep muddy red solution was stirred for 20 minutes, then added over a 10-minute period to a -100° C solution of the phosphonochloridate from Example 2, part J in THF (15 ml). The resulting yellow mixture was stirred at minus 100° C for 30 minutes, then quenched with 50% saturated $NH_4Cl$. The solution was warmed to room temperature, diluted with $H_2O$, and poured into saturated $NaHCO_3$. The aqueous phase was extracted with $Et_2O$. The $Et_2O$ extract was washed with brine, dried ($Na_2SO_4$), filtered and stripped. The resulting yellow oil was chromatographed (flash, Merck $SiO_2$, EtOAc in hexane) to afford compound C as an oily foam (1.056 g, 49%).

TLC: $R_f$ = 0.19 & 0.14 (40% EtOAc in hexane)

D. (S)-4-[[[2-Cyclopropyl-4-(4-fluorophenyl)-5-methyl-6-phenyl-3-pyridinyl]ethenyl]methoxyphosphinyl]-3-hydroxybutanoic acid, methyl ester

A solution of compound C (1.030 g, 1.35 mmol) in THF (20 ml) was treated with HOAc (400 μl, 420 mg, 7.0 mmol) and tetra-n-butylammonium fluoride (1.0 M in THF, 6.7 mmol). After stirring at room temperature for 16 hours, the solution was poured into saturated $NaHCO_3$ and extracted with EtOAc. The EtOAc extracted with washed with brine, dried ($Na_2SO_4$), filtered, and stripped to give an oil, which was subsequently chromatographed (flash, Merck $SiO_2$, 1:1-acetone: hexane followed by 5:2-acetone:hexane). Compound D (647 mg, 92%) was obtained as an oily foam.

TLC: $R_f$ = 0.31 (1:1-acetone:hexane)

E. (S,E)-4-[[2-[2-Cyclopropyl-4-(4-fluorophenyl)-5-methyl-6-phenyl-3-pyridinyl]ethenyl]hydroxyphosphinyl]-3-hydroxybutanoic acid, disodium salt

A solution of compound D (320 mg, 0.61 mmol) in dioxane (4.5 ml) was treated with 1 N NaOH (2.1 ml, 2.1 mmol) and the mixture was stirred at 50° C for 1.75 hours. The solvent was evaporated and the residue was taken up in $H_2O$ and chromatographed on HP-20, eluting in succession with $H_2O$ (150 ml) and 50% MeOH in $H_2O$ (200 ml). The desired fractions were pooled and evaporated and the residue was taken up in $H_2O$ and lyophilized to give Example 19 (292 mg, 85%) as a white solid.

m.p.: 310° C (decomp.); $[\alpha]_D$ = +6.7° (MeOH, 6.3 mg in 1.25 ml)
TLC: $R_f$ = 0.08 (8:1:1-$CH_2Cl_2$:HOAc:MeOH)

Microanalysis for $C_{27}H_{25}FNNa_2O_5P$ * 1.33 $H_2O$:
Calc'd: C 57.56; H 4.95; N 2.49; F 3.37; P 5.50
Found: C 57.54; H 4.99; N 2.51; F 3.54; P 5.70

Example 20

(S)-4-[[2-[2-Cyclopropyl-4-(4-fluorophenyl)-5-methyl-6-phenyl-3-pyridinyl]ethyl]hydroxyphosphinyl[-3-hydroxybutanoic acid, disodium salt

A. (S)-4-[[[2-Cyclopropyl-4-(4-fluorophenyl)-5-methyl-6-phenyl-3-pyridinyl]ethyl]hydroxyphosphinyl]-3-hydroxybutanoic acid, disodium salt

A mixture of compound D from Example 18 (308 mg, 0.59 mmol) and Pd on carbon (10% Pd on C, 129 mg) in MeOH (20 ml) was shaken under 50 psi of $H_2$ for 7 hours. The solution was filtered, stripped, and chromatographed (flash, Merck $SiO_2$, 14:11-acetone:hexane followed by 100% acetone) to give compound A (271 mg, 87%) as a colorless oil.

TLC: $R_f$ = 0.29 (1:1-acetone:hexane)

B. (S)-4-[[2-[2-Cyclopropyl-4-(4-fluorophenyl)-5-methyl-6-phenyl-3-pyridinyl]ethyl]hydroxyphosphinyl[-3-hydroxybutanoic acid, disodium salt

A solution of compound A (260 mg, 0.495 mmol) in dioxane (3 ml) was treated with 1 N NaOH (1.8 ml, 1.8 mmol) and the mixture was stirred at 50° C for 2.5 hours. The solvent was evaporated and the residue was chromatographed on HP-20, eluting in succession with $H_2O$ (150 ml) and 50% MeOH in $H_2O$ (150 ml). The desired fractions were pooled and evaporated and the residue was taken up in $H_2O$ and lyophilized to give Example 20 (242 mg, 87%) as a white solid.

m.p.: 300° C (decomp.); $[\alpha]_D$ = -0.3° (MeOH, 4.0 mg in 1.25 ml)

TLC: $R_f$ = 0.09 (8:1:1-$CH_2Cl_2$:HOAc:MeOH)

Microanalysis for $C_{27}H_{27}FNNa_2O_5P$ * 1.25 $H_2O$:
Calc'd: C 57.50; H 5.27; N 2.48; F 3.37; P 5.49
Found: C 57.46; H 5.23; N 2.38; F 3.38; P 5.61

Example 21

(S)-4-[[[4-(4-Fluorophenyl)-6,7-dihydro-2-(1-methylethyl)-5H-benzo[6,7]cyclohepta[1,2-b]pyridin-3-yl]-methoxy]hydroxyphosphinyl]-3-hydroxybutanoic acid, disodium salt

A. (S)-4-[[[4-(4-Fluorophenyl)-6,7-dihydro-2-(1-methylethyl)-5H-benzo[6,7]cyclohepta[1, 2-b]pyridin-3-yl)-methoxy]methoxyphosphinyl]-3-[[(1,1-dimethylethyl)diphenylsilyl]oxy]butanoic acid, methyl ester

The phosphonochloridate from Example 2, part J (2.157 g, 3.41 mmol) was partitioned between EtOAc and 5% $KHSO_4$. The EtOAc layer was washed three times with 5% $KHSO_4$, then with brine, then dried ($Na_2SO_4$), filtered and stripped to give a colorless oil (phosphonic acid monomethyl ester). The oil was dissolved in dry $CH_2Cl_2$ (20 ml) and treated with N,N-diethyltrimethylsilylamine (0.992 g, 6.85 mmol). After stirring at room temperature for one hour, the solvent was removed in vacuo and the residue was azeotroped with dry toluene (20 ml). The residue was re-dissolved in dry $CH_2Cl_2$ (20 ml), cooled to 0° C and treated with DMF (0.050 g, 0.68 mmol) and oxalyl chloride (0.52 g, 4.10 mmol). After 15 minutes, the solution was warmed to room temperature and stirred for an additional 45 minutes. The solvent was stripped and the yellow residue was azeotroped with toluene (20 ml) and dried in vacuo (oil pump) for 1 hour. The residue was then dissolved in pyridine (15 ml) and the temperature of the solution was lowered to 0° C. A pyridine solution (5 ml) of compound C from Example 63 (0.691 g, 1.91 mmol) was then added to the flask

dropwise and the solution was allowed to stir 17 hours at 0° C. The reaction was then quenched with saturated $NH_4Cl$, and the mixture diluted with EtOAc (50 ml). The organic layer was washed with water and brine, then dried over $Na_2SO_4$, filtered and concentrated to an amber oil. The oil was purified by flash chromatography on Merck silica gel in 30% EtOAc in hexane. After combination and concentration of those fractions containing product, compound A was obtained as a yellow oil (0.604 g, 40%).

TLC: $R_f$ = 0.53 (40% EtOAc in hexane).

B. (S)-4-[[[4-(4-Fluorophenyl)-6,7-dihydro-2-(1-methylethyl)-5H-benzo[6,7]cyclohepta [1,2-b]pyridin-3-yl]-methoxy]methoxyphosphinyl]-3-hydroxybutanoic acid, methyl ester

TBAF (1 M in THF, 2.25 ml, 2.25 mmol) was added to a THF (20 ml) solution of compound A (0.597 mmol) and acetic acid (0.180 g, 3.01 mmol), and the solution was allowed to stir at room temperature for 17 hours. More TBAF (1.0 M in THF, 1.12 g, 1.12 mmol) and HOAc (0.090 g, 1.50 mmol) were added to the solution, which stirred another 3 hours. The solution was then diluted with EtOAc (50 ml), washed with saturated $NaHCO_3$, water and brine, dried over $Na_2SO_4$, and filtered and concentrated to an orange oil. The oil was purified by flash chromatography on Merck silica gel in 40% acetone in hexane. Product fractions were combined and concentrated to afford compound B as a clear oil (0.404 g, 97%).

TLC: $R_f$ = 0.63 (50% acetone in hexane).

C. (S)-4-[[[4-(4-Fluorophenyl)-6,7-dihydro-2-(1-methylethyl)-5H-benzo[6,7]cyclohepto [1,2-b]pyridin-3-yl]-methoxy]hydroxyphosphinyl]-3-hydroxybutanoic acid, disodium salt

An aqueous solution of NaOH (1 M in $H_2O$, 1.99 ml, 1.99 mmol) was added to a solution of compound B (0.370 g, 0.67 mmol) in dioxane (10 ml). The mixture was stirred at 55° C for 17 hours. The solution was then concentrated to a white solid. The solid was dissolved in hot $H_2O$ to form a white slurry. The slurry was applied to a column of HP20 gel, and the column was eluted first with 200 ml $H_2O$, then with 400 ml 30% MeOH in $H_2O$. Those fractions containing the product were combined and concentrated to a white solid. The solid was dissolved in water and lyophilized to afford Example 21 as a white, fluffy solid (0.280 g, 73%).

Melting point: 300° C (decomp).

Elemental analysis for $C_{28}H_{29}NFNa_2PO_6$*2.00 mole $H_2O$

Calc'd: C 55.36, H 5.47, N 2.31, F 3.13, P 5.10

Found: C 55.65, H 5.38, N 2.19, F 3.12, P 5.17

Optical rotation: $[a]_D$ = -0.3° (MeOH, c = 3.8 mg/ml).

Examples 22 to 26

These examples were prepared following the procedures of Example 99, substituting the appropriate pyridine methanol to form the product. The examples follow the formula

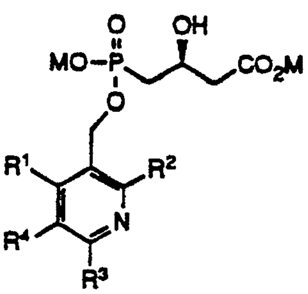

in which M is sodium and $R^1$ to $R^4$ are as defined in the table below.

| Example | $R^1$ | $R^2$ | $R^3$ | $R^4$ |
|---|---|---|---|---|
| 22 | 4-F-$C_6H_4$ | i-$C_3H_7$ | $C_6H_5$ | H |
| 23 | 4-F-$C_6H_4$ | i-$C_3H_7$ | $C_6H_5$ | $CH_3$ |
| 24 | 4-F-$C_6H_4$ | i-$C_3H_7$ | $C_6H_5$ | $CH_2CH_3$ |
| 25 | 4-F-$C_6H_4$ | c-$C_3H_5$ | $C_6H_5$ | $CH_3$ |
| 26 | 4-F-$C_6H_4$ | $CH_2CH_3$ | $C_6H_5$ | $CH_3$ |

Example 27

This example embodies reaction scheme 9. All reactions were carried out under a static atmosphere of argon and stirred magnetically unless otherwise noted. All reagents used were of commercial quality and were obtained from Aldrich Chemical Co. Dry THF and $Et_2O$ were obtained by distillation from the sodium ketyl of benzophenone under nitrogen. Dry $CH_2Cl_2$, were obtained by distillation from $CaH_2$ under nitrogen. Pyridine and dioxane were obtained from American Burdick and Jackson and were stored over 4 angstrom molecular sieves. All flash chromatographic separations were performed using E. Merck silica gel (60, particle size, 0.040-0.063 mm). Reactions were monitored by TLC using 0.25 mm E. Merck silica gel plates (60 F)

A. 2-Methyl-1-(2-aminophenyl)-1-propanone

A cold (0 °C) solution of anthranilonitrile (8.507 g, 72.0 mmol) in dry $Et_2O$ (30 ml) was treated dropwise with i-propylmagnesium chloride (2.0 M in $Et_2O$, 100 ml, 200 mmol) over a 15-minute period. After the addition was complete, the mixture was warmed to room temperature and stirring continued for 4.5 hours. The solution was re-cooled to 0 °C and carefully quenched by the addition of 10% HCl (150 ml). The mixture was warmed to room temperature, stirred for 30 minutes, re-cooled to 0 °C and made basic by the addition of solid NaOH (25 g). The aqueous layer was extracted with $Et_2O$ (3 x 200 ml ) and the combined ethereal extracts were washed with brine (150 ml) and dried ($Na_2SO_4$). The solution was evaporated and the crude product was flash chromatographed on a silica gel column (50 cm x 200 cm) using 15% EtOAc in hexane as the eluting solvent mixture to afford compound A as a golden yellow oil (yield 10.916 g, 92%); $R_f$ 0.32 (20% EtOAc in hexane). Pure compound A may also be obtained in slightly diminished yield (75%) by direct distillation of the crude product (bp 86-91 °C/0.5 Torr).

Microanalysis for $C_{10}H_{13}NO$:

| | | | |
|---|---|---|---|
| Calc'd: | C 73.58 | H 8.03 | N 8.58 |
| Found: | C 73.57 | H 8.01 | N 8.94 |

B. 3-[[2-(2-Methyl-1-oxopropyl)phenyl]amino]-3-oxopropanoic acid, ethyl ester

A mixture of compound A (5.000 g, 30.6 mmol) and dry pyridine (7.4 ml), 7.2 g, 91.5 mmol) in dry $CH_2Cl_2$ (60 ml) at -78 °C was treated dropwise with a solution of ethyl malonyl chloride (Aldrich, tech. grade, 4.62 g, 30.7 mmol based on 100% purity) in $CH_2Cl_2$ (10 ml) over a 15-minute period. Thirty minutes after the addition, the reaction was quenched with $H_2O$ (50 ml) and warmed to room temperature. The mixture was extracted with $Et_2O$ (150 ml) and the organic extract is washed in succession with $H_2O$ (50 ml), 5% HCl (50 ml), $H_2O$ (50 ml), and brine (50 ml), then dried ($Na_2SO_4$), filtered, and concentrated affording a red-brown oil. Flash column chromatography (50 cm x 230 cm) of the crude oil on silica gel using 25% EtOAc in hexane as the eluant provides compound B as a near-colorless oil; yield 5.620 g, (66%); $R_f$ 0.49 (40% EtOAc in hexane).

**Microanalysis for $C_{15}H_{19}NO_4$:**

| | | | |
|---|---|---|---|
| Calc'd: | C 64.96 | H 6.91 | N 5.05 |
| Found: | C 64.94 | H 6.80 | N 6.40 |

C. 2-Hydroxy-4-(1-methylethyl)-3-quinolinecarboxylic acid, ethyl ester

A solution of compound B (4.555 g, 16.4 mmol) in absolute EtOH (65 ml) was treated with a solution of EtONa in EtOH (21% by weight, 1.8 g, 6.8 mmol) and the mixture was stirred at room temperature for 5 minutes, then at reflux for 5 minutes. The solution was cooled and partitioned between EtOAc (200 ml) and 50% saturated $NH_4Cl$ (150 ml). The layers were shaken and separated and the organic layer is washed with brine (2 x 75 ml) and dried ($Na_2SO_4$). Filtration and solvent removal afforded a solid, which was recrystallized from EtOAc/hexane giving compound C (2 crops) as fine white needles; yield 3.950 g, (93%); $R_f$ 0.16 (40% EtOAc in hexane).

m.p. 195.2-196.8 °C

**Microanalysis for $C_{15}H_{17}NO_3$:**

| | | | |
|---|---|---|---|
| Calc'd: | C 69.48 | H 6.61 | N 5.40 |
| Found: | C 69.47 | H 6.61 | N 5.38 |

D. 3-(Hydroxymethyl)-4-(1-methylethyl)-2-quinolinol

To a cold (0 °C) slurry of $LiAlH_4$ (1.624 g, 42.8 mmol) in dry THF (200 ml) was added compound C (10.000 g, 38.6 mmol) portionwise as a solid (Caution: vigorous $H_2$ evolution). After stirring at 0 °C for 45 minutes, the solution was carefully quenched by the successive addition of $H_2O$ (75 ml) and saturated $NH_4Cl$ (150 ml). The mixture was extracted with EtOAc (2 x 150 ml) and the pooled organic extracts were washed with brine (100 ml), dried ($Na_2SO_4$), filtered and evaporated to provide a yellow solid. The solid residue was recrystallized from EtOAc, giving compound D as a pale yellow amorphous solid (2 crops); yield 7.803 g, (93%); $R_f$ 0.18 (1:1 acetone:hexane).

m.p. 149.5-152.0 °C

**Microanalysis for $C_{13}H_{15}NO_2$:**

| | | | |
|---|---|---|---|
| Calc'd: | C 71.86 | H 6.96 | N 6.45 |
| Found: | C 71.61 | H 6.94 | N 6.31 |

E. 2-Hydroxy-4-(1-methylethyl)-3-quinolinecarboxaldehyde

A mixture of compound D (3.000 g, 13.8 mmol) and $MnO_2$, (Aldrich, activated type, 4.02 g, 46.2 mmol) in $CH_2Cl_2$ (100 ml) was stirred at room temperature for 24 hours. The reaction was treated with additional $MnO_2$, (3.00 g 34.5 mmol) and the mixture was subsequently refluxed for 3 days. After cooling to room temperature, the solution is filtered through a Celite® pad, which was washed with $CH_2Cl_2$. Evaporation of the filtrate solvent gives a greenish solid which is dissolved in acetone and filtered through a plug of silica gel (100 g), washing with 1:1 acetone:hexane. The filtrate is stripped and the residue is recrystallized from EtOAc/hexane affording compound E (two crops) as bright yellow crystals (2.512 g, 85% yield); $R_f$ 0.44 (1:1 acetone:hexane).

m.p. 177.0-179.0 °C

**Microanalysis for $C_{13}H_{13}NO_2$:**

| | | | |
|---|---|---|---|
| **Calc'd:** | **C 72.54** | **H 6.09** | **N 6.51** |
| **Found:** | **C 72.42** | **H 6.03** | **N 6.32** |

F. 4-(1-Methylethyl)-2-[[(trifluoromethyl)sulfonyl]oxy]-3-quinolinecarboxaldehyde

A bright yellow solution of compound E (1.961 g, 9.1 mmol) and dry pyridine (2.20 ml, 2.15 g, 27.2 mmol) in dry $CH_2Cl_2$ (33 ml) is cooled to 0° C and subsequently treated dropwise with neat trifluoromethanesulfonic anhydride (1.80 ml, 3.02 g, 10.7 mmol) over a 5-minute period. After stirring for 1.75 hours at 0° C, the tan solution is partitioned between $Et_2O$ (100 ml) and saturated $NaHCO_3$ (60 ml). The layers are shaken and separated and the organic layer is washed with brine (50 ml), dried ($Na_2SO_4$), filtered, and stripped. The residue is flash chromatographed on a silica gel column (50 cm x 160 cm) using 20% EtOAc in hexane as the eluting solvent mixture to afford compound F as a white solid. Recrystallization of the solid from hexane gives the product as cubic crystals; yield 2.836 g, (90%); $R_f$ 0.34 (20% EtOAc in hexane).

m.p. 69-71° C

**Microanalysis for $C_{14}H_{12}F_3NO_4S$:**

| | | | | | |
|---|---|---|---|---|---|
| **Calc'd:** | **C 48.41** | **H 3.48** | **N 4.03** | **F 16.33** | **S 9.23** |
| **Found:** | **C 48.40** | **H 3.26** | **N 3.79** | **F 16.04** | **S 9.18** |

G. 2-(4-Fluorophenyl)-4-(1-methylethyl)-3-quinolinecarboxaldehyde

A mixture of compound F (2.000 g, 5.76 mmol), (4-fluorophenyl)tributylstannane (2.400 g, 6.23 mmol) prepared by reaction of the corresponding lithium or Grignard reagent with tri-n-butyltin chloride in $Et_2O$ at -78° C), anhydrous LiCl (832 mg, 19.6 mmol), and BHT (50 mg) in dry dioxane (35 ml) was treated with $(PPh_3)_4Pd$ (200 mg, 0.17 mmol) at room temperature. The temperature of the reaction was slowly raised to 100° C over a 2-hour period and heating was continued for 13 hours. The dark mixture was cooled to room temperature and partitioned between $Et_2O$ (100 ml) and $H_2O$ (75 ml). The layers were shaken and separated and the organic layer is washed in succession with $H_2O$ (50 ml), 15% $NH_4OH$ (75 ml), $H_2O$ (50ml), and brine (50 ml), then dried ($Na_2SO_4$), filtered, and stripped. The residual yellow oil was flash-chromatographed on a silica gel column (50 cm x 150 cm) using 15% EtOAc in hexane as the eluting solvent mixture to afford the desired product as a pale yellow oil that solidified on standing. Recrystallization of the solid from hexane (2 crops) gave compound G as pale yellow needles; yield 1.597 g, (95%); $R_f$ 0.45 (20%EtOAc in hexane).

m.p. 69.3-71.5° C

Microanalysis for $C_{19}H_{16}FNO$:

| | | | | |
|---|---|---|---|---|
| Calc'd: | C 77.79 | H 5.50 | N 4.78 | F 6.48 |
| Found: | C 77.83 | H 5.44 | N 4.82 | F 6.43 |

The abbreviations used herein have the following meanings:

| | |
|---|---|
| HOAc | Acetic Acid |
| $Et_2O$ | Diethyl Ether |
| THF | Tetrahydrofuran |
| EtOH | Ethanol |
| LAH | Lithium Aluminum Hydride |
| $LiAlH_4$ | Lithium Aluminum Hydride |
| DIBAL-H | Diisobutyl Aluminum Hydride |

| | |
|---|---|
| DMF | Dimethylformamide |
| DMSO | Dimethyl Sulfoxide |
| MeONa | Sodium Methoxide |
| EtONa | Sodium Ethoxide |
| $KOBu_t$ | Potassium t-butoxide |
| NaH | Sodium Hydride |
| LDA | Lithium Diisopropylamide |
| LiTMP | Lithium Tetramethylpiperidide |
| $LiN(TMS)_2$ | Lithium Bistrimethylsilylamide |
| $NH_2OH.HCl$ | Hydroxylamine Hydrochloride |
| $NH_4OAc$ | Ammonium Acetate |
| $Cu(OAc)_2$ | Copper (II) Acetate |
| MeOH | Methanol |
| Pd | Palladium |
| Pt | Platinum |
| Ru | Ruthenium |
| EtOAc | Ethyl Acetate |
| n-BuLi | n-Butyl Lithium |
| BSTFA | Bis(trimethylsilyl)trifluoroacetamide |
| TMSBr | Trimethylsilyl bromide |
| NMO | 4-methyl morpholine N-oxide |
| TPAP | tetrapropylammonium perruthenate |
| Pd/C | Palladium on carbon |
| BHT | Butylated hydroxytoluene |
| $PPh_3$ | Triphenylphosphine |
| TBAF | Tetrabutyl Ammonium Fluoride |
| $(MeO)_2POCHN_2$ | Dimethyl Diazomethylphosphonate |
| $CHCl_3$ | Chloroform |
| $Bu_3SnH$ | Tri-n-butyl tin Hydride |
| $Bu_4NF$ | Tetrabutylammonium fluoride |
| AIBN | $\alpha,\alpha'$-Azaisobutyrylnitrile |
| TEA | Triethylamine |
| $ClSi(t\text{-butyl})Ph_2$ | t-Butyldiphenylsilyl Chloride |
| $Clsi(t\text{-butyl})Me_2$ | t-Butyldimethylsilyl Chloride |
| $ClSi\ Ph_3$ | Triphenylsilyl Chloride |
| HF | Hydrofluoric Acid |
| m-CPBA | meta-Chloroperoxybenzoic Acid |
| $CF_3CO_3H$ | Peroxytrifluoroacetic Acid |
| t-butylLi (or t-BuLi) | t-Butyl Lithium |
| EDTA | Ethylenediaminetetroacetic acid |

## Claims

1. A compound of the formula

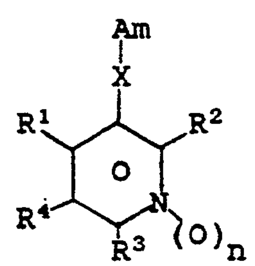

wherein:

Am is a binding domain sidechain;

X is a linker;

$R^1$ and $R^2$ are the same or different and are each independently selected from
(i) hydrogen,
(ii) alkyl,
(iii) aryl,
(iv) cycloalkyl,
(v) aralkyl,
(vi) aralkoxy,
(vii) alkenyl,
(viii)cycloalkenyl, and
(ix) heterocyclo;

$R^3$ is selected from
(i) hydrogen,
(ii) lower alkyl,
(iii) aryl,
(iv) cycloalkyl,
(v) alkoxy,
(vi) aralkyl,
(vii) aralkoxy,
(viii)alkenyl,
(ix) cycloalkenyl,
(x) halo-substituted alkyl,
(xi) adamantyl, and
(xii) heterocyclo;

$R^4$ is selected from
(i) hydrogen,
(ii) lower alkyl,
(iii) aryl,
(iv) cycloalkyl,
(v) alkoxy,
(vi) aralkyl,
(vii) aralkoxy,
(viii)alkenyl,
(ix) cycloalkenyl,
(x) adamantyl,
(xi) halogen,
(xii)halo-substituted alkyl (e.g., trifluoromethyl), and
(xiii)heterocyclo (e.g., thienyl, benzodioxolyl);

or

$R^3$ and $R^4$ taken together can be $-(CH_2)p-$,

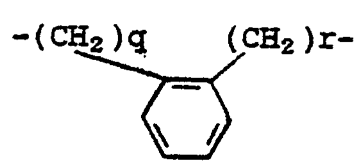

or $(CH=CH)_2$; but when $A_m$ is

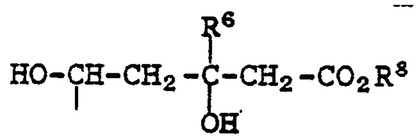

or a $\delta$ lactone thereof, $R^3$ and $R^4$ cannot be $(CH=CH)_2$;

$R^6$ is hydrogen or lower alkyl;

$R^8$ is hydrogen, lower alkyl, alkali metal, or alkaline earth metal;
n is 0 or 1;
p is 3, 4 or 5;
q is 0, 1, 2, or 3; and
r is 0, 1, 2, or 3;
provided that at least one of $R^1$, $R^2$, $R^3$ or $R^4$ is alkenyl, cycloalkyl, cycloalkenyl, or heterocyclo, or one of $R^3$ and $R^4$ is adamantyl or together are $-(CH_2)p-$,

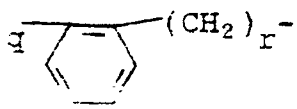

or $(CH=CH)_2$, or $R^4$ is halogen.

2. The compound defined in Claim 1 wherein Am is a phosphinic acid or salt thereof.

3. The compound defined in Claim 2 wherein Am is

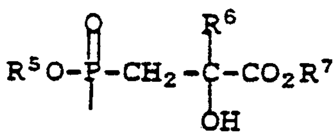

wherein:
$R^5$ and $R^7$ are independently selected from hydrogen, lower alkyl, alkali metal salt ion and alkaline earth metal salt ion; and
$R^6$ is hydrogen or lower alkyl.

4. The compound defined in Claim 1 wherein Am is a dihydroxy acid or salt thereof.

5. The compound defined in Claim 4 wherein Am is

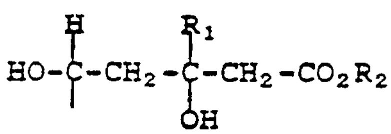

wherein $R_1$, is H or lower alkyl; and $R_2$ is H or lower alkyl in free acid form or in the form of a physiologically acceptable and hydrolyzable ester or δ lactone thereof, or an alkali metal salt ion or alkaline earth metal salt ion.

6. The compound defined in Claim 2 wherein:
X is $-(CH_2)_a$, $-CH=CH-$, $-C\equiv C-$, or $-CH_2O-$;
and
"a" is 1, 2, or 3.

7. The compound defined in Claim 5 wherein:
X is $-(CH_2)_a$, $-CH=CH-$, $-C\equiv C-$, or $-CH_2O-$;
and
"a" is 1, 2, or 3.

8. The compound defined in Claim 6 wherein X is trans-CH=CH-.

9. The compound defined in Claim 6 wherein X is cis-CH=CH-.

10. The compound defined in Claim 7 wherein X is trans-CH = CH-.

11. The compound defined in Claim 7 wherein x is cis-CH = CH-.

12. The compound defined in Claim 1 having the name
(S)-4-[[[4-Fluorophenyl)-2-(1-methylethyl)-5H-indeno[1,2-b]pyridin-3-yl]ethynyl]hydroxyphospphinyl]-3-hydroxybutanoic acid, disodium salt;
(S)-4-[[[4-(4-Fluoro-3-methylphenyl)-2-(1-methylethyl)-6-phenyl-3-pyridinyl]ethynyl]-hydroxyphosphinyl-3-hydroxybutanoic acid, disodium salt;
(S)-4-[[[4-(4-Fluorophenyl)-5,6-dihydro-2-(1-methylethyl)benzo[h]quinolin-3-yl]ethynyl]-hydroxyphosphinyl]-3-hydroxybutanoic acid, disodium salt;
(S)-4-[[[6-(1,3-Benzodioxol-4-yl)-4-(4-fluorophenyl)-2-(1-methylethyl)-3-pyridinyl]ethynyl]-hydroxyphosphinyl]-3-hydroxybutanoic acid, disodium salt;
(S)-4-[[[6-(1,3-Benzodioxol-5-yl)-4-(4-fluorophenyl)-2-(1-methylethyl)-3-pyridinyl]ethynyl]-hydroxyphosphinyl]-3-hydroxybutanoic acid, disodium salt;
(S)-4-[[[4-(4-Fluorophenyl)-5,6,7,8-tetrahydro-2-(1-methylethyl)-3-quinolinyl]-ethynyl]-hydroxyphosphinyl]-3-hydroxybutanoic acid, disodium salt;
(S)-4-[[[4-(4-Fluoro-3-methylphenyl)-2-(1-methylethyl)-3-quinolinyl]ethynyl]hydroxyphosphinyl]-3-hydroxybutanoic acid, 1-oxide, disodium salt;
(S)-4-[[[4-(1,1-Dimethylethyl)-2-(4-fluorophenyl)-3-quinolinyl]ethynyl]hydroxyphosphinyl]-3-hydroxybutanoic acid, disodium salt;
(S)-4-[[[2-(4-Fluoro-3-methylphenyl)-4-(1-methylethyl)-3-quinolinyl]-ethynyl]hydroxyphosphinyl]-3-hyroxybutanoic acid, sodium salt;
(3R,5S,6E)-7-[4-(4-Fluoro-3-methylphenyl)-2-(1-methylethyl)-6-phenyl-3-pyridinyl]-3,5-dihydroxy-6-heptenoic acid, monosodium salt;
(S)-4-[[[4-(4-Fluorophenyl)-6,7-dihydro-2-(1-methylethyl)-5H-benzo[6,7]cyclohepta[1,2-b]pyridin-3-yl]methoxy]hydroxyphosphinyl]-3-hydroxybutanoic acid, disodium salt; and
(S)-4-[[[4-(4-Fluorophenyl)-5-methyl-2-(1-methylethenyl)-6-phenyl-3-pyridinyl]methoxy]-hydroxyphosphinyl]-3-hydroxybutanoic acid, disodium salt.

13. A hypocholesterolemic or hypolipidemic composition comprising a compound as defined in Claim 1 and a pharmaceutically acceptable carrier thereof.

14. Use of a compound according to claim 1 for preparing a drug for inhibiting cholesterol biosynthesis by administration to a mammal.